# FASCICULE DE BREVET EUROPEEN

(11) **EP 0 737 187 B1**
(45) Date de publication et mention de la délivrance du brevet: **06.05.1999**
(21) Numéro de dépôt: 95906357.9
(22) Date de dépôt: 29.12.1994
(51) Int. Cl.: C07D 265/36, C07C 49/84, C07C 255/56, A61K 31/535

(54) **DERIVES DE LA BENZOXAZINE, LEURS PROCEDES D'OBTENTION ET LEUR UTILISATION EN TANT QUE MEDICAMENTS**
BENZOXAZINDERIVATE, VERFAHREN DIESE ZU ERHALTEN UND IHRE VERWENDUNG ALS MEDIKAMENTE
BENZOXAZINE DERIVATIVES, METHODS FOR OBTAINING SAME AND THEIR USE AS DRUGS

(30) Priorité: 29.12.1993 FR 9315836
(43) Date de publication de la demande: 16.10.1996
(73) Titulaire: Laboratoires Pharmascience, 92400 Courbevoie (FR)
(72) Inventeur: FLEURY, Maurice-Bernard, F-92200 Neuilly-sur-Seine (FR); MAURETTE, Jean-Marc, F-92250 La Garenne-Colombes (FR); LARGERON, Martine, F-92200 Neuilly-sur-Seine (FR)
(74) Mandataire: Grosset-Fournier, Chantal Catherine
(86) Numéro de dépôt international: FR9401557
(87) Numéro de publication internationale: WO9518114

(56) Documents cités:
- EP-A- 0 310 096
- EP-A- 0 530 444
- FR-A- 2 377 202
- FR-A- 2 504 917
- US-A- 4 263 322
- Book no. 10(3), 1971, 'BEILSTEIN HANDBUCH DER ORGANISCHEN CHEMIE, DRITTES ERGäNZUNGSWERK', HANS G. BOIT SPRINGER VERLAG, VIERTE AUFLAGE, BERLIN

## Description

La présente invention a pour objet des dérivés de la benzoxazine, ainsi que leurs procédés d'obtention. L'invention vise également les compositions pharmaceutiques comprenant ces dérivés, et leurs utilisations notamment dans le cadre du traitement de pathologies associées à la présence de radicaux libres, et plus particulièrement de la maladie d'Alzheimer.

L'hexahydroxy-2,3,4,3',4',5'-benzophénone, désignée également Exifone, a été décrite dans le brevet français publié sous le numéro 2 257 272 et déposé sous le numéro 74 01228 le 15 janvier 1974, en tant que médicament utilisable dans le cadre du traitement des maladies du système circulatoire capillaire.

L'Exifone est décrite dans le brevet français susmentionné parmi un ensemble de composés présentant une action de protection des catécholamines qui est mise à profit dans le traitement des maladies du système circulatoire (artères, veines) capillaire, et des maladies découlant d'un trouble de l'hémostase et/ou de l'agrégation plaquettaire, en particulier les maladies variqueuses et hémorroïdaires. En outre, certains des composés décrits dans ce brevet, dont l'Exifone, sont présentés comme possédant des propriétés anti-inflammatoires et améliorent la résistance capillaire.

L'Exifone a par la suite été plus particulièrement décrite dans le brevet publié sous le numéro 2 377 202 et déposé sous le numéro 77 00706 le 12 janvier 1977 en tant que certificat d'addition au brevet français n° 74 01228 susmentionné.

L'Exifone est décrite dans ce certificat d'addition comme pouvant être utilisée dans le cadre du traitement des déficits fonctionnels et des perturbations métaboliques qui découlent d'une insuffisance circulatoire cérébrale et/ou périphérique. Les déficits fonctionnels et troubles métaboliques susceptibles d'être traités par l'Exifone et particulièrement cités dans ce certificat d'addition, sont les suivants:
* toute symptomatologie fonctionnelle traduisant une insuffisance vasculaire cérébrale, soit par vasorégulation de la microcirculation, soit par amélioration de l'oxygénation cérébrale, et plus particulièrement:
   - les troubles psychiques et intellectuels:
      . modification de l'humeur,
      . troubles du comportement du sommeil,
      . défaut d'attention et de concentration,
      . troubles de la mémoire et de l'idéation.
   - les troubles sensoriels:
      . céphalées, vertiges, acouphènes et hypoacousie.
* les accidents vasculaires cérébraux subaigus et peu profonds.
* en neurochirurgie, les syndromes post-commotionnels et post-traumatiques ou séquelles d'interventions chirurgicales (oedème cérébral notamment).
* en ophtalmologie, toutes les affections d'origine vasculaire:
   - rétinopathies dégénératives (diabète, hypertension),
   - spasmes rétiniens,
   - syndromes ischémiques du fond de l'oeil.
* en ORL:
   - syndromes cochléo-vestibulaires: acouphène, vertiges, maladie de Ménière, hypoacousie et tympano-labyrinthopexie, sclérose,
   - suite d'interventions chirurgicales et séquelles de traumatisme.

Plus récemment encore, l'Exifone a été décrite comme étant active dans le cadre du traitement de la maladie d'Alzheimer, notamment par ses effets sur les troubles mnésiques, le comportement et la dépendance des malades atteints de démence sénile de type Alzheimer (Piette et al., La Revue de Gériatrie, Tome 11, n° 8, 1986).

Cependant, l'utilisation de l'Exifone en tant que médicament se heurte à des difficultés liées notamment à la faible solubilité de l'Exifone dans les solvants aqueux, ainsi qu'à sa faible biodisponibilité, et en raison de fortes liaisons s'établissant entre cette dernière et les protéines dans l'organisme.

Par ailleurs, l'Exifone serait susceptible d'être mal tolérée, en raison notamment d'une certaine hépatotoxicité qui aurait été constatée chez quelques patients.

Le composé de formule suivante 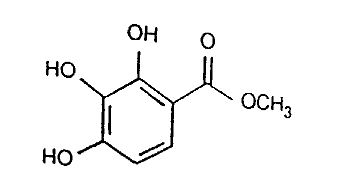 encore désigné composé de formule (VIIIf) ci-après, est utilisé dans le cadre de la préparation de benzamides et d'acides hydroxybenzohydroxamiques antitumoraux décrits dans le brevet américain n° 4,263,322 déposé le 1er mars 1979 au nom de van't Riet et al. Ce composé est également utilisé dans la synthèse de composés décrits ci-après dans le cadre de la présente invention.

La présente invention concerne les composés répondant à la formule générale (I) suivante: 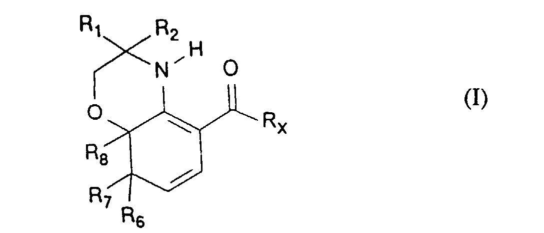 dans laquelle:
- R₁ et R₂, indépendamment l'un de l'autre, représentent un groupe alkyle de 1 à 3 atomes de carbone, ou un groupe de formule -CH₂-ORₐ, Rₐ représentant un atome d'hydrogène ou un groupe méthyle,
- Rₓ représente:
   . un groupe alkyle de 1 à 3 atomes de carbone, notamment un groupe méthyle, ou
   . un groupe -ORₓ₁, Rₓ₁ représentant un groupe alkyle de 1 à 3 atomes de carbone, notamment un groupe méthoxy, ou
   . un groupe de formule -CH₂-X dans laquelle X représente un atome d'halogène, notamment le chlore, ou
   . un groupe de formule 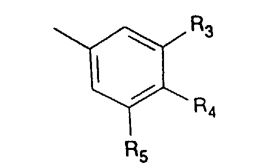 dans laquelle:
      . R₃ et R₅, indépendamment l'un de l'autre, représentent un atome d'hydrogène, ou un groupe -OH, ou un groupe -OCH₃,
      . R₄ représente un atome d'hydrogène, ou un groupe -OR_{b}, R_{b} représentant un atome d'hydrogène ou un groupe méthyle, ou R₄ représente un groupe de formule -OR_{c}, R_{c} représentant un groupe de formule -CH₂-CO-C₆H₄R' dans laquelle R' représente un atome d'hydrogène, -CN ou -OCH₃,
- soit R₆ représente un groupe -OR_{d}, R_{d} représentant un atome d'hydrogène ou un groupe méthyle, lorsque R₇ et R₈ s'associent pour former une double liaison,
- soit R₈ représente un groupe -ORₑ dans lequel Rₑ représentant un atome d'hydrogène ou un groupe méthyle, lorsque R₆ et R₇ s'associent pour former avec l'atome de carbone en position 8 du cycle, un groupe C = O.

L'invention a plus particulièrement pour objet les composés correspondant à la formule (II) suivante: 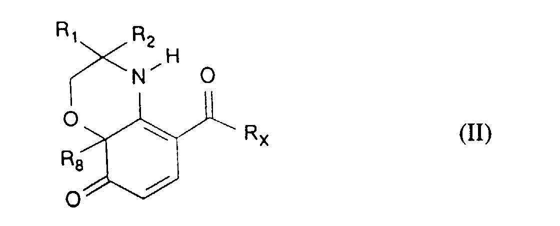 dans laquelle R₁, R₂, R₈ et Rₓ sont tels que définis ci-dessus.

L'invention concerne plus particulièrement les composés correspondant à la formule (III) suivante: 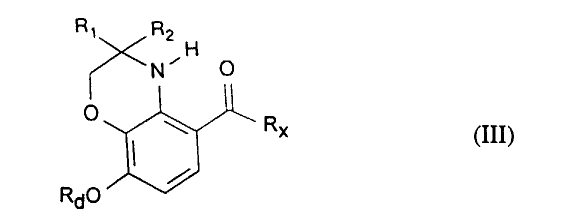 dans laquelle R₁, R₂, R_{d} et Rₓ sont tels que définis ci-dessus.

L'invention a également plus particulièrement pour objet les composés correspondant à la formule (IV) suivante: 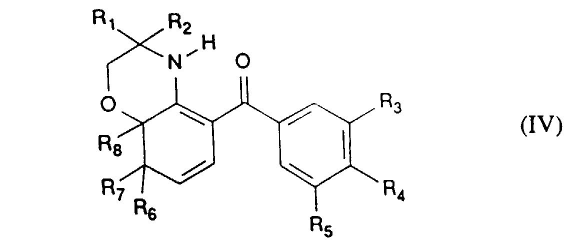 dans laquelle R₁ à R₈ sont tels que définis ci-dessus.

L'invention concerne plus particulièrement encore les composés correspondant à la formule (V) suivante: 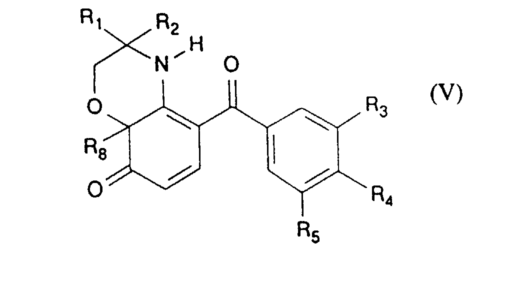 dans laquelle R₁ à R₅ et R₈ sont tels que définis ci-dessus.

Des composés de formule (V) particulièrement préférés sont ceux correspondant aux formules suivantes:

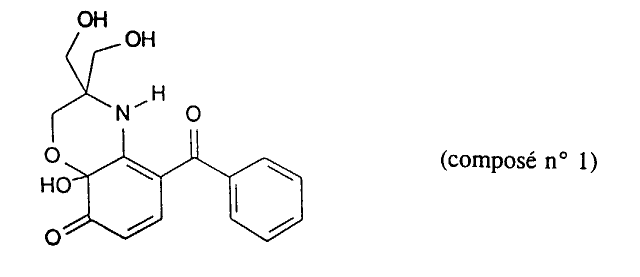

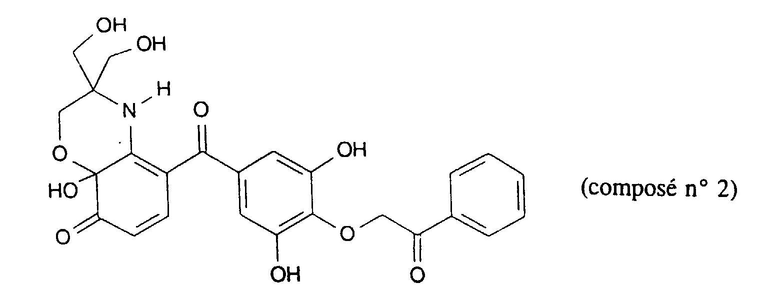

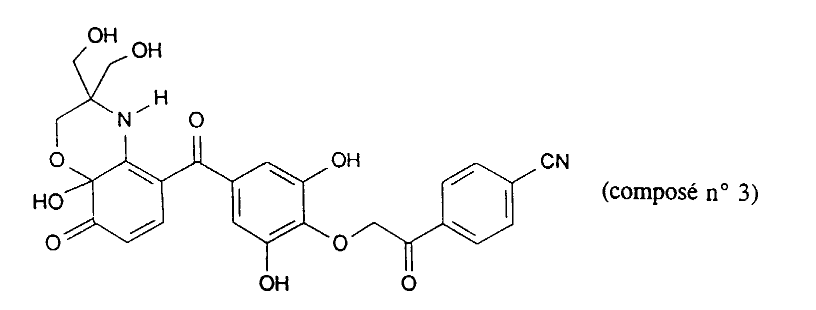

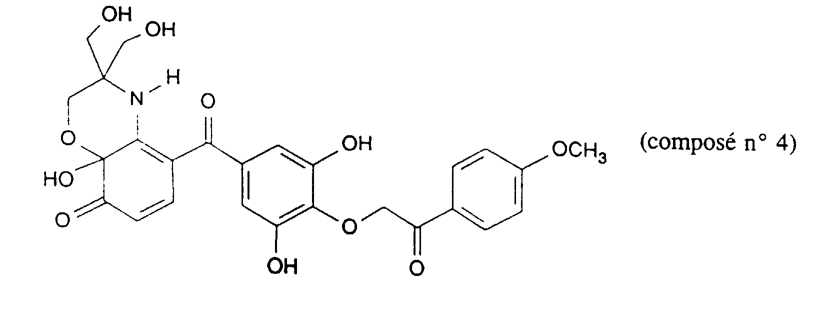

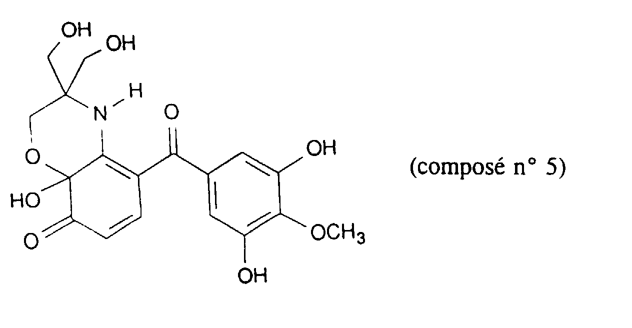

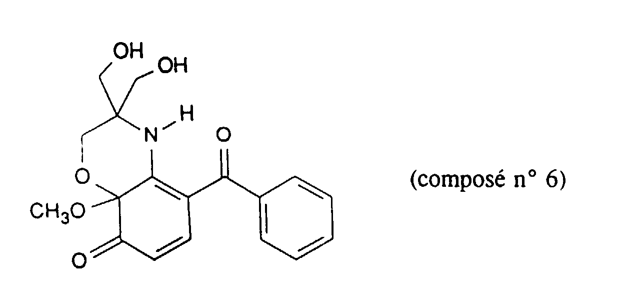

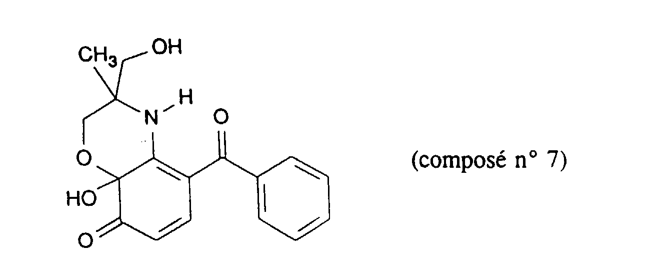

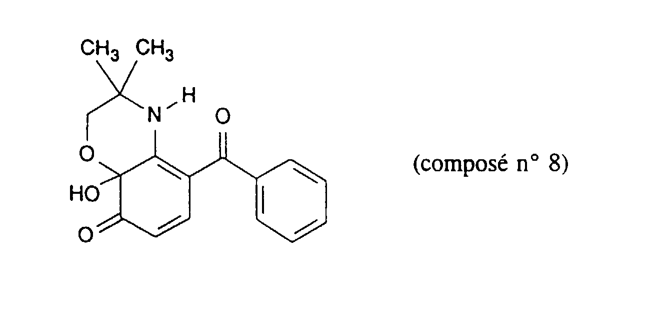

L'invention vise également plus particulièrement les composés correspondant à la formule (VI) suivante: 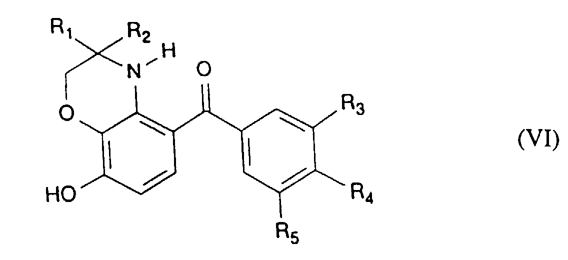 dans laquelle R₁, R₂, R₃, R₄ et R₅ sont tels que définis ci-dessus.

Des composés de formule (VI) préférés dans le cadre de la présente invention sont ceux correspondant aux formules suivantes:

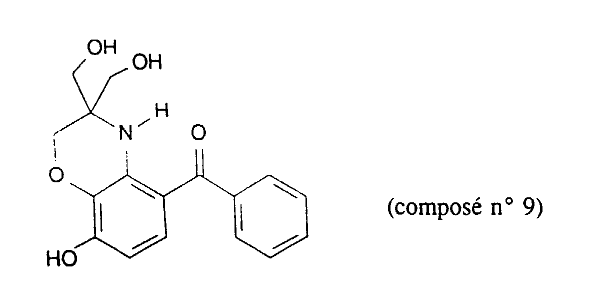

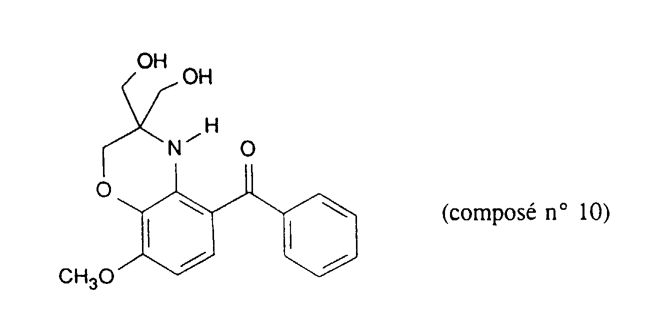

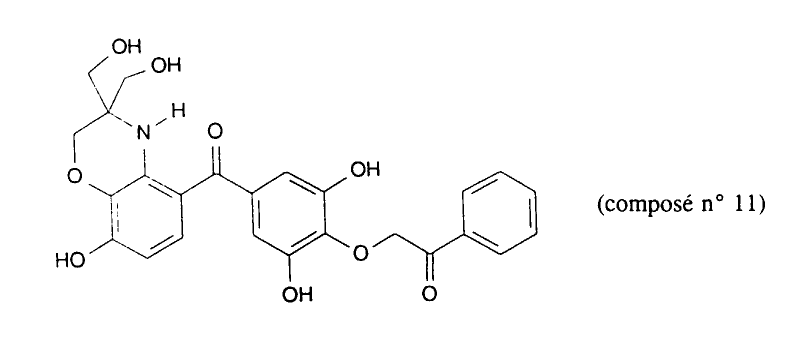

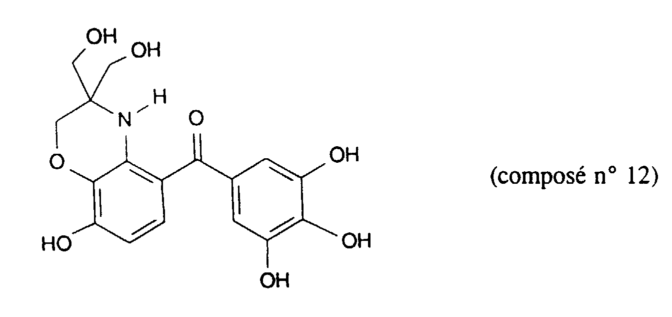

D'autres composés préférés dans le cadre de la présente invention sont ceux correspondant à la formule (VII) suivante: 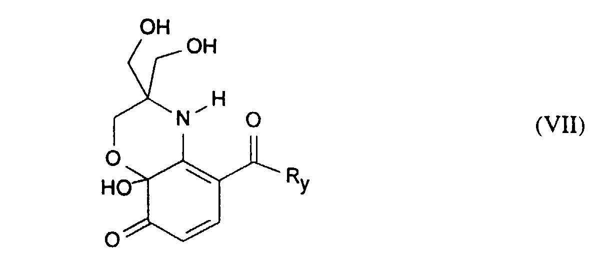 dans laquelle R_{y} représente:
- un groupe méthyle (composé n° 13), ou
- un groupe méthoxy (composé n° 14), ou
- un groupe -CH₂Cl (composé n° 15)

L'invention a également pour objet toute composition pharmaceutique, caractérisée en ce qu'elle comprend, à titre de principe actif, au moins un composé selon l'invention, choisi parmi ceux décrits ci-dessus, en association avec un véhicule pharmaceutiquement acceptable.

Les compositions pharmaceutiques selon l'invention se présentent généralement sous une forme administrable par voie orale, notamment sous forme de comprimés, ou de gélules, ou sous une forme administrable par voie parentérale, notamment sous forme de préparation injectable par voie intraveineuse, intramusculaire, ou sous-cutanée.

Avantageusement, la posologie journalière pour les formes orales est d'environ 3 mg/kg à environ 20 mg/kg, de préférence environ 15 mg/kg, et la posologie journalière pour les formes parentérales est d'environ 1 mg/kg à environ 5 mg/kg.

De préférence, les doses unitaires de principe actif en fonction des différentes formes de présentation des compositions pharmaceutiques selon l'invention, sont les suivantes:
- forme orale: environ 1 mg/kg à environ 10 mg/kg, avantageusement environ 5 mg/kg,
- forme parentérale: environ 0,3 mg/kg à environ 1 mg/kg.

L'invention concerne plus particulièrement l'utilisation d'au moins un composé selon l'invention, pour l'obtention d'un médicament destiné au traitement des affections, notamment celles citées ci-dessus, contre lesquelles les benzophénones en général, et l'Exifone en particulier, sont susceptibles d'être actives.

Les médicaments selon l'invention sont plus particulièrement destinés à être utilisés dans le cadre du traitement des troubles cognitifs, tels que les troubles de la mémoire, notamment chez les sujets vieillissants.

Avantageusement, les médicaments selon l'invention sont destinés à être utilisés dans le cadre du traitement des affections cérébrales susceptibles d'être dues, et/ou entretenues, voire aggravées, par la présence de radicaux libres dans le cerveau, et plus particulièrement au traitement de la maladie d'Alzheimer, de la maladie de Parkinson, de la Trisomie 21 (syndrome de Down), de la schizophrénie et de l'épilepsie.

L'invention a également pour objet les procédés de préparation des composés susmentionnés.

Un procédé de préparation des composés selon l'invention, comprend les étapes suivantes:
a) traitement du dérivé de formule (VIII) suivante: 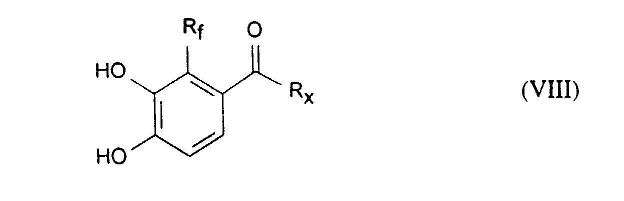 dans laquelle R_{f} représente H ou OH, et Rₓ est tel que défini ci-dessus,
   . soit par voie chimique par mise en présence dudit dérivé de formule (VIII) avec un dérivé de formule NH₂-C(R₁,R₂)-CH₂OH dans laquelle R₁ et R₂ sont tels que définis ci-dessus, en présence d'un oxyde de métal, choisi notamment parmi Ag₂O, AgO et PtO₂, en milieu alcoolique, de préférence méthanolique,
   . soit par voie électrochimique par électrolyse à potentiel contrôlé dudit dérivé de formule (VIII) en présence d'un dérivé de formule NH₂-C(R₁,R₂)-CH₂OH tel que décrit ci-dessus, en solution alcoolique, de préférence méthanolique, et en présence d'un électrolyte support, choisi notamment parmi les sels de tétraéthylammonium,
   ce qui conduit à l'obtention d'un composé de formule (II) suivante: 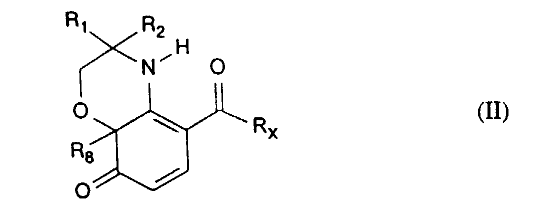 dans laquelle R₈ représente un groupe -OH, et R₁, R₂ et Rₓ sont tels que définis ci-dessus,
   sous réserve que lorsque Rₓ du dérivé de la formule (VIII) susmentionnée, représente un groupe de formule 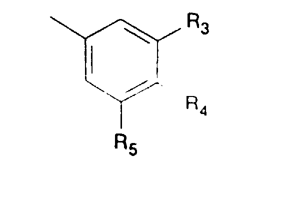 dans laquelle R₄ représente un groupe -OH, et l'un au moins des groupements R₃ ou R₅ représente un groupe -OH, ce qui correspond au dérivé de formule (VIII bis), l'étape de traitement chimique ou électrochimique décrite ci-dessus est précédée par une étape de protection du groupe -OH correspondant à R₄ dudit dérivé de formule (VIII bis), cette étape de protection étant réalisée par traitement du dérivé de formule (VIII bis) avec un dérivé de formule X¹-CH₃ ou X¹-R_{c} dans laquelle X¹ représente un atome d'halogène, notamment un atome d'iode ou de brome, et R_{c} est tel que défini ci-dessus,
   ce qui conduit à l'obtention d'un dérivé de formule (VIII ter) correspondant à un dérivé de formule (VIII bis), dans laquelle l'un au moins de R₃ ou de R₅ représente -OH, tandis que R₄ représente un groupe -OCH₃ ou -OR_{c}, R_{c} ayant la définition indiquée ci-dessus, ce dérivé de formule (VIII ter) étant ensuite traité par voie chimique ou électrochimique de la manière indiquée ci-dessus par un dérivé de formule NH₂-C(R₁,R₂)-CH₂OH en milieu alcoolique, ce qui conduit à l'obtention d'un composé de formule (II) susmentionnée dans laquelle R₈ représente un groupe -OH, et R₁ et R₂ sont tels que définis ci-dessus, et Rₓ représente un groupe de formule 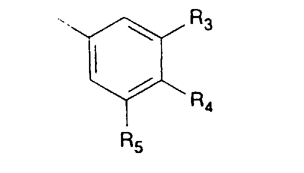 dans laquelle l'un au moins de R₃ ou de R₅ représente -OH, R₄ représente -OCH₃ ou -OR_{c}, R_{c} étant défini ci-dessus,
b) le cas échéant,
   - soit alkylation du composé de formule (II) obtenu à l'étape a) précédente, par traitement de ce composé en milieu basique par un dérivé de formule X²-CH₃, dans laquelle X² représente un atome d'halogène, notamment l'iode, ce qui conduit à l'obtention d'un composé de formule (II) susmentionnée dans laquelle R₈ représente un groupe -OCH₃, et R₁, R₂ et Rₓ sont tels que définis ci-dessus,
   - soit réduction du composé de formule (II) obtenu à l'étape a) précédente:
      . soit par voie chimique par traitement du composé de formule (II) par une solution d'acide acétique en présence de Zn (avantageusement environ cinq équivalents de Zn) à température ambiante, ce qui conduit à l'obtention d'un composé de formule (III) suivante: 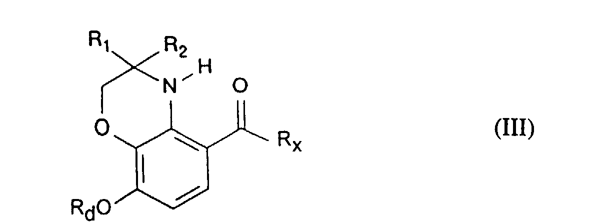 dans laquelle R_{d} représente H, et R₁, R₂, et Rₓ sont tels que définis ci-dessus, sous réserve que lorsque le composé de formule (II) susmentionnée est tel que Rₓ représente un groupe de formule suivante: 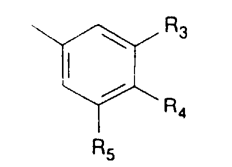 dans laquelle R₄ représente un groupe -OR_{c}, R_{c} étant tel que défini ci-dessus, et R₃ et R₅ étant tels que définis ci-dessus, la réduction par voie chimique susmentionnée est réalisée soit avec environ cinq équivalents de zinc, le temps de contact entre ledit composé de formule (II) et le zinc étant d'environ 2 minutes, ce qui conduit à l'obtention d'un composé de formule (III) susmentionnée, dans laquelle R₄, dans la définition de Rₓ, représente un groupe -OR_{c}, R_{c} étant tel que défini ci-dessus, et R₃ et R₅ étant tels que définis ci-dessus, soit avec environ cent équivalents de zinc, le temps de contact entre ledit composé de formule (II) et le zinc étant d'environ 10 minutes, ou avantageusement avec environ cinq équivalents du zinc, le temps de contact susmentionné étant alors d'environ 30 minutes, ce qui conduit, dans ces deux derniers cas, à l'obtention d'un composé de formule (III) susmentionnée dans laquelle R₄, dans la définition de Rₓ, représente un groupe -OH, et R₃ et R₅ étant tels que définis ci-dessus,
      . soit par voie électrochimique, par électrolyse à potentiel contrôlé du composé de formule (II) susmentionnée en solution alcoolique, de préférence méthanolique, en présence d'un électrolyte support, choisi notamment parmi les sels de tétraéthylammonium, ce qui conduit à l'obtention du composé de formule (III) susmentionnée,
   sous réserve que lorsque le composé de formule (II) susmentionnée est tel que Rₓ représente un groupe de formule suivante: 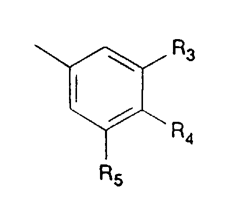 dans laquelle R₄ représente un groupe -OR_{c}, R_{c} étant tel que défini ci-dessus, et R₃ et R₅ étant tels que définis ci-dessus, le potentiel d'électrolyse est choisi soit de manière à obtenir un composé de formule (III) susmentionnée, dans laquelle R₄, dans la définition de Rₓ, représente un groupe -OR_{c}, R_{c} étant tel que défini ci-dessus, et R₃ et R₅ étant tels que définis ci-dessus (ledit potentiel d'électrolyse étant avantageusement choisi entre -0,9 V e.c.s. et -1,1 V e.c.s.), soit de manière à obtenir un composé de formule (III) susmentionnée dans laquelle R₄, dans la définition de Rₓ, représente un groupe -OH, et R₃ et R₅ étant tels que définis ci-dessus (ledit potentiel d'électrolyse étant alors avantageusement choisi entre -1,3 V e.c.s. et -1,6 V e.c.s.),
c) le cas échéant, alkylation du composé de formule (III) obtenu à l'étape b) précédente, par traitement de ce composé en milieu basique par un dérivé de formule X³-CH₃, dans laquelle X³ représente un atome d'halogène, notamment l'iode, ce qui conduit à l'obtention d'un composé de formule (III) susmentionnée dans laquelle R_{d} représente un groupe -OCH₃, et R₁, R₂ et Rₓ sont tels que définis ci-dessus.

S'agissant des dérivés de formule (VIII) de départ du procédé d'obtention décrit ci-dessus des composés de l'invention, ces dérivés peuvent être obtenus, lorsque le groupe Rₓ dans la formule (VIII) représente un groupe de formule: 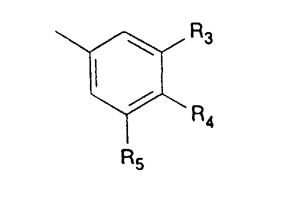 dans laquelle R₃, R₄ et R₅, indépendemment l'un de l'autre, représentent des atomes d'hydrogène, ou un groupe -ORg, Rg représentant un atome d'hydrogène ou un groupe méthyle,
par réaction d'un acide carboxylique aromatique, dont le noyau est substitué ou non par des groupements hydroxyles, sur des phénols en présence de catalyseurs tels que le chlorure d'aluminium anhydre (réaction de Friedel et Craft), le chlorure de zinc anhydre seul ou avec l'oxychlorure de phosphore, le trifluorure de bore, par la réaction dite de Fries, notamment selon le schéma réactionnel suivant: 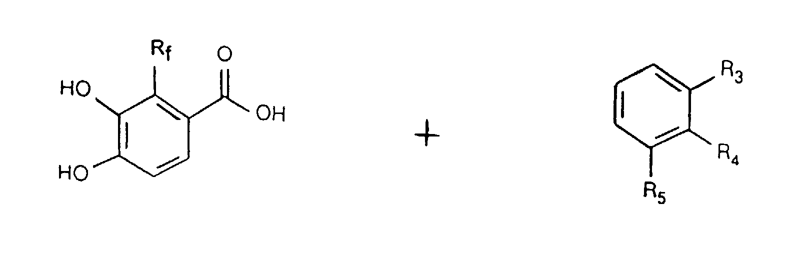 R_{f} représentant H ou OH, R₃, R₄ ou R₅ étant tels que définis ci-dessus, l'un de R₃, R₄ ou R₅ représentant obligatoirement OH, 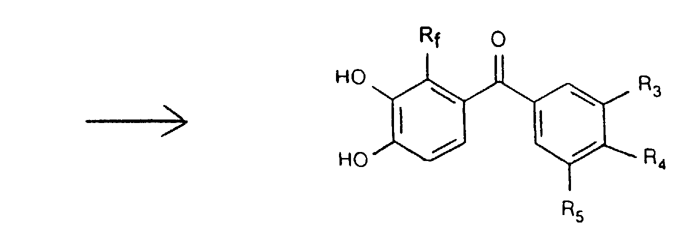 ou selon le schéma réactionnel suivant: 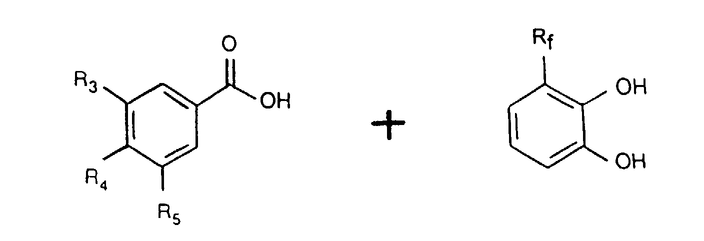 R_{f}, R₃, R₄ ou R₅ indépendamment les uns des autres, représentant H ou un groupe -OH.

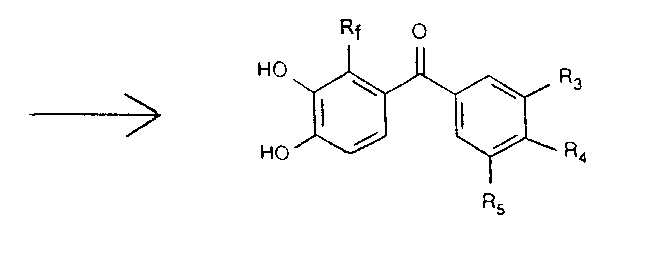

Des méthodes de préparation des dérivés de formule (VIII) susmentionnée sont décrites plus en détail dans le brevet français n° 2 257 272 cité plus haut, ou encore dans la description détaillée qui suit de l'invention.

S'agissant des dérivés de formule (VIII) susmentionnée, dans laquelle R_{f} représente H ou un groupe -OH, et Rₓ représente un groupe alkyle de 1 à 3 atomes de carbone, notamment un groupe méthyle, ou un groupe de formule -CH₂-X⁴ dans laquelle X⁴ représente un atome d'halogène, notamment le chlore, ceux-ci sont, soit des produits commerciaux, disponibles notamment chez Aldrich, soit des produits dont la synthèse est indiquée dans la description détaillée qui suit de l'invention.

Enfin, s'agissant des dérivés de formule (VIII) susmentionnée dans laquelle R_{f} représente H ou un groupe -OH, et Rₓ représente un groupe -ORₓ₁ Rₓ₁ représentant un groupe alkyle de 1 à 3 atomes de carbone, notamment un groupe méthoxy, ces derniers sont obtenus par estérification des dérivés de formule: 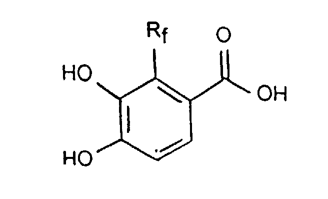 dans laquelle R_{f} représente H ou un groupe -OH (commercialisés par Aldrich), plus particulièrement par traitement de ces derniers dérivés avec un composé de formule X⁵-Rₓ₁, Rₓ₁ étant tel que défini ci-dessus, et X⁵ représentant un atome d'halogène, notamment à l'aide du chlorure de thionyle selon la méthode décrite plus en détail dans la description détaillée qui suit de l'invention.

L'invention vise également les nouveaux produits intermédiaires de formule (VIII) en tant que tels.

A ce titre, l'invention concerne plus particulièrement les dérivés de formule (VIII) suivants:

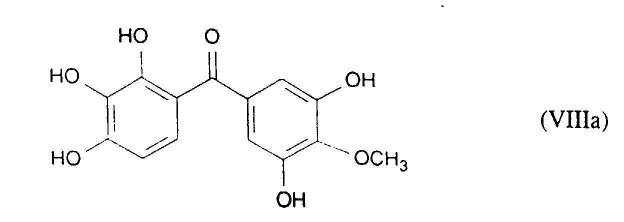

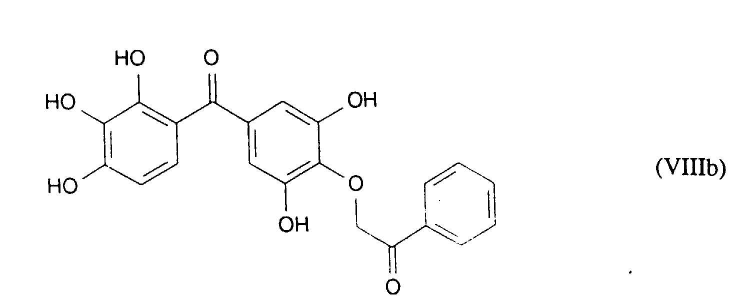

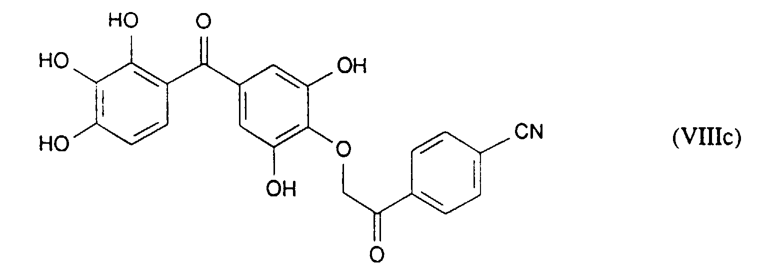

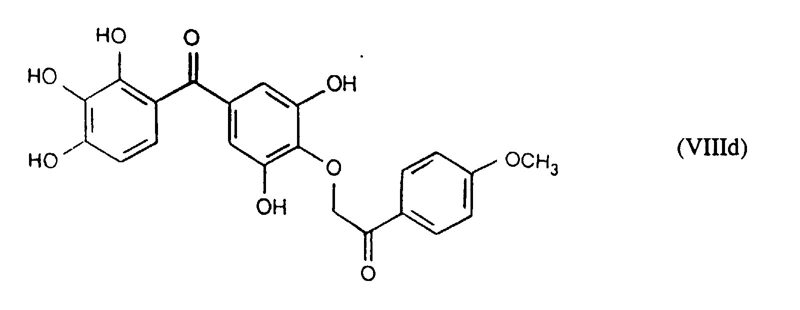

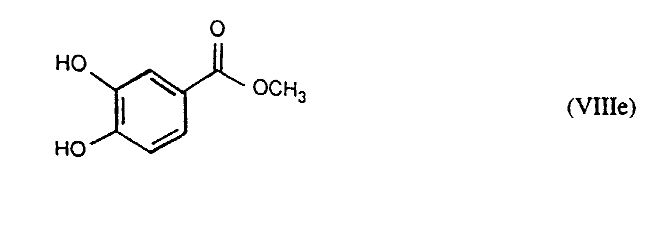

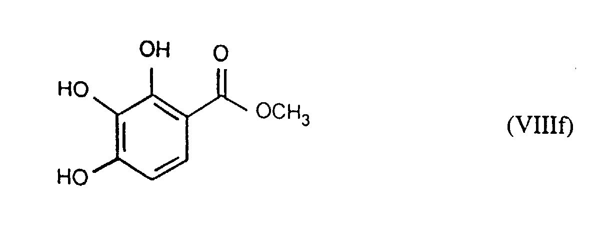

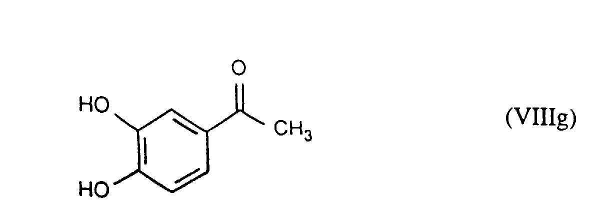

Les dérivés de formule NH₂-C(R₁,R₂)-CH₂OH dans laquelle R₁ et R₂ sont tels que définis ci-dessus, utilisés dans le procédé de préparation susmentionné des composés de l'invention, sont des produits commerciaux, disponibles notamment chez Aldrich.

La présente invention sera davantage illustrée à l'aide des exemples qui suivent, donnés à titre non limitatif, de la préparation de composés de l'invention et de l'étude pharmacologique réalisée à partir de certains de ces composés.

### I. Exemples de préparation de composés selon l'invention

Dans ces exemples, les spectres de RMN ont été réalisés à 300 MHz (spectre proton) et 75 MHz (spectre carbone 13) dans le deutérodiméthylsulfoxyde à l'aide d'un spectromètre Brüker AC 300; les déplacements chimiques sont exprimés en ppm et les constantes de couplage en Hertz; s désigne un singulet, d un doublet, t un triplet, dd un doublet de doublet, et m un multiplet. Les spectres de masse ont été enregistrés sur un spectromètre Nermag R10-10C (Ionisation Chimique, gaz réactant: ammoniac). Les chromatographies sur colonne ont été réalisées à la pression atmosphérique en utilisant une silice de granulométrie 40-63 µm. Les points de fusion ont été déterminés au moyen d'un banc de Köfler.

### A) Synthèse des composés de formule générale (VIII).

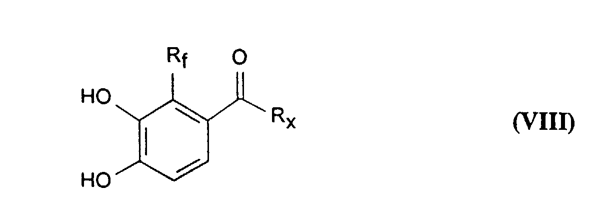

### Exemple 1. Synthèse de la [2,3,4-trihydroxyphényl] [3',5'-dihydroxy-4'-méthoxyphényl] méthanone (composé VIIIa).

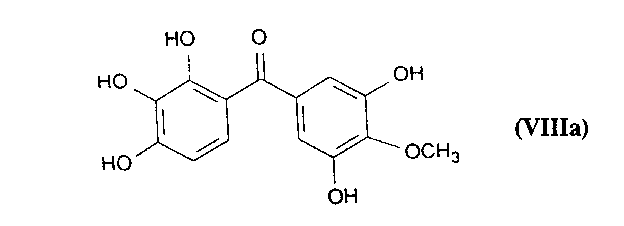

A une solution de 5,0 g de [2,3,4-trihydroxyphényl] [3',4',5'-trihydroxyphényl] méthanone dans 25 cm³ de diméthylformamide maintenue à 25°C, sous atmosphère d'azote, on ajoute 3,0 g de carbonate de lithium puis 30 cm³ d'iodure de méthyle. Le mélange réactionnel est ensuite agité vingt heures à 25°C. On ajoute alors 500 cm³ d'une solution de tampon acéto-acétique molaire dont le pH est voisin de 4,50. La solution résultante est extraite avec 800 cm³ d'acétate d'éthyle. Les phases organiques réunies sont lavées par 300 cm³ d'eau distillée, puis séchées sur du sulfate de sodium anhydre. Après filtration, puis évaporation à sec sous pression réduite (2,7 kPa) à 40°C de l'acétate d'éthyle, on obtient 4,5 g d'un résidu qui est purifié par chromatographie sur un fritté qui contient 25 g de gel de silice [éluant: dichlorométhane/acétone, (90/10 en volumes)] en recueillant des fractions de 15 cm³. Les fractions contenant le mélange des deux dérivés monométhylés en position -4 et -4' sont concentrées à sec sous pression réduite (2,7 kPa), à une température voisine de 40°C. On obtient, après cristallisation dans un mélange dichlorométhane/acétone (95/5 en volumes), 1,1 g de [2,3,4-trihydroxyphényl] [3',5'-dihydroxy-4'-méthoxyphényl] méthanone sous forme d'une poudre jaune pâle fondant aux alentours de 205°C (avec décomposition).

Spectre de RMN ¹H: δ 3,75 (s, 3H, OCH₃); 6,40 [d, 1H, H(5), J = 9Hz]; 6,60 [s, 2H, H(2') et H(6')]; 7,00 [d, 1H, H(6), J = 9Hz]; 8,70 [s, 1H, OH(3), échange D₂O]; 9,50 [s, 2H, OH(3') et OH(5'), échange D₂O]; 10,30 [s, 1H, OH(4), échange D₂O]; 12,00 [s, 1H, OH(2), échange D₂O].

Spectre de RMN ¹³C: δ 60,8 (OCH₃); 108,5 [CH(5)]; 109,6 [CH(2') et CH(6')]; 113,8 (C-1); 125,9 [CH(6)]; 133,7 (C-3); 134,2 (C-4'); 139,6 (C-1'); 151,5 (C-3' et C-5'); 153,2 et 153,4 (C-2 et C-4); 200,00 (CO, méthanone).

Spectre de masse (I.C.): m/z = 293 (MH⁺).

### Exemple 2. Synthèse de la [2,3,4-trihydroxyphényl] [3',5'-dihydroxy-4'-(2-oxo-2-phényléthoxy)phényl] méthanone (composé VIIIb).

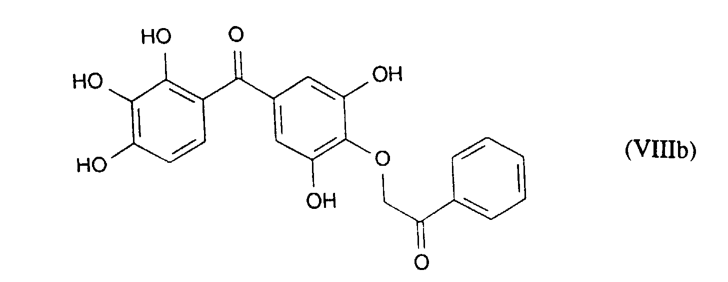

A une solution de 5,0 g de [2,3,4-trihydroxyphényl] [3',4',5'-trihydroxyphényl] méthanone dans 20 cm³ de diméthylformamide maintenue à 50°C, sous atmosphère d'azote, on ajoute 3,0 g de carbonate de lithium puis 3,5 g d'ω-bromoacétophénone. Le mélange réactionnel est ensuite agité six heures à 50°C. On ajoute alors 400 cm³ d'une solution de tampon acéto-acétique molaire dont le pH est voisin de 4,50. La solution résultante est extraite avec 800 cm³ d'acétate d'éthyle. Les phases organiques réunies sont lavées par 300 cm³ d'eau distillée, puis séchées sur sulfate de sodium anhydre. Après filtration puis évaporation à sec sous pression réduite (2,7 kPa) à 40°C de l'acétate d'éthyle, on obtient 9 g d'un résidu qui est purifié par chromatographie sur un fritté qui contient 50 g de gel de silice [éluant: dichlorométhane/acétone (90/10 en volumes)] en recueillant des fractions de 30 cm³. Les fractions contenant le mélange de dérivés monoacétylés en position -4 et 4' sont concentrées à sec sous pression réduite (2,7 kPa), à une température voisine de 40°C. On obtient, après cristallisation dans un mélange dichlorométhane/acétone (98/2 en volumes), 2,6 g de [2,3,4-trihydroxyphényl] [3',5'-dihydroxy-4'-(2-oxo-2-phényléthoxy)phényl] méthanone sous forme d'une poudre jaune fondant aux alentours de 180°C (avec décomposition).

Spectre de RMN ¹H: δ 4,00 (d, 1H, CH₂, J = 12Hz); 4,10 (d, 1H, CH₂, J = 12 Hz); 6,40 [d, 1H, H(5), J = 9Hz]; 6,75 [d, 1H, H(2') ou H(6'), J = 1,7Hz]; 6,80 [d, 1H, H(2') ou H(6'), J = 1,7Hz]; 7,02 [d, 1H, H(6), J = 9Hz]; 7,40 à 7,60 [m, 5H, aromatiques (phényléthoxy)]; 8,70 [s, 1H, OH(3), échange D₂O]; 9,70 [s, 2H, OH(3') et OH(5'), échange D₂O]; 10,20 [s, 1H, OH(4), échange D₂O]; 12,60 [s, 1H, OH(2), échange D₂O].

Spectre de RMN ¹³C: δ 71,3 (CH₂); 95,2 (Cq, phényléthoxy); 108,6 [CH(5)]; 110,9 [CH(2') et CH(6')]; 114,1(C-1); 125,7 [CH(6)]; 127,5 [CH(méta), phényléthoxy]; 129,4 [CH(ortho), phényléthoxy]; 130,0 [CH(para), phényléthoxy]; 131,4 (C4'); 133,8 (C-3); 135,9 (C-1'); 141,0 et 143,5 (C-3' et C-5'), 147,0 (cétone, phényléthoxy); 153,0 (C-2 et C-4); 199,0 (CO, méthanone).

Spectre de masse (I.C.): m/z = 397 (MH⁺)

### Exemple 3. Synthèse de la [2,3,4-trihydroxyphényl] [3',5'-dihydroxy-4'-(2-oxo-2-p. cyanophényléthoxy)phényl] méthanone (composé VIIIc).

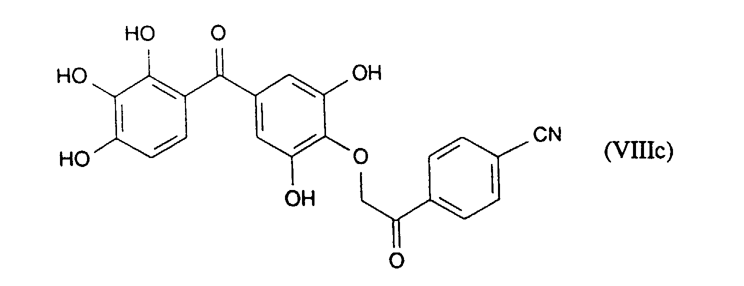

En opérant comme dans l'exemple 2, mais en remplaçant l'ω-bromoacétophénone par 4,0 g d'ω-bromo4-méthoxyacétophénone on obtient, après chromatographie sur fritté, 3,0 g de mélange de dérivés monoacétylés en position -4 et -4'. Une recristallisation dans l'acétone permet d'isoler 2,0 g de [2,3,4-trihydroxyphényl] [3',5'-dihydroxy-4'-(2-oxo-2-p.cyanophényléthoxy) phényl] méthanone, sous forme d'une poudre jaune pâle fondant au dessus de 280°C.

Spectre de RMN ¹H: δ 4,05 (d, 1H, CH₂, J = 12Hz); 4,25 (d, 1H, CH₂, J = 12Hz); 6,40 [d, 1H, H(5), J = 9Hz]; 6,70 et 6,75 [s, 2H, H(2') et H(6')]; 7,00 [d, 1H, H(6), J = 9Hz]; 7,80 [d, 2H, CH(en γ du CN), p.cyanophényléthoxy, J = 9Hz); 7,95 [d, 2H, CH (en β du CN), p.cyanophényléthoxy, J = 9Hz]; 8,70 [s, 1H, OH(3), échange D₂O]; 9,80 [s, 2H, OH(3') et OH(5'), échange D₂O]; 10,20 [s, 1H, OH(4) échange D₂O]; 11,90 [s, 1H, OH(2), échange D₂O].

Spectre de RMN ¹³C: δ 70,7 (CH₂); 94,9 [Cq(en δ du CN), p.cyanophényléthoxy]; 108,5 [CH(5)]; 110,7 et 111,0 [CH(2') et CH(6')]; 112,9 [Cq(en α du CN), p.cyanophényléthoxy] ; 114,1 (C-1); 119,6 (CN); 125,7 [CH(6)]; 128,6 [CH(en γ du CN), p. cyanophényléthoxy]; 131,5 (C-4'); 133,5 [CH(en β du CN), p.cyanophényléthoxy]; 133,8 (C-3); 135,7 (C-1'); 143,1 (CO, p.cyanophényléthoxy); 146,0 et 147,0 (C-3' et C-5'); 153,0 et 153,2 (C-2 et C4); 199,0 (CO, méthanone).

Spectre de masse(I.C.): m/z = 422 (MH⁺)

### Exemple 4. Synthèse de la [2,3,4-trihydroxyphényl] [3',5'-dihydroxy-4'-(2-oxo-2-p. méthoxyphényléthoxy)phényl] méthanone (composé VIIId).

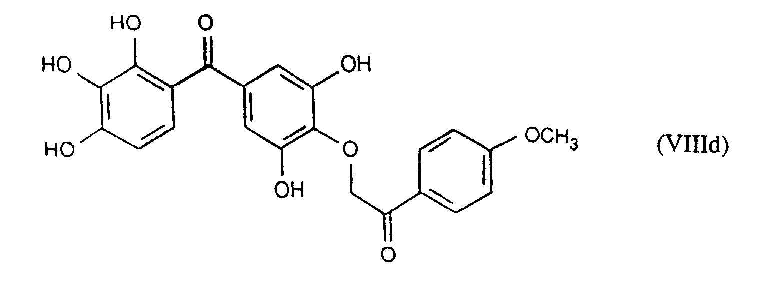

En opérant comme dans l'exemple 2, mais en remplaçant l'ω-bromoacétophénone par 4,1 g d'ω-bromo-4-méthoxyacétophénone on obtient, après chromatographie sur fritté, 3,0 g de mélange de dérivés monoacétylés en position -4 et -4'. Une recristallisation dans un mélange dichlorométhane/acétone (90/10 en volumes) permet d'isoler 2,0 g de [2,3,4-trihydroxyphényl] [3',5'-dihydroxy-4'-(2-oxo-2-p.méthoxyphényléthoxy) phényl] méthanone, sous forme d'une poudre jaune pâle.

Spectre de RMN ¹H: δ 3,75 (OCH₃); 3,95 (d, 1H, CH₂, J = 12Hz); 4,15 (d, 1H, CH₂, J = 12Hz); 6,40 [d, 1H, H(5), J = 9Hz]; 6,70 et 6,75 [s, 2H, H(2') et H(6')]; 6,95 [d, 2H, H(en β du OCH₃), p.méthoxyphényléthoxy]; 7,00 [d, 1H, H(6), J = 9Hz]; 7,50 [d, 2H, H(en γ du OCH₃), p.méthoxyphényléthoxy, J = 9Hz]; 8,75 [s, 1H, OH(3), échange D₂O]; 9,70 [s, 2H, OH(3') et OH(5'), échange D₂O]; 10,10 [s, 1H, OH(4), échange D₂O]; 12,00 [s, 1H, OH(2), échange D₂O].

Spectre de masse (I.C.): m/z = 427 (MH⁺).

### Exemple 5. Synthèse du 3,4-dihydroxyphényl-1-carboxylate de méthyle (composé VIIIe)

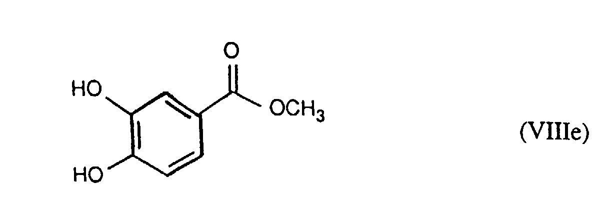

A une solution de 1,00 g d'acide 3,4-dihydroxybenzoïque dans 5 cm³ de méthanol, on ajoute 0,85 g de chlorure de thionyle. Le mélange réactionnel est ensuite agité cinq heures à 60°C. Le méthanol est alors évaporé à sec, sous pression réduite (2,7 kPa), à une température voisine de 40°C. Le résidu est repris par 20 cm³ d'une solution de tampon acéto-acétique molaire dont le pH est voisin de 4,5. La solution résultante est alors extraite avec 50 cm³ d'acétate d'éthyle. Les phases organiques réunies sont séchées sur du sulfate de sodium anhydre, puis filtrées. L'acétate d'éthyle est évaporé à sec sous pression réduite (2,7 kPa), à une température voisine de 40°C. On obtient 1,0 g de 3,4-dihydroxyphényl-1-carboxylate de méthyle sous forme d'une poudre blanche fondant aux alentours de 140°C.

Spectre de RMN ¹H: δ 3,8 (s, 3H, CH₃); 6,8 [d, 1H, H(5), J = 8Hz]; 7,3 [dd, 1H, H(6), J = 8Hz, J = 2Hz]; 7,4 [d, 1H, H(2), J = 2Hz]; 9,6 [large s, 2H, OH(3) et OH(4), échange D₂O].

Spectre de RMN ¹³C: δ 52,7 (CH₃); 116,4 et 117,4 [CH(2) et CH(5)]; 121,6 (C-1); 122,9 [CH(6)]; 146,2 (C-3); 151,5 (C4); 167,3 (CO, carboxylate de méthyle).

Spectre de masse (I.C.): m/z = 169 (MH⁺).

### Exemple 6. Synthèse du 2,3,4-trihydroxyphényl-1-carboxylate de méthyle (composé VIIIf)

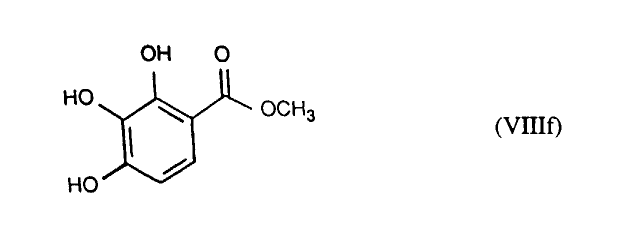

On opère de manière analogue à celle décrite dans l'exemple 5, mais à partir de 1,0 g d'acide 2,3,4-trihydroxybenzoïque et de 0,8 g de chlorure de thionyle. On obtient 0,8 g de 2,3,4-trihydroxyphényl-1-carboxylate de méthyle sous forme d'une poudre blanche fondant aux alentours de 160°C.

Spectre de RMN ¹H: δ 3,8 (s, 3H, CH₃); 6,4 [d, 1H, H(5), J = 9Hz]; 7,2 [d, 1H, H(6), J = 9Hz]; 8,6 et 9,9 [large s, 2H, OH(3) et OH(4), échange D₂O]; 10,7 [large s, 1H, OH(2), échange D₂O].

Spectre de RMN ¹³C: δ 53,2 (CH₃); 105,5 (C-1); 108,9 [CH(5)]; 121,8 [CH(6)]; 133,7 (C-3); 152,0 et 152,9 (C-2 et C4); 171,2 (CO, carboxylate de méthyle).

Spectre de masse (I.C.): m/z = 185 (MH⁺).

### Exemple 7. Synthèse de la 3,4-dihydroxyacétophénone (composé VIIIg)

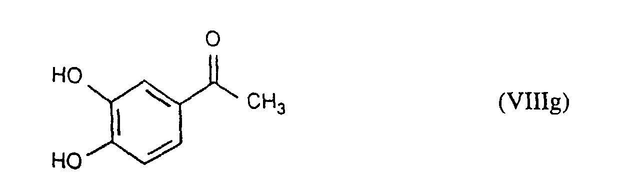

On procède à l'électrolyse à potentiel contrôlé de 200 cm³ d'une solution hydroalcoolique (tampon acéto-acétique molaire, pH 4,5/méthanol, 50/50 en volumes), de 0,47 g de ω-chloro-3,4-dihydroxyacétophénone, sous atmosphère d'azote, à 25°C, à une électrode de mercure dont le potentiel est fixé à -1,0 V e.c.s. A la fin de l'électrolyse, lorsque l'intensité du courant est devenue négligeable (0,5 mA) devant l'intensité du courant initial (70 mA), la solution est alors concentrée à 100 cm³, sous pression réduite (2,7 kPa), à une température voisine de 40°C. La solution résultante est extraite avec 250 cm³ d'acétate d'éthyle. Les phases organiques réunies sont séchées sur du sulfate de sodium anhydre, filtrées et le solvant évaporé à sec, sous pression réduite (2,7 kPa), à une température voisine de 40°C. On obtient alors 0,36 g de 3,4-dihydroxyacétophénone sous forme d'une poudre blanche fondant aux alentours de 120°C.

Spectre de RMN ¹H: δ 2,5 (s, 3H, CH₃); 6,8 [d, 1H, H(5), J = 9Hz]; 7,3 [s, 1H, H(2)]; 7,4 [d, 1H, H(6), J = 9Hz]; 9,0 [large s, 2H, OH(3) et OH(4), échange D₂O].

Spectre de RMN ¹³C: δ 27,3 (CH₃); 116,0 et 116,1 [CH(2) et CH(5)]; 122,8 [CH(6)]; 130,0 (C-1); 146,3 (C-3); 151,9 (C4), 197,0 (CO, acétophénone).

Spectre de masse (I.C.): m/z = 153 (MH⁺).

### B) Synthèse des composés de formule générale II.

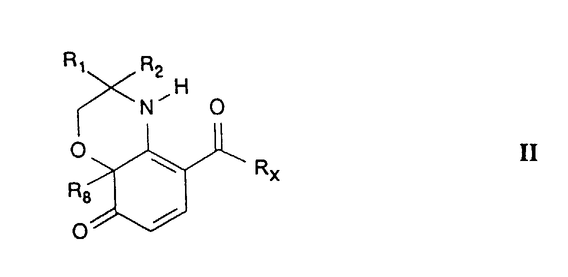

### Exemple 8. Synthèse de la (RS)-5-benzoyl-3,3-bis (hydroxyméthyl)-8a-hydroxy-3,4-dihydro-2H-1,4-benzoxazin-8(8aH)-one (composé n° 1).

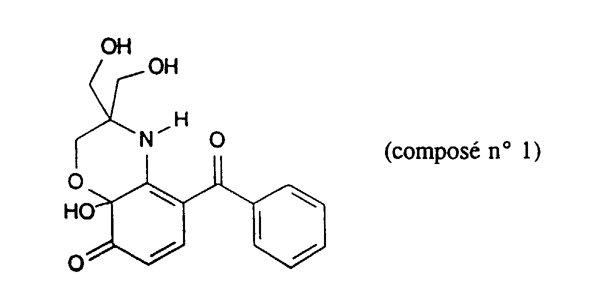

### a) Synthèse par voie chimique

0,46 g de 2,3,4-trihydroxybenzophénone est dissous dans 1 litre d'une solution méthanolique contenant 12,10 g de TRIS, agitée sous atmosphère d'azote, à la température ambiante. On ajoute ensuite 2,30 g d'oxyde d'argent (Ag₂O). Le mélange réactionnel est agité 2 heures, puis filtré; le méthanol est alors évaporé à sec, sous pression réduite (2,7 kPa), à une température voisine de 40°C. Le résidu est repris par 200 cm³ d'une solution de tampon acéto-acétique molaire, dont le pH est voisin de 4,50. La solution résultante est alors extraite avec 400 cm³ d'acétate d'éthyle. Les phases organiques réunies sont séchées sur du sulfate de sodium anhydre, puis filtrées; l'acétate d'éthyle est évaporé à sec sous pression réduite (2,7 kPa), à une température voisine de 40°C. Le résidu est repris par 2 cm³ d'acétate d'éthyle et la solution obtenue est déposée sur une colonne de 20 g de gel de silice (diamètre: 1,8 cm, hauteur 60 cm). La colonne est éluée avec un mélange acétate d'éthyle/méthanol (98/2 en volumes), en recueillant des fractions de 5 cm³. Les fractions contenant le produit attendu sont concentrées à sec sous pression réduite (2,7 kPa), à une température voisine de 40°C. On obtient 0,53 g de (RS)-5-benzoyl-3,3-bis (hydroxyméthyl)-8a-hydroxy-3,4-dihydro-2H-1,4-benzoxazin-8(8aH)-one sous forme d'une poudre orange, fondant aux alentours de 170°C (avec décomposition).

Spectre de RMN ¹H: δ 3,40 [dd, 1H, CH₂OH(3), J = 12Hz, J = 5Hz]; 3,50 [d, 2H, CH₂OH(3), J = 5Hz]; 3,60 [dd, 1H, CH₂OH(3), J = 12Hz, J = 5Hz]; 3,85 [d, 1H, CH₂(2), J = 12Hz]; 4,20 [d, 1H, CH₂(2), J = 12Hz]; 5,20 [d, 1H, H(7), J = 10Hz]; 5,40 [m, 2H, OH(alcool), échange D₂O]; 7,20 [d, 1H, H(6), J = 10Hz], 7,50 [m, 5H, aromatiques, benzoyl(5)]; 8,30 [s, 1H, OH(8a), échange D₂O]; 12,30 [s, 1H, NH, échange D₂O].

Spectre de RMN ¹³C: δ 56,5 (C-3); 58,3 [CH₂(2)]; 60,7 [CH₂OH(3)], 61,2 [CH₂OH(3)]; 86,6 (C-8a); 98,8 (C-5); 107,7 [CH(7)]; 127,5 [CH(méta), benzoyl(5)]; 128,2 [CH(ortho), benzoyl(5)]; 130,0 [CH(para), benzoyl(5)]; 139,4 [Cq, benzoyl(5)], 146,0 [CH(6)]; 167,1 (C-4a); 190,0 [CO(8)]; 192,6 [CO, benzoyl(5)].

Spectre de masse (I.C.): m/z = 332 (MH⁺).

### b) synthèse par voie électrochimique

b-1) L'électrolyse à potentiel contrôlé est réalisée au moyen d'un montage à trois électrodes. La cellule d'électrolyse est constituée par un ensemble de verreries rodées, les compartiments anodique et cathodique étant concentriques et séparés par une paroi de verre fritté de porosité 7. Un potentiostat-galvanostat Tacussel PJT 120V-1A et un intégrateur Tacussel IG6N complètent le circuit.

L'électrode de travail est une toile de platine irridié à 10%, de 25 mm de hauteur et de 60 mm de diamètre intérieur. L'électrode auxiliaire est une lame de platine. L'électrode de référence est une électrode au calomel à solution saturée de chlorure de potassium (e.c.s.)

On procède à l'électrolyse d'une solution de 0,115 g de 2,3,4-trihydroxy benzophénone dans 250 cm³ de méthanol contenant 3,020 g de Tris et 1,150 g de perchlorate de tétraéthylammonium comme électrolyte support, sous atmosphère d'azote, à 25°C, à une électrode de platine dont le potentiel est fixé à +0,4V e.c.s. A la fin de l'électrolyse, lorsque l'intensité du courant est devenue négligeable (0,5 mA) devant l'intensité du courant initial (60 mA), la solution résultante est évaporée à sec sous pression réduite (2,7 kPa) à une température voisine de 40°C. Le résidu est repris par 100 cm³ d'une solution de tampon acéto-acétique molaire dont le pH est voisin de 4,5. La solution résultante est alors extraite avec 200 cm³ d'acétate d'éthyle. Les phases organiques réunies sont séchées sur du sulfate de sodium anhydre, filtrées et le solvant évaporé à sec sous pression réduite (2,7 kPa), à une température voisine de 40°C. Le résidu est alors repris par 1 cm³ d'acétate d'éthyle et la solution obtenue est déposée sur une colonne de 10 g de gel de silice (diamètre 1,3 cm, hauteur 60 cm). la colonne est éluée avec un mélange acétate d'éthyle/méthanol (98/2 en volumes), en recueillant des fractions de 2 cm³. Les fractions contenant le produit attendu sont concentrées à sec sous pression réduite (2,7 kPa), à une température voisine de 40°C. On obtient 0,12 g de composé n° 1 dont les caractéristiques sont identiques à celles du produit obtenu au paragraphe a) ci-dessus.

b-2) En opérant comme dans le paragraphe b-1) ci-dessus, mais en remplaçant l'électrode de platine par une nappe de mercure dont le potentiel est fixé à 0,0 V e.c.s., on procède à l'électrolyse de 0,115 g de 2,3,4-trihydroxybenzophénone. On obtient 0,100 g de composé n° 1 dont les caractéristiques sont identiques à celles du produit obtenu au paragraphe a) ci-dessus.

b-3) Le composé n° 1 peut également être obtenu en opérant comme dans le paragraphe b-1) ci-dessus, mais à partir de 0,11 g de 3,4-dihydroxybenzophénone. On obtient ainsi 0,08 g de composé n° 1 dont les caractéristiques sont identiques à celles du produit obtenu au paragraphe a) ci-dessus.

### Exemple 9. Synthèse de la (RS)-5-[3,5-dihydroxy-4-(2-oxo-2-phényléthoxy)]-benzoyl-3,3-bis(hydroxyméthyl)-8a-hydroxy-3,4-dihydro-2H-1,4-benzoxazin-8(8aH)-one (composé n° 2).

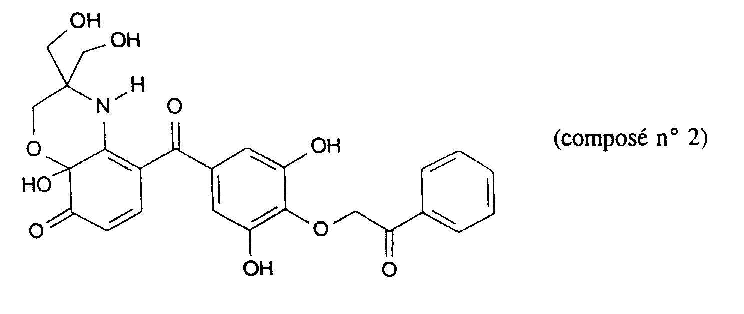

On opère d'une manière analogue à celle décrite dans l'exemple 8 a), mais à partir de 0,79 g de [2,3,4-trihydroxyphényl] [3',5'-dihydroxy-4'-(2-oxo-2-phényléthoxy) phényl] méthanone (composé VIIIb décrit dans l'exemple 2), de 12,10 g de Tris et de 2,30 g d'oxyde d'argent (Ag₂O). Après purification par chromatographie sur une colonne de 30 g de gel de silice (diamètre: 2,0 cm, hauteur: 60 cm) éluée avec un mélange acétate d'éthyle/méthanol (97/3 en volumes), on obtient 0,40 g de (RS)-5-[3,5-dihydroxy-4-(2-oxo-2-phényléthoxy)-benzoyl]-3,3-bis(hydroxyméthyl -8a-hydroxy-3,4-dihydro-2H-1,4-benzoxazin-8(8aH)-one sous forme d'une poudre orange fondant aux alentours de 185°C (avec décomposition).

Spectre de RMN ¹H: δ 3,40 [dd, 1H, CH₂OH(3), J = 12Hz, J = 5Hz]; 3,50 [d, 2H, CH₂OH(3), J = 5Hz]; 3,60 [dd, 1H, CH₂OH(3), J = 12Hz, J = 5Hz]; 3,85 [d, 1H, CH₂(2), J = 12Hz]; 3,90 [dd, 1H, CH₂(phényléthoxy), J = 12Hz, J = 5Hz]; 4,20 [d, 2H, CH₂(phényléthoxy) et CH₂(2), J = 12Hz]; 5,20 [dd, 1H, H(7), J = 10Hz, J = 2Hz]; 5,35 [m, 2H, OH(alcool), échange D₂O]; 6,55 [m, 2H, H(2) et H(6), benzoyl(5)]; 7,30 [dd, 1H, H(6), J = 10Hz, J = 2Hz]; 7,40 à 7,60 [m, 5H, aromatiques, phényléthoxy]; 8,30 [s, 1H, OH(8a), échange D₂O]; 9,60 [s, 2H, OH(3) et OH(5), benzoyl(5), échange D₂O]; 12,10 [s, 1H, NH, échange D₂O].
Spectre de RMN ¹³C: δ 57,7 (C-3); 59,5 [CH₂(2)]; 61,9 [CH₂OH(3)], 62,4 [CH₂OH(3)]; 71,3 [CH₂(phényléthoxy)]; 88,0 (C-8a); 95,2 [Cq(phényléthoxy)]; 99,9 (C-5); 108,3 [CH(7)]; 109,3 et 109,8 [CH(2) et CH(6), benzoyl(5)]; 127,4 [CH(méta), phényléthoxy]; 129,3 [CH (ortho), phényléthoxy]; 130,2 [CH (para), phényléthoxy]; 134,5 [C-4, benzoyl(5)]; 135,7 [C-1, benzoyl(5)]; 141,1 et 143,6 [C-3 et C-5, benzoyl(5)]; 146,9 (CO, phényléthoxy); 147,7 [CH(6)]; 168,2 (C-4a); 191,4 [CO(8)]; 192,4 [CO, benzoyl(5)]. Spectre de masse (I.C.): m/z = 498 (MH⁺).

### Exemple 10. Synthèse de la (RS)-5-[3,5-dihydroxy-4-(2-oxo-2-p.cyanophényléthoxy)]benzoyl-3,3-bis(hydroxyméthyl)-8a-hydroxy-3,4-dihydro-2H-1, 4-benzoxazin-8(8aH)-one (composé n° 3).

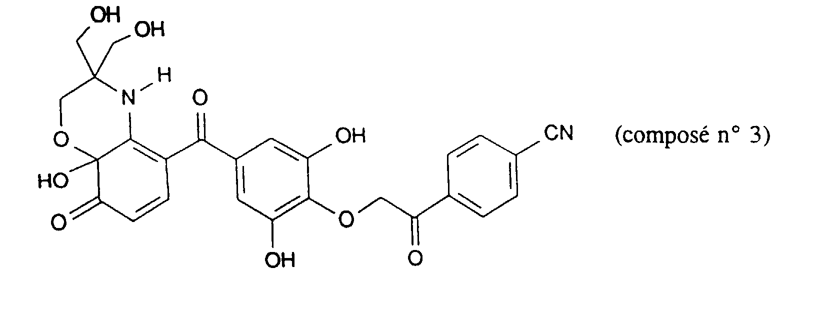

On opère de manière analogue à celle décrite dans l'exemple 8 a), mais à partir de 0,84 g de [2,3,4-trihydroxyphényl] [3',5'-dihydroxy-4'-(2-oxo-2-p.cyanophényléthoxy)phényl] méthanone (composé VIIIc décrit dans l'exemple 3), de 12,10 g de Tris et de 2,30 g d'oxyde d'argent (Ag₂O). Après purification par chromatographie sur une colonne de 30 g de gel de silice (diamètre 2,0 cm, hauteur 60 cm), éluée avec un mélange acétate d'éthyle/méthanol (97/3 en volumes), on obtient 0,42 g de (RS)-5-[3,5-dihydroxy-4-(2-oxo-2-p.cyanophényléthoxy)]benzoyl-3,3-bis(hydroxyméthyl)-8a-hydroxy-3,4-dihydro-2H-1,4-benzoxazin-8(8aH)-one sous forme d'une poudre orange fondant aux alentours de 215°C (avec décomposition).

Spectre de RMN ¹H: δ 3,35 [d, 1H, CH₂OH(3), J = 11Hz]; 3,50 [s, 2H, CH₂OH(3)]; 3,60 [d, 1H, CH₂OH(3), J = 11Hz]; 3,80 [d, 1H, CH₂(2)]; 3,90 [d, 1H, CH₂(p.cyanophényléthoxy)]; 4,20 [m, 2H, CH₂(p.cyanophényléthoxy) et CH₂(2)]; 5,20 [dd, 1H, H(7), J = 9Hz, J = 2Hz]; 5,35 [m, 2H, OH(alcool), échange D₂O]; 6,55 [m, 2H, H(2) et H(6), benzoyl(5)]; 7,35 [dd, 1H, H(6), J = 9Hz, J = 2Hz]; 7,75 [d, 2H, H(en γ du CN), p.cyanophényléthoxy, J = 9Hz]; 7,95 [d, 2H, H(en β du CN), p.cyanophényléthoxy, J = 9Hz]; 8,25 [s, 1H, OH(8a), échange D₂O]; 9,70 [s, 2H, OH(3) et OH(5), benzoyl(5), échange D₂O]; 12,10 [s, 1H, NH, échange D₂O].

Spectre de RMN ¹³C: δ 57,7 (C-3); 59,7 [CH₂(2)]; 61,9 [CH₂OH(3)], 62,4 [CH₂OH(3)]; 70,7 (CH₂, p.cyanophényléthoxy); 87,9 (C-8a); 94,9 [Cq(en δ du CN), p.cyanophényléthoxy]; 99,8 (C-5); 108,3 [CH(7)]; 109,2 et 109,9 [CH(2) et CH(6), benzoyl(5)]; 112,9 [Cq(en α du CN), p.cyanophényléthoxy]; 119,7 (CN); 128,6 [CH(en γ du CN), p.cyanophényléthoxy]; 133,0 [C4, benzoyl(5)]; 133,5 [CH(en β du CN), p.cyanophényléthoxy]; 134,3 [C-1, benzoyl(5)]; 143,2 (cétone, p.cyanophényléthoxy); 146,0 et 147,0 [C-3 et C-5, benzoyl(5)]; 147,7 [CH(6)]; 168,2 (C4a); 191,4 [CO(8)]; 192,8 [CO, benzoyl(5)].

Spectre de masse (I.C.): m/z = 523 (MH⁺).

### Exemple 11. Synthèse de la (RS)-5-[3,5-dihydroxy-4-(2-oxo-2-p.méthoxyphényléthoxy)]benzoyl-3,3-bis(hydroxyméthyl)-8a-hydroxy-3,4-dihydro-2H-1,4-benzoxazin-8(8aH)-one (composé n ° 4).

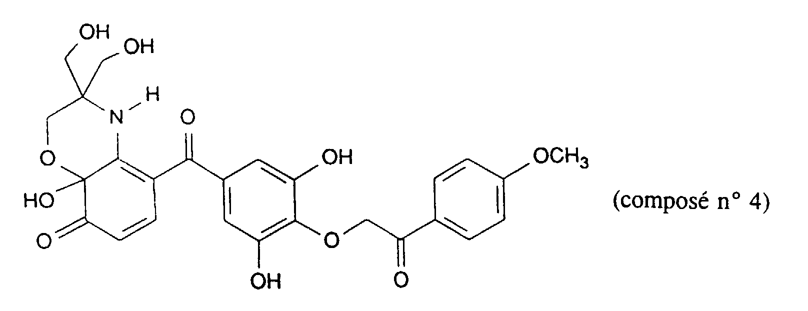

On opère de manière analogue à celle décrite dans l'exemple 8 a), mais à partir de 0,85 g de [2,3,4-trihydroxyphényl] [3',5'-dihydroxy-4'-(2-oxo-2-p.méthoxyphényléthoxy)phényl] méthanone (composé VIIId décrit dans l'exemple 4), de 12,10 g de Tris et de 2,30 g d'oxyde d'argent (Ag₂O). Après purification par chromatographie sur une colonne de 30 g de gel de silice (diamètre 2,0 cm, hauteur 60 cm), éluée avec un mélange acétate d'éthyle/méthanol (97/3 en volumes), on obtient 0,37 g de (RS)-5-[3,5-dihydroxy-4-(2-oxo-2-p.méthoxyphényléthoxy)]benzoyl-3,3-bis(hydroxyméthyl)-8a-hydroxy-3,4-dihydro-2H-1,4-benzoxazin-8(8aH)-one sous forme d'une poudre orange.

Spectre de RMN ¹H: δ 3,35 [d, 1H, CH₂OH(3) J = 11Hz]; 3,50 [s, 2H, CH₂OH(3)]; 3,60 [d, 1H, CH₂OH(3), J = 11Hz]; 3,75 (s, 3H, OCH₃); 3,80 (d, 1H, CH₂(2), J = 11Hz]; 3,90 [d, 1H, CH₂(p.méthoxyphényléthoxy)]; 4,20 [m, 2H, CH₂(p.méthoxyphényléthoxy) et CH₂(2)]; 5,20 [dd, 1H, H(7), J = 9Hz, J = 2Hz]; 5,40 [large s, 2H, OH(alcool), échange D₂O]; 6,40 à 6,60 [m, 2H, H(2) et H(6), benzoyl(5)]; 7,00 [d, 2H, H(en β du OCH₃), p.méthoxyphényléthoxy, J = 9Hz]; 7,35 [dd, 1H, H(6), J = 9Hz, J = 2Hz]; 7,50 [d, 2H, H(en γ du OCH₃), J = 9Hz]; 8,30 [s, 1H, OH(8a), échange D₂O]; 9,65 [s, 2H, OH(3) et OH(5), benzoyl(5), échange D₂O]; 12,10 [s, 1H, NH, échange D₂O].

Spectre de RMN ¹³C: δ 56,3 (OCH₃); 57,7 (C-3); 59,5 [CH(2)]; 61,9 [CH₂OH(3)], 62,4 [CH₂OH(3)]; 71,4 (CH₂, p.méthoxyphényléthoxy); 88,0 (C-8a); 95,2 [Cq(en δ du OCH₃), p.méthoxyphényléthoxy]; 99,9 (C-5); 108,3 [CH(7)]; 114,8 et 115,2 [CH(2) et CH(6), benzoyl(5)]; 128,8 [CH(en γ du OCH₃, p.méthoxyphényléthoxy]; 131,6 [CH (en β du OCH₃), p.méthoxyphényléthoxy]; 133,2 [C-4, benzoyl(5)]; 134,5 [C-1, benzoyl(5)] 146,9 (CO, p.méthoxyphényléthoxy); 147,7 [CH(6)]; 160,7 [Cq(en α du OCH₃)]; 168,2 (C4a); 191,4 [CO(8)]; 193,4 [CO, benzoyl(5)].

Spectre de masse (I.C.): m/z = 528 (MH⁺).

### Exemple 12. Synthèse de la (RS)-5-(3, 5-dihydroxy-4-méthoxy)-benzoyl-3,3-bis (hydroxyméthyl)-8a-hydroxy-3,4-dihydro-2H-1,4-benzoxazin-8(8aH)-one (composé n° 5)

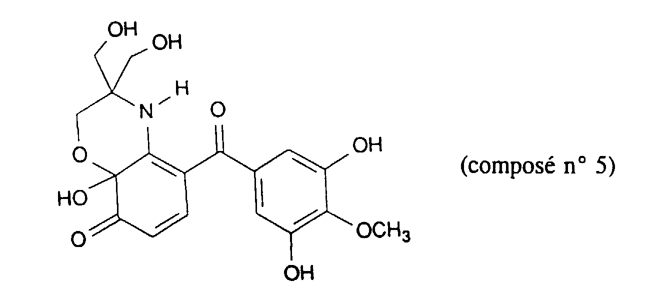

On opère d'une manière analogue à celle décrite dans l'exemple 8 a), mais à partir de 0,58 g de [2,3,4-trihydroxyphényl] [3',5'-dihydroxy-4'-méthoxy-phényl] méthanone (composé VIIIa décrit dans l'exemple 1), de 12,10 g de Tris et de 1,24 g d'oxyde d'argent (AgO). Après purification par chromatographie sur une colonne de 20 g de gel de silice (diamètre 1,8 cm, hauteur 60 cm), éluée avec un mélange acétate d'éthyle/méthanol (95/5 en volumes), on obtient 0,3 g de (RS)-5-(3,5-dihydroxy-4-méthoxy)-benzoyl-3,3-bis(hydroxyméthyl)-8a-hydroxy-3,4-dihydro-2H-1,4-benzoxazin-8(8aH)-one, sous forme d'une poudre orange fondant aux alentours de 150°C (avec décomposition).

Spectre de RMN ¹H: δ 3,35 [d, 1H, CH₂OH(3), J = 11Hz]; 3,50 [s, 2H, CH₂OH(3)]; 3,60 [d, 1H, CH₂OH(3), J = 11Hz]; 3,75 (s, 3H, OCH₃); 3,85 [d, 1H, CH₂(2), J = 12Hz]; 4,20 [d, 1H, CH₂(2), J = 12Hz]; 5,15 [d, 1H, H(7), J = 10Hz]; 5,40 [large s, 2H, OH(alcool), échange D₂O]; 6,40 [s, 2H, H(2) et H(6), benzoyl (5)]; 7,30 [d, 1H, H(6), J = 10Hz]; 8,25 [large s, 1H, OH(8a), échange D₂O]; 9,40 [large s, 2H, OH(3') et OH(5'), échange D₂O]; 12,10 [s, 1H, NH, échange D₂O].

Spectre de RMN ¹³C: δ 57,7 (C-3); 59,5 [CH₂(2)]; 60,8 (OCH₃); 61,9 [CH₂OH(3)], 62,4 [CH₂OH(3)]; 87,9 (C-8a); 100,0 (C-5); 108,3 [CH(7); CH(2) et CH(6), benzoyl(5)]; 135,7 [C4, benzoyl(5)]; 138,3 [C-1, benzoyl(5)]; 147,8 [CH(6)]; 151,6 [C-3 et C-5, benzoyl(5)]; 168,2 (C-4a); 191,4 [CO(8)]; 193,5 [CO, benzoyl(5)].

Spectre de masse (I.C.): m/z = 377 (MH⁺).

### Exemple 13. Synthèse de la (RS)-5-benzoyl-3,3-bis-(hydroxyméthyl)-8a-méthoxy-3,4-dihydro-2H-1,4-benzoxazin-8(8aH)-one (composé n° 6).

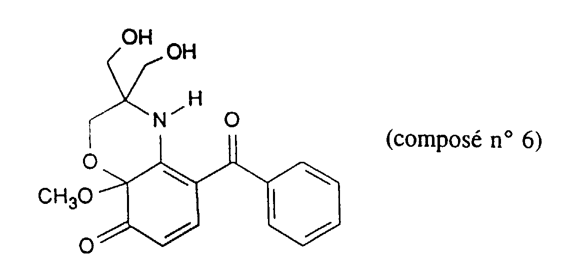

A une solution de 0,160 g de composé n° 1 dans 2 cm³ de diméthylformamide maintenue à la température ambiante, sous atmosphère d'azote, on ajoute 0,027 g de méthylate de sodium en solution dans 0,5 cm³ de méthanol, puis 0,5 cm³ d'iodure de méthyle. Le mélange réactionnel est ensuite agité trois heures à la température ambiante. On ajoute alors 38 cm³ d'eau distillée. La solution résultante est extraite avec 100 cm³ d'acétate d'éthyle. Les phases organiques réunies sont lavées par 100 cm³ d'eau distillée, puis séchées sur sulfate de sodium anhydre. Après filtration puis concentration à sec, sous pression réduite (2,7 kPa) à 40°C des phases organiques, on obtient 0,18 g d'un résidu qui est purifié par chromatographie sur une colonne de 7 g de gel de silice (diamètre 1,3 cm, hauteur 60 cm). La colonne est éluée avec un mélange acétate d'éthyle/méthanol (97/3 en volumes), en recueillant des fractions de 3 cm³. Les fractions contenant le produit attendu sont concentrées à sec sous pression réduite (2,7 kPa), à une température voisine de 40°C. On obtient 0,16 g de (RS)-5-benzoyl-3,3-bis-(hydroxyméthyl)-8a-méthoxy-3,4-dihydro-2H-1,4-benzoxazin-8(8aH)-one, sous forme d'une poudre orange fondant aux alentours de 168°C (avec décomposition).

Spectre de RMN ¹H: δ 3,20 (s, 3H, OCH₃); 3,40 [dd, 1H, CH₂OH(3), J = 12Hz, J = 5Hz]; 3,50 [d, 2H, CH₂OH(3), J = 5Hz]; 3,65 [dd, 1H, CH₂OH(3), J = 12Hz, J = 5Hz]; 3,95 [s, 2H, CH₂(2)]; 5,20 [d, 1H, H(7), J = 10Hz]; 5,40 [m, 2H, OH(alcool), échange D₂O]; 7,20 [d, 1H, H(6), J = 10Hz]; 7,40 à 7,50 [m, 5H, aromatiques, benzoyl(5)]; 12,30 (s, 1H, NH, échange D₂O).

Spectre de RMN ¹³C: δ 51,0 (OCH₃); 57,0 (C-3); 59,5 [CH₂(2)]; 61,5 [CH₂OH(3)]; 62,0 [CH₂OH(3)]; 90,0 (C-8a); 100,0 (C-5); 108,0 [CH(7)]; 128,0 [CH(méta), benzoyl(5)]; 129,0 [CH(ortho), benzoyl(5)]; 130,8 [CH(para), benzoyl(5)]; 139,7 [Cq, benzoyl(5)];147,0 [CH(6)]; 166,0 (C4a); 189,0 [CO (8)]; 193,0 [CO, benzoyl(5)]. Spectre de masse (I.C.): m/z = 346 (MH⁺).

### Exemple 14. Synthèse de la (RS)-5-benzoyl-3,3-bis-(hydroxyméthyl, méthyl)-8a-hydroxy-3, 4-dihydro-2H-1, 4-benzoxazin-8(8aH)-one (composé n° 7).

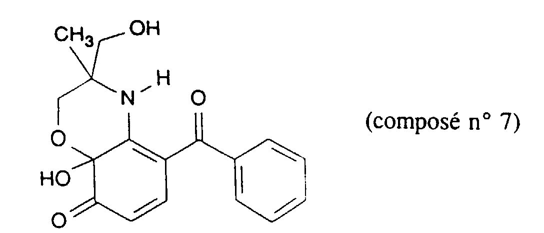

a) En opérant comme dans l'exemple 8 a), mais en remplaçant le TRIS par 10,5 g de 2-amino-2-méthyl-propanediol, on obtient 0,38 g de (RS)-5-benzoyl-3,3-bis-(hydroxyméthyl,méthyl)-8a-hydroxy-3,4-dihydroxy-2H-1,4-benzoxazin-8(8aH)-one sous forme d'une poudre orange fondant aux alentours de 155°C (avec décomposition).

Il s'agit d'un mélange de deux diastéréoisomères A et B.

Spectre de RMN ¹H du diastéréoisomère A (65%): δ 1,25 [s, 3H, CH₃(3)]; 3,45 [s, 2H, CH₂OH(3)]; 3,85 [d, 1H, CH₂(2), J = 12Hz]; 4,15 [d, 1H, CH₂(2), J = 12Hz]; 5,20 [d, 1H, H(7), J = 10Hz]; 5,45 [large s, 1H, OH(alcool), échange D₂O]; 7,20 [d, 1H, H(6), J = 10Hz], 7,45 [m, 5H, aromatiques, benzoyl(5)]; 8,40 [large s, 1H, OH(8a), échange D₂O]; 12,20 [s, 1H, NH, échange D₂O].

Spectre de RMN ¹³C du diastéréoisomère A: δ 21,6 [CH₃(3)] ; 54,7 (C-3); 62,6 [CH₂(2)]; 67,2 [CH₂OH(3)]; 87,7 (C-8a); 99,8 (C-5); 108,9 [CH(7)]; 128,7 [CH(méta), benzoyl(5)]; 129,4 [CH(ortho), benzoyl(5)]; 131,2 [CH(para), benzoyl(5)]; 140,5 [Cq, benzoyl(5)]; 147,2 [CH(6)]; 168,1 (C-4a); 191,2 [CO(8)]; 194,0 [CO, benzoyl(5)].

Spectre de masse (I.C.): m/z = 316 (MH⁺)

Spectre de RMN ¹H du diastéréoisomère B (35%): δ 1,35 [s, 3H, CH₃(3)]; 3,47 [s, 2H, CH₂OH(3)]; 3,75 [d, 1H, CH₂(2), J = 12Hz]; 4,25 [d, 1H, CH₂(2), J = 12 Hz]; 5,15 [d, 1H, H(7), J = 10Hz]; 5,45 [large s, 1H, OH(alcool), échange D₂O]; 7,15 [d, 1H, H(6), J = 10Hz]; 7,45 [m, 5H, aromatiques, benzoyl(5)]; 8,30 [large s, 1H, OH(8a), échange D₂O]; 12,30 [s, 1H, NH, échange D₂O].

Spectre de RMN ¹³C du diastéréoisomère B: δ 24,7 [CH₃(3)]; 55,5 (C-3); 63,6 [CH₂(2)]; 66,0 [CH₂OH(3)]; 87,9 (C-8a); 98,8 (C-5); 108,7 [CH(7)]; 128,7 [CH(méta), benzoyl(5)]; 129,4 [CH(ortho), benzoyl(5)]; 131,2 [CH(para), benzoyl(5)]; 140,5 [Cq, benzoyl(5)]; 147,2 [CH(6)]; 166,0 (C4a); 191,2 [CO(8)]; 194,0 [CO, benzoyl(5)].

Spectre de masse (I.C.): m/z = 316 (MH⁺).

b) Le composé n° 7 peut également être obtenu en opérant comme dans l'exemple 8 b-1), mais à partir de 0,110 g de 3,4-dihydroxybenzophénone et en remplaçant le Tris par 2,62 g de 2-amino-2-méthyl-propanediol. On obtient ainsi 0,055 g de composé n° 7 dont les caractéristiques sont identiques à celles du produit obtenu dans l'exemple 14 a).

### Exemple 15. Synthèse de la (RS)-5-benzoyl-3,3-bis-(méthyl)-8a-hydroxy-3,4-dihydro-2H-1,4-benzoxazin-8(8aH)-one (composé n° 8).

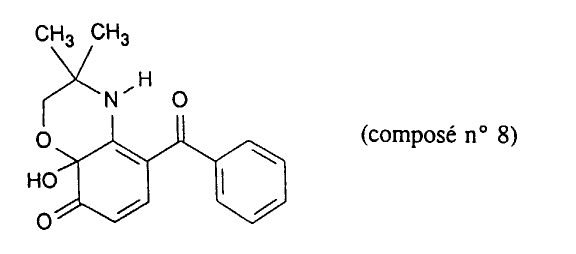

On opère d'une manière analogue à celle décrite dans l'exemple 8 a), mais en remplaçant Ag₂O par 1,24 g de AgO et le Tris par 8,9 g de 2-amino-2-méthylpropanol. Après purification par chromatographie sur une colonne de 20 g de gel de silice (diamètre: 1,8 cm, hauteur 60 cm), éluée avec un mélange dichlorométhane/méthanol (98/2 en volumes), on obtient 0,23 g de (RS)-5-benzoyl-3,3-bis-(méthyl)-8a-hydroxy-3,4-dihydro-2H-1,4-benzoxazin-8(8aH)-one sous forme d'une poudre orange fondant aux alentours de 140°C (avec décomposition).

Spectre de RMN 1H: δ 1,35 [s, 3H, CH₃(3)]; 1,40 [s, 3H, CH₃(3)]; 3,75 [d, 1H, CH₂(2), J = 12Hz]; 4,20 [d, 1H, CH₂(2), J = 12Hz]; 5,20 [d, 1H, H(7), J = 10Hz]; 7,20 [d, 1H, H(6), J = 10Hz]; 7,50 [m, 5H, aromatiques, benzoyl(5)]; 8,35 [s, 1H, OH(8a), échange D₂O]; 12,30 [s, 1H, NH, échange D₂O].

Spectre de RMN ¹³C: δ 25,8 [CH₃(3)]; 28,8 [CH₃(3)]; 52,1 (C-3); 66,7 [CH₂(2)]; 87,8 (C-8a); 99,7 (C-5); 108,7 [CH(7)]; 128,7 [CH(méta), benzoyl(5)]; 129,5 [CH(ortho), benzoyl(5)]; 131,3 [CH(para), benzoyl(5)]; 140,5 [Cq, benzoyl(5)]; 147,2 [CH(6)]; 167,9 (C-4a); 191,2 [CO(8)]; 193,7 [CO, benzoyl(5)].

Spectre de masse (I.C.): m/z = 300 (MH⁺).

### Exemple 16. Synthèse de la [(RS)-3,3-bis-(hydroxyméthyl)-8a-hydroxy-8-oxo-3, 4,8,8a-tétrahydro-2H-1,4-benzoxazin-5-yl]éthanone (composé n° 13).

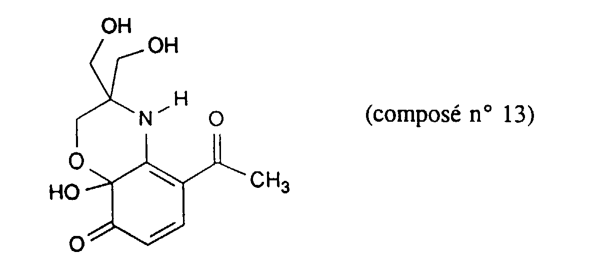

On opère d'une manière analogue à celle décrite dans l'exemple 8 a), mais à partir de 0,34 g de 2,3,4-trihydroxyacétophénone. Après purification par chromatographie sur une colonne de 20 g de gel de silice (diamètre: 1,8 cm, hauteur 60 cm), éluée avec un mélange acétate d'éthyle/méthanol (95/5 en volumes), on obtient 0,08 g de [(RS)-3,3-bis-(hydroxyméthyl)-8a-hydroxy-8-oxo-3,4,8,8a-tétrahydro-2H-1,4-benzoxazin-5-yl] éthanone, sous forme d'une poudre orange.

Spectre de RMN ¹H: δ 2,20 (s, 3H, CH₃); 3,35 [dd, 1H, CH₂OH(3), J = 11Hz, J = 4Hz]; 3,40 [d, 2H, CH₂OH(3), J = 4Hz]; 3,55 [dd, 1H, CH₂OH(3), J = 11Hz, J = 4Hz]; 3,80 [d, 1H, CH₂(2), J = 11Hz]; 4,15 [d, 1H, CH₂(2), J = 11Hz]; 5,30 [m, 3H, H(7), et OH(alcool), échange D₂O]; 7,55 [d, 1H, H(6), J = 10Hz]; 8,10 [s, 1H, OH(8a), échange D₂O]; 11,90 [s, 1H, NH, échange D₂O].

Spectre de RMN ¹³C: δ 27,6 (CH₃); 57,3 [CH₂(2)]; 59,6 (C-3); 61,9 [CH₂OH(3)]; 62,3 [CH₂OH(3)]; 87,6 (C-8a); 100,8 (C-5); 109,0 [CH(7)]; 146,9 [CH(6)]; 166,0 (C4a); 191,6 [CO(8)]; 196,0 [CO, éthanone].

Spectre de masse (I.C.): m/z = 270 (MH⁺).

### Exemple 17. Synthèse de la (RS)-3,3-bis-(hydroxyméthyl)-8a-hydroxy-8-oxo-3,4,8,8a-tétrahydro-2H-1,4-benzoxazine-5-carboxylate de méthyle (composé n°14).

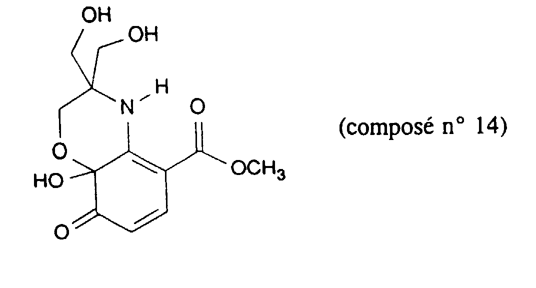

On opère d'une manière analogue à celle décrite dans l'exemple 8 a), mais à partir de 0,37 g de 2,3,4-trihydroxyphényl-1-carboxylate de méthyle (composé VIIIf décrit dans l'exemple 6). Après purification par chromatographie sur une colonne de 20 g de gel de silice (diamètre 1,8 cm, hauteur 60 cm), éluée avec un mélange acétate d'éthyle/méthanol (95/5 en volumes), on obtient 0,12 g de (RS)-3,3-bis-(hydroxyméthyl)-8a-hydroxy-8-oxo-3,4,8,8a-tétrahydro-2H-1,4-benzoxazine-5-carboxylate de méthyle sous la forme d'une poudre orange.

Spectre de RMN ¹H: δ 3,30 [dd, 1H, CH₂OH(3), J = 11Hz, J = 5Hz]; 3,40 [d, 2H, CH₂OH(3), J = 5Hz]; 3,55 [dd, 1H, CH₂OH(3), J = 11Hz, J = 5Hz]; 3,70 (s, 3H, OCH₃); 3,80 [d, 1H, CH₂(2), J = 12Hz]; 4,15 [d, 1H, CH₂(2), J = 12Hz]; 5,30 [m, 3H, H(7) et OH(alcool), échange D₂O]; 7,45 [d, 1H, H(6), J = 10Hz]; 8,05 [s, 1H, OH(8a), échange D₂O]; 10,0 [s, 1H, NH, échange D₂O].

Spectre de RMN ¹³C: δ 52,0 (OCH₃); 57,2 (C-3); 59,7 [CH₂(2)]; 62,0 [CH₂OH(3)] ; 62,3 [CH₂OH(3)] ; 87,9 (C-8a); 90,0 (C-5); 109,8 [CH(7)]; 144,7 [CH(6)]; 165,4 (C4a); 168,1 [CO, carboxylate de méthyle]; 191,9 [CO(8)].

Spectre de masse (I.C.): m/z = 286 (MH⁺).

### Exemple 18. Synthèse de la [(RS)-3,3-bis-(hydroxyméthyl)-8a-hydroxy-8-oxo-3,4,8,8a-tétrahydro-2H-1,4-benzoxazin-5-yl] chloroéthanone (composé n° 15).

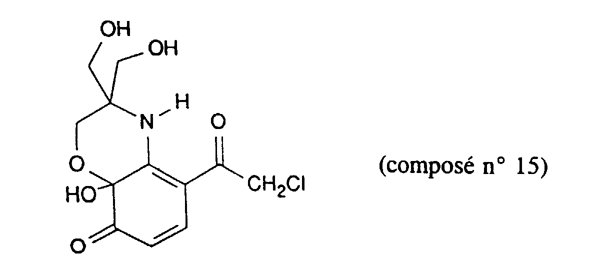

En opérant comme dans l'exemple 8 b-1), mais à partir de 0,093 g de (3,4-dihydroxy) chloroacétophénone, on obtient ainsi 0,015 g de [(RS)-3,3-bis-(hydroxyméthyl)-8a-hydroxy-8-oxo-3,4,8,8a-tétrahydro-2H-1,4-benzoxazin-5-yl] chloroéthanone, sous forme d'une poudre orange.

Spectre de RMN ¹H: δ 3,35 [d, 1H, CH₂OH(3), J = 11Hz]; 3,40 [s, 2H, CH₂OH(3)]; 3,55 [d, 1H, CH₂OH(3), J = 11Hz]; 3,85 [d, 1H, CH(2), J = 11Hz]; 4,15 [d, 1H, CH₂(2), J = 11Hz]; 4,60 [d, 2H, CH₂Cl, J = 7Hz]; 5,30 [d, 1H, H(7), J = 10Hz]; 5,40 [large s, 2H, OH(alcool), échange D₂O]; 7,50 [d, 1H, H(6), J = 10Hz]; 8,25 [s, 1H, OH(8a), échange D₂O]; 11,90 [s, 1H, NH, échange D₂O].

Spectre de masse (I.C.): m/z = 304 (MH⁺).

### C) Synthèse des composés de formule générale (III)

### Exemple 19. Synthèse de la [3,3-bis(hydroxyméthyl)-8-hydroxy-3,4-dihydro-2H-1,4-benzoxazin-5-yl] [phényl] méthanone (composé n° 9)

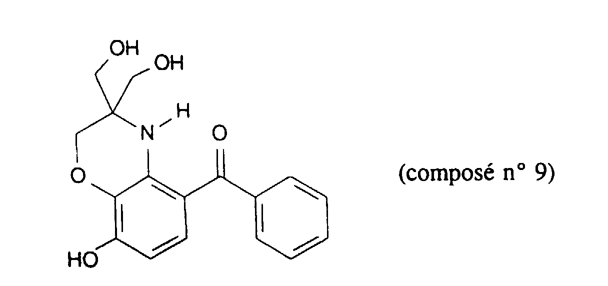

Le composé n° 9 peut être préparé par électrolyse à potentiel contrôlé du composé n° 1. Le dispositif d'électrolyse est identique à celui décrit dans l'exemple 8 b-1), à l'exception de l'électrode de travail qui est remplacée par une nappe de mercure.
On procède à l'électrolyse d'une solution de 0,16 g de composé n° 1, dans 250 cm³ de méthanol contenant 2,3 g de perchlorate de tétraéthylammonium comme électrolyte de support, sous atmosphère d'azote, à 25°C, à une cathode de mercure dont le potentiel est fixé à -1,5V e.c.s. A la fin de l'électrolyse, lorsque l'intensité du courant est devenue négligeable (0,5 mA) devant l'intensité du courant initial (80 mA), la solution résultante est évaporée à sec sous pression réduite (2,7 kPa), à une température voisine de 40°C. Le résidu est repris par 50 cm³ d'une solution de tampon acéto-acétique molaire dont le pH est voisin de 4,5. La solution résultante est alors extraite avec 100 cm³ d'acétate d'éthyle. Les phases organiques réunies sont séchées sur du sulfate de sodium anhydre, filtrées et le solvant est évaporé à sec sous pression réduite (2,7 kPa), à une température voisine de 40°C. Après purification par chromatographie sur une colonne de 10 g de gel de silice (diamètre 1,3 cm, hauteur 60 cm), éluée avec de l'acétate d'éthyle, on obtient 0,13 g de composé [3,3-bis(hydroxyméthyl)-8-hydroxy-3,4-dihydro-2H-1,4-benzoxazin-5-yl] [phényl] méthanone sous forme d'une poudre jaune fondant aux alentours de 90°C (avec décomposition).

Spectre de RMN ¹H: δ 3,30 [dd, 2H, CH₂OH(3), J = 10Hz, J = 4Hz]; 3,45 [dd, 2H, CH₂OH(3), J = 10Hz, J = 4Hz]; 3,90 [s, 1H, CH₂(2)]; 5,00 [t, 2H, OH(alcool), J = 4Hz, échange D₂O]; 6,05 [d, 1H, H(7), J = 9Hz]; 6,80 [d, 1H, H(6), J = 9Hz]; 7,50 (m, 5H, phényl); 8,80 [s, 1H, NH, échange D₂O]; 9,90 [s, 1H, OH(8), échange D₂O].

Spectre de RMN ¹³C: δ 56,5 (C-3); 61,2 [CH₂OH(3)]; 65,7 [CH₂(2)]; 105,2 [CH(7)]; 111,2 (C-5); 129,2 [CH(ortho et méta), phényl]; 129,7 [CH(6)]; 130,2 (C-8a); 131,3 [CH,(para), phényl]; 141,0 (Cq, phényl); 141,7 (C4a); 150,8 (C-8); 197,0 (CO, méthanone).

Spectre de masse (I.C.): m/z = 316 (MH⁺).

### Exemple 20. Synthèse de la [3,3-bis(hydroxyméthyl)-8-méthoxy-3,4-dihydro-2H-1,4-benzoxazin-5-yl] [phényl] méthanone (composé n° 10).

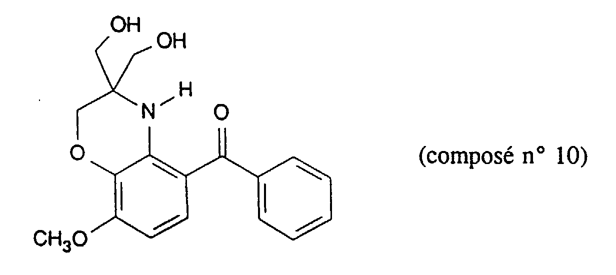

On opère d'une manière analogue à celle décrite dans l'exemple 13, mais à partir de 0,16 g de composé n° 9. Après purification par chromatographie sur une colonne de 10 g de gel de silice (diamètre 1,3 cm, hauteur 60 cm), éluée avec un mélange acétate d'éthyle/cyclohexane (75/25 en volumes), on obtient 0,13 g de [3,3-bis(hydroxyméthyl)-8-méthoxy-3,4-dihydro-2H-1,4-benzoxazin-5-yl] [phényl] méthanone sous forme d'une poudre jaune fondant aux alentours de 168°C (avec décomposition).

Spectre de RMN ¹H: δ 3,35 [dd, 2H, CH₂OH(3), J = 11Hz, J = 5Hz]; 3,50 [dd, 2H, CH₂OH(3), J = 11Hz, J = 5Hz]; 3,80 (s, 3H, OCH₃); 3,90 [s, 2H, CH₂(2)]; 5,05 [t, 2H, OH(alcool), J = 5Hz, échange D₂O]; 6,35 [d, 1H, H(7), J = 9Hz]; 6,95 [d, 1H, H(6), J = 9Hz]; 7,50 (m, 5H, phényl); 8,60 [s, 1H, NH, échange D₂O].

Spectre de RMN ¹³C: δ 56,3 (C-3); 56,7 (CH₃); 61,1 [CH₂OH(3)], 65,6 [CH₂(2)]; 100,6 [CH(7)]; 112,6 (C-5); 129,2 et 129,3 [CH(ortho et méta), phényl]; 129,6 [CH(6)]; 131,4 (C-8a); 131,6 [CH(para), phényl]; 140,0 (C-4a); 141,3 (Cq, phényl); 152,3 (C-8); 197,0 (CO, méthanone).

Spectre de masse (I.C.): m/z = 330 (MH⁺).

### Exemple 21. Synthèse de la [3,3-bis(hydroxyméthyl)-8-hydroxy-3,4-dihydro-2H-1,4-benzoxazin-5-yl] [3',5'-dihydroxy-4'-(2-oxo-2-phényléthoxy) phényl] méthanone (composé n° 11).

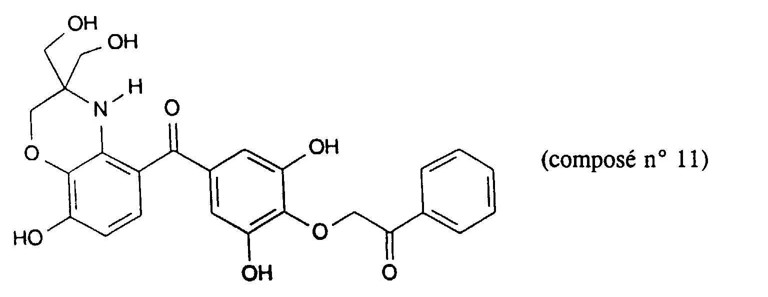

0,58 g de composé n° 2 est dissous dans 25 cm³ d'une solution d'acide acétique glacial dilué au quart dans du méthanol. On ajoute ensuite en 5 minutes, à la température ambiante, 0,38 g de zinc en poudre. Le mélange réactionnel est agité 2 minutes. Après filtration, on ajoute 20 cm³ d'eau distillée. La solution résultante est concentrée à 10 cm³, sous pression réduite (2,7 kPa), à une température voisine de 40°C. Le refroidissement du mélange réactionnel entraîne la précipitation d'un produit qui est lavé à l'eau et recristallisé dans un mélange dichlorométhane/acétone (98/2 en volumes). On obtient 0,39 g de [3,3-bis(hydroxyméthyl)-8-hydroxy-3,4-dihydro-2H-1,4-benzoxazin-5-yl] [3',5'-dihydroxy-4'-(2-oxo-2-phényléthoxy)phényl] méthanone sous forme d'une poudre jaune vif fondant aux alentours de 270°C (avec décomposition).

Spectre de RMN ¹H: δ 3,35 [dd, 2H, CH₂OH(3), J = 11Hz, J = 5Hz]; 3,50 [dd, 2H, CH₂OH(3), J = 11Hz, J = 5Hz]; 3,90 [s, 2H, CH₂(2)]; 3,95 [d, 1H, CH₂(phényléthoxy), J = 11Hz]; 4,20 [d, 1H, CH₂(phényléthoxy), J = 11Hz]; 5,05 [t, 2H, OH(alcool) J = 5Hz, échange D₂O]; 6,10 [d, 1H, H(7), J = 9Hz]; 6,60 [m, 2H, H(2') et H(6')]; 7,00 [d, 1H, H(6), J = 9Hz]; 7,40 à 7,60 [m, 5H, aromatiques, phényléthoxy]; 8,55 [s, 1H, NH, échange D₂O]; 9,60 [s, 2H, OH(3') et OH(5'), échange D₂O]; 9,85 [s, 1H, OH(8), échange D₂O].

Spectre de RMN ¹³C: δ 56,5 (C-3); 61,3 [CH₂OH(3)]; 65,8 [CH₂(2)]; 71,3 [CH₂(phényléthoxy)]; 95,2 (Cq, phényléthoxy); 104,9 [CH(7)]; 110,2 et 110,6 [CH(2') et CH(6')]; 111,4 (C-5); 127,5 [CH(méta), phényléthoxy]; 129,4 [CH(ortho et para), phényléthoxy]; 130,0 [CH(6)]; 130,4 (C-8a); 133,9 (C4'); 134,6 (C-1'); 140,7 (C-4a); 141,1 et 143,3 (C-3' et C-5'); 146,6 (CO, phényléthoxy); 150,6 (C-8); 196,1 (CO, méthanone).

Spectre de masse (I.C.): m/z = 482 (MH⁺).

### Exemple 22. Synthèse de la [3,3-bis(hydroxyméthyl)-8-hydroxy-3,4-dihydro-2H-1,4-benzoxazin-5-yl] [3',4',5'-trihydroxyphényl] méthanone (composé n° 12).

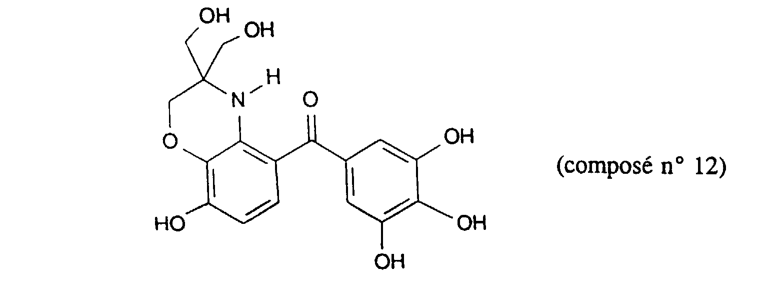

0,56 g de composé n° 2 est dissous dans 25 cm³ d'une solution d'acide acétique glacial dilué au quart dans du méthanol. On ajoute ensuite, en 5 minutes, à la température ambiante, 0,38 g de zinc en poudre. Le mélange réactionnel est agité 30 minutes. Après filtration, on ajoute 20 cm³ d'eau distillée. La solution résultante est concentrée à 20 cm³ sous pression réduite (2,7 kPa), à une température voisine de 40°C, puis extraite avec 100 cm³ d'acétate d'éthyle. Les phases organiques réunies sont séchées sur du sulfate de sodium anhydre puis filtrées; l'acétate d'éthyle est évaporé à sec sous pression réduite (2,7 kPa) à 40°C. Le résidu, recristallisé dans un mélange dichlorométhane/acétone (95/5 en volumes) permet d'obtenir 0,29 g de [3,3-bis-(hydroxyméthyl)-8-hydroxy-3,4-dihydro-2H-1,4-benzoxazin-5-yl] [3',4',5'-trihydroxyphényl] méthanone sous la forme d'une poudre jaune fondant aux alentours de 150°C (avec décomposition).

Spectre de RMN ¹H: δ 3,30 [dd, 2H, CH₂OH(3), J = 11Hz, J = 5Hz]; 3,50 [dd, 2H, CH₂OH(3), J = 11Hz, J = 5Hz]; 3,90 [s, 2H, CH₂(2)]; 5,00 [t, 2H, OH(alcool), J = 5Hz, échange D₂O]; 6,10 [d, 1H, H(7), J = 9Hz]; 6,50 [s, 2H, H(2') et H(6')]; 7,00 [d, 1H, H(6), J = 9Hz]; 8,30 [s, 1H, NH, échange D₂O]; 8,70 [s, 1H, OH(4'), échange D₂O]; 9,15 [s, 2H, OH(3') et OH(5'), échange D₂O]; 9,70 [s, 1H, OH(8), échange D₂O].

Spectre de RMN ¹³C: δ 56,3 (C-3); 61,3 [CH₂OH(3)]; 65,9 [CH₂(2)]; 104,5 [CH(7)]; 109,7 [CH(2') et CH(6')]; 111,9 (C-5); 129,2 [CH(6)]; 130,4 (C-8a); 131,5 (C-1'); 137,4 (C-4a); 140,2 (C4'); 146,3 (C-3' et C-5'); 150,2 (C-8); 196,5 (CO, méthanone).

Spectre de masse (I.C.): m/z = 364 (MH⁺).

### II. Etude pharmacologique

La production de radicaux libres est impliquée dans le processus de la sénescence normale ou pathologique et dans les pathologies qui leurs sont fréquemment associées: maladie de PARKINSON, maladie d'ALZHEIMER, ischémie et hypoxie cérébrales.

La littérature abonde en publications établissant un lien entre les radicaux libres (et particulièrement le NO·-oxyde nitrique) et les dommages causés aux cellules cérébrales. Citons quelques publications représentatives:
- Ted M. DAWSON, Valiona L. DAWSON, Solomon H. SNYDER. A novel neuronal messenger molecule in brain: the free radical, nitric oxyde. Ann. Neurol. 1992; 32: 297-311.
- C. JEANDEL, M.B. NICOLAS, F. DUBOIS, F. NABET-BELLEVILLE, F. PENIN, G. CUNY. Lipid peroxidation and free radical scavengers in Alzheimer's disease. Gerontoly, 1989; 35: 275-282.
- V. E. KAGAN et al. Antioxidant protection of the brain against oxidative stress, in FREE RADICALS IN THE BRAIN, Springer-Verlag-Berlin, 1992, 49-61.
- James B. LOHR. Oxygen radicals and neuropsychiatric illness - some speculations. Arch. gen. psychiatry, 1991, 48: 1097-1106.
- L. PACKER. Free radicals scavengers and antioxidants in prophylaxy and treatment of brain disease in FREE RADICALS IN THE BRAIN, Springer-Verlag-Berlin, 1992, 1-20.
- P.M. SINET, I. CEBALLOS-PICOT. Role of free radicals in Alzheimer's disease and Down's syndrome. in FREE RADICALS IN THE BRAIN, Springer-Verlag-Berlin, 1992, 91-98.
- Solomon SNYDER, David BREDT. les fonctions biologiques du monoxyde d'azote. Pour la Science, N° 177, juillet 1992, 70-77.

L'action cytotoxique de l'alloxane, à l'origine de la création du test du diabète alloxanique décrit ci-après, est classiquement reliée à une production excessive de radicaux libres oxygénés: HO·, 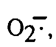 H₂O₂. Cette observation est par ailleurs corroborée par le fait que l'action diabétogène de l'alloxane est prévenue par des enzymes (catalase, SuperOxydeDismutase) ou des réactifs (alcools, DMSO, diméthylurée, thiourée, vitamine E, vitamine C,...) capteurs de radicaux libres.

Il est par conséquent reconnu qu'une substance capable de s'opposer à l'apparition du diabète alloxanique possède des propriétés antiradicalaires.

En utilisant ce modèle chez le rat, BENTUE-FERRER et al. (Fundam. Clin. Pharmacol., 1989, **3**, 323-328) ont montré que l'exifone exerce des propriétés antiradicalaires marquées après administration intraveineuse.

Ce test du diabète alloxanique chez le rat, tel que décrit ci-après, a été utilisé pour étudier les propriétés antiradicalaires des composés MBF 378, MBF 379, et MBF 380 (correspondant respectivement aux composés n° 2, n° 11, et n° 12 de l'invention et décrits ci-dessus), en comparaison avec l'Exifone.

### A) Matériel et méthodes

### - animaux

Les expériences ont été réalisées sur des rats mâles Wistar pesant entre environ 180g et environ 200 g au début des expériences.

### - protocole expérimental

Les animaux ont été privés de nourriture pendant 18 heures avant injection intraveineuse (i.v.) d'alloxane, et nourris 3 heures après cette injection.

Au premier jour de l'expérience, les animaux ont été légèrement anesthésiés à l'éther. Ils ont ensuite reçu une injection i.v. d'alloxane à 40 mg/kg, puis, 30 secondes plus tard, une injection du composé testé ou du véhicule.

Au second jour, c'est à dire 24 heures plus tard, les animaux ont été anesthésiés à nouveau (selon la même procédure), et ont reçu une seconde injection i.v. du composé testé ou du véhicule.

Un prélèvement de sang a été effectué 1 heure avant l'injection d'alloxane, c'est à dire avant l'anesthésie du premier jour. Un second prélèvement de sang a été effectué 48 heures après l'injection d'alloxane, c'est à dire 24 heures après la seconde injection du composé testé ou du véhicule.

### - traitements

Les rats ont été choisis au hasard pour subir les différents traitements suivants:
1: groupe de contrôle (10 rats) traité par l'alloxane et NaCI (Tableau 1)
2: groupe de contrôle (10 rats) traité par l'alloxane et du polyéthylène glycol 300 (PEG 300) (Tableau2)
3: groupe (13 rats) traité par l'alloxane et Exifone 60 mg/kg (Tableau 3)
4: groupe (10 rats) traité par l'alloxane et MBF 378 60 mg/kg (Tableau 4)
5: groupe (10 rats) traité par l'alloxane et MBF 379 60 mg/kg (Tableau 5)
6: groupe (10 rats) traité par l'alloxane et MBF 380 60 mg/kg (Tableau 6)
7: groupe (10 rats) traité par l'alloxane et MBF 378 30 mg/kg (Tableau 7)
8: groupe (10 rats) traité par l'alloxane et MBF 379 30 mg/kg (Tableau 8)
9: groupe (10 rats) traité par l'alloxane et MBF 380 30 mg/kg (Tableau 9)
10: groupe (4 rats) traité par l'alloxane et MBF 379 15 mg/kg (Tableau 10)

Les composés testés ont été injectés en une fois à raison de 0,2 ml/100g de poids corporel dans chaque cas. Ils ont été mis en solution dans un mélange de PEG 300 (Sigma) et d'eau distillée (solution aqueuse à 50%).

### - dosage du glucose

Les prélèvements sanguins de 1,5 ml ont été collectés dans des tubes contenant de l'héparine. Le glucose plasmatique (g/l) a été dosé par la technique de polarisation par fluorescence (TDX Abbott).

### B) Résultats

Les résultats sont présentés sur les tableaux 1 à 11 qui suivent (le tableau 11 représentant la moyenne des résultats obtenus avec les différents groupes 1 à 10 étudiés)

L'alloxane induit une augmentation très significative des niveaux plasmatiques de glucose dans les groupes traités par NaCI (Tableau 1; 0,46 ± 0,05 g/l avant injection d'alloxane, contre 5,13 ± 0,28 g/l après injection d'alloxane), ou par le PEG 300 (Tableau 2; 0,51 ± 0,05 g/l avant injection d'alloxane, contre 4,43 ± 0,30 g/l après injection d'alloxane).

Les autres groupes traités ont également présentés une augmentation significative des niveaux plasmatiques de glucose. L'Exifone (Tableau 3: 1,29 ± 0,06 g/l), et les trois dérivés MBF 378 (Tableau 4: 1,35 ± 0,02 g/l), MBF 379 (Tableau 5: 1,28 ± 0,04 g/l), et MBF 380 (Tableau 6: 1,66 ± 0,20 g/l), à 60 mg/kg, ainsi que le composé MBF 379 à 30 mg/kg (Tableau 8: 1,85 ± 0,39 g/l), ont permis de réduire de façon très significative les diabètes induits par l'alloxane dans les différents groupes étudiés. A un niveau moindre, MBF 378 et MBF 380 à 30 mg/kg se sont également révélés être des protecteurs efficaces contre le diabète alloxanique (Tableau 7: 3,42 ± 0,36 g/l, et Tableau 9: 4,04 ± 0,36 g/l, respectivement).

Il convient de noter également que le PEG 300 ne modifie pas de façon significative les effets de l'alloxane (Tableau 2: 4,43 ± 0,30 g/l, Tableau 1: 5,13 ± 0,28 g/l, respectivement)

En conclusion, les effets protecteurs de l'Exifone et des trois composés étudiés dans le test ci-dessus, sont dus à leur pouvoir de capteurs de radicaux libres. Si l'on prend en compte les rapports entre les poids moléculaires de ces trois composés MBF 378, MBF 379 et MBF 380, et le poids moléculaire de l'Exifone (à savoir: 1,78, 1,73 et 1,30, respectivement), on peut en conclure que ces trois composés sont plus actifs que l'Exifone, et que le MBF 379 est le composé le plus actif parmi ceux étudiés.

**Tableau 1 :**

| Valeurs de la glycémie 1h avant et 48h après injection i.v. de 40 mg/kg d'alloxane chez les rats. **Groupe contrôle NaCl 9‰. Résultats individuels.** Moyennes (M) ± erreur standard à la moyenne (SEM) | | |
|---|---|---|
| **RAT** | **Glycémie (g/l)** | |
| | **Avant alloxane** | **Après alloxane** |
| **1** | 0.48 | 6.91 |
| **2** | 0.58 | 5.68 |
| **3** | 0.48 | 5.43 |
| **4** | 0.52 | 4.63 |
| **5** | 0.26 | 4.07 |
| **6** | 0.20 | 4.54 |
| **7** | 0.32 | 5.98 |
| **8** | 0.48 | 4.30 |
| **9** | 0.55 | 4.65 |
| **10** | 0.74 | 5.07 |
| **M±SEM** | **0.46 ± 0.05** | **5.13 ± 0.28** |

**Tableau 2 :**

| Valeurs de la glycémie 1h avant et 48h après injection i.v. de 40 mg/kg d'alloxane chez les rats. **Groupe contrôle PEG 300 - résultats individuels.** Moyennes ± erreur standard à la moyenne | | |
|---|---|---|
| **RAT** | **Glycémie (g/l)** | |
| | **Avant alloxane** | **Après alloxane** |
| **1** | 0.67 | 2.23 |
| **2** | 0.69 | 5.27 |
| **3** | 0.53 | 5.22 |
| **4** | 0.53 | 5.46 |
| **5** | 0.32 | 4.06 |
| **6** | 0.35 | 3.76 |
| **7** | 0.74 | 4.22 |
| **8** | 0.39 | 4.67 |
| **9** | 0.40 | 4.69 |
| **10** | 0.49 | 4.71 |
| **M±SEM** | **0.51 ± 0.05** | **4.43 ± 0.30** |

**Tableau 3 :**

| Valeurs de la glycémie 1h avant et 48h après injection i.v. de 40 mg/kg d'alloxane chez les rats. **Groupe Exifone 60 mg/kg i.v. - Résultats individuels.** Moyennes ± erreur standard à la moyenne | | |
|---|---|---|
| **RAT** | **Glycémie (g/l)** | |
| | **Avant alloxane** | **Après alloxane** |
| **1** | 0.84 | 1.33 |
| **2** | 0.80 | exitus |
| **3** | 0.98 | 1.43 |
| **4** | 0.82 | 1.31 |
| **5** | 0.83 | exitus |
| **6** | 0.51 | 1.52 |
| **7** | 0.34 | 1.40 |
| **8** | 0.37 | 0.93 |
| **9** | 0.34 | exitus |
| **10** | 0.32 | 1.17 |
| **11** | 0.42 | exitus |
| **12** | 0.70 | exitus |
| **13** | 0.76 | 1.22 |
| **M±SEM** | **0.62 ± 0.07** | **1.29 ± 0.06** |

**Tableau 4 :**

| Valeurs de la glycémie 1h avant et 48h après injection i.v. de 40 mg/kg d'alloxane chez les rats. **Groupe MBF 378 60 mg/kg i.v. - Résultats individuels.** Moyennes ± erreur standard à la moyenne | | |
|---|---|---|
| **RAT** | **Glycémie (g/l)** | |
| | **Avant alloxane** | **Après alloxane** |
| **1** | 0.52 | 1.20 |
| **2** | 0.68 | 1.39 |
| **3** | 0.87 | 1.33 |
| **4** | 0.80 | 1.44 |
| **5** | 0.67 | 1.35 |
| **6** | 0.31 | 1.32 |
| **7** | 0.38 | 1.32 |
| **8** | 0.29 | 1.36 |
| **9** | 0.41 | 1.42 |
| **10** | 0.29 | 1.39 |
| **M±SEM** | **0.52 ± 0.07** | **1.35 ± 0.02** |

**Tableau 5 :**

| Valeurs de la glycémie 1h avant et 48h après injection i.v. de 40 mg/kg d'alloxane chez les rats. **Groupe MBF 379 60 mg/kg i.v. - Résultats individuels.** Moyennes ± erreur standard à la moyenne | | |
|---|---|---|
| **RAT** | **Glycémie (g/l)** | |
| | **Avant alloxane** | **Après alloxane** |
| **1** | 0.40 | 1.38 |
| **2** | 0.39 | 1.39 |
| **3** | 0.50 | 1.31 |
| **4** | 0.50 | 1.45 |
| **5** | 0.31 | 1.37 |
| **6** | 0.46 | 1.34 |
| **7** | 0.32 | 1.20 |
| **8** | 0.45 | 1.08 |
| **9** | 0.36 | 1.04 |
| **10** | 0.37 | 1.27 |
| **M±SEM** | **0.41 ± 0.02** | **1.28 ± 0.04** |

**Tableau 6 :**

| Valeurs de la glycémie 1h avant et 48h après injection i.v. de 40 mg/kg d'alloxane chez les rats. **Groupe MBF 380 60 mg/kg i.v. - Résultats individuels.** Moyennes ± erreur standard à la moyenne | | |
|---|---|---|
| **RAT** | **Glycémie (g/l)** | |
| | **Avant alloxane** | **Après alloxane** |
| **1** | 0.30 | 1.44 |
| **2** | 0.39 | 1.54 |
| **3** | 0.34 | 1.66 |
| **4** | 0.43 | 1.30 |
| **5** | 0.35 | 1.28 |
| **6** | 0.67 | 1.36 |
| **7** | 0.42 | 1.46 |
| **8** | 0.65 | 1.78 |
| **9** | 0.51 | 3.37 |
| **10** | 0.39 | 1.45 |
| **M±SEM** | **0.44 ± 0.04** | **1.66 ± 0.20** |

**Tableau 7 :**

| Valeurs de la glycémie 1h avant et 48h après injection i.v. de 40 mg/kg d'alloxane chez les rats. **Groupe MBF 378 30 mg/kg i.v. - Résultats individuels.** Moyennes ± erreur standard à la moyenne | | |
|---|---|---|
| **RAT** | **Glycémie (g/l)** | |
| | **Avant alloxane** | **Après alloxane** |
| **1** | 0.32 | 1.10 |
| **2** | 0.29 | 2.61 |
| **3** | 0.38 | 3.91 |
| **4** | 0.42 | 2.74 |
| **5** | 0.33 | 3.08 |
| **6** | 0.31 | 2.84 |
| **7** | 0.63 | 4.44 |
| **8** | 0.53 | 4.63 |
| **9** | 0.53 | 4.60 |
| **10** | 0.81 | 4.26 |
| **M±SEM** | **0.45 ± 0.05** | **3.42 ± 0.36** |

**Tableau 8 :**

| Valeurs de la glycémie 1h avant et 48h après injection i.v. de 40 mg/kg d'alloxane chez les rats. **Groupe MBF 379 30 mg/kg i.v. - Résultats individuels.** Moyennes ± erreur standard à la moyenne | | |
|---|---|---|
| **RAT** | **Glycémie (g/l)** | |
| | **Avant alloxane** | **Après alloxane** |
| **1** | 0.28 | 1.07 |
| **2** | 0.64 | 1.29 |
| **3** | 0.31 | 1.18 |
| **4** | 0.31 | 1.20 |
| **5** | 0.44 | 1.27 |
| **6** | 0.52 | 1.09 |
| **7** | 0.52 | 4.36 |
| **8** | 0.61 | 4.04 |
| **9** | 0.51 | 1.42 |
| **10** | 0.85 | 1.59 |
| **M±SEM** | **0.50 ± 0.06** | **1.85 ± 0.39** |

**Tableau 9 :**

| Valeurs de la glycémie 1h avant et 48h après injection i.v. de 40 mg/kg d'alloxane chez les rats. **Groupe MBF 380 30 mg/kg i.v. - Résultats individuels.** Moyennes ± erreur standard à la moyenne | | |
|---|---|---|
| **RAT** | **Glycémie (g/l)** | |
| | **Avant alloxane** | **Après alloxane** |
| **1** | 0.34 | 3.73 |
| **2** | 0.30 | 3.76 |
| **3** | 0.34 | 4.12 |
| **4** | 0.34 | 4.68 |
| **5** | 0.63 | 4.94 |
| **6** | 0.54 | 4.81 |
| **7** | 0.51 | 1.42 |
| **8** | 0.55 | 3.05 |
| **9** | 0.59 | 4.55 |
| **10** | 0.67 | 5.38 |
| **M±SEM** | **0.48 ± 0.04** | **4.04 ± 0.36** |

**Tableau 10 :**

| Valeurs de la glycémie 1h avant et 48h après injection i.v.de 40 mg/kg d'alloxane chez les rats. **Groupe MBF 379 15 mg/kg i.v. - Résultats individuels.** Moyennes ± erreur standard à la moyenne | | |
|---|---|---|
| **RAT** | **Glycémie (g/l)** | |
| | **Avant alloxane** | **Après alloxane** |
| **1** | 0.85 | 3.70 |
| **2** | 0.85 | 3.61 |
| **3** | 0.94 | 5.74 |
| **4** | 0.75 | 2.53 |
| **M±SEM** | **0.85 ± 0.04** | **3.89 ± 0.67** |

**Tableau 11 :**

| Valeurs de la glycémie 1h avant et 48h après injection i.v. de 40 mg/kg d'alloxane chez les rats. **Effets de l'exifone et de ses trois dérivés.** Moyennes ± erreur standard à la moyenne avec 10 animaux par groupe sauf pour exifone 60 mg/kg et MBF 379 15 mg/kg avec 8 et 4 animaux, respectivement. | | |
|---|---|---|
| **GROUPE** | **Glycémie (g/l)** | |
| | **Avant alloxane** | **Après alloxane** |
| **Contrôle (NaCI 9‰)** | 0.46 ± 0.05 | 5.13 ± 0.28 |
| **Contrôle (PEG 300)** | 0.51 ± 0.05 | 4.43 ± 0.30 |
| **Exifone 60 mg/kg** | 0.62 ± 0.07 | 1.29 ± 0.06 |
| **MBF 378 60 mg/kg** | 0.52 ± 0.07 | 1.35 ± 0.02 |
| **MBF 379 60 mg/kg** | 0.41 ± 0.02 | 1.28 ± 0.04 |
| **MBF 380 60 mg/kg** | 0.44 ± 0.04 | 1.66 ± 0.20 |
| **MBF 378 30 mg/kg** | 0.45 ± 0.05 | 3.42 ± 0.36 |
| **MBF 379 30 mg/kg** | 0.50 ± 0.06 | 1.85 ± 0.39 |
| **MBF 380 30 mg/kg** | 0.48 ± 0.04 | 4.04 ± 0.36 |
| **MBF 379 15 mg/kg** | 0.85 ± 0.04 | 3.89 ± 0.67 |

## Revendications

1. Composés répondant à la formule générale (I) suivante: 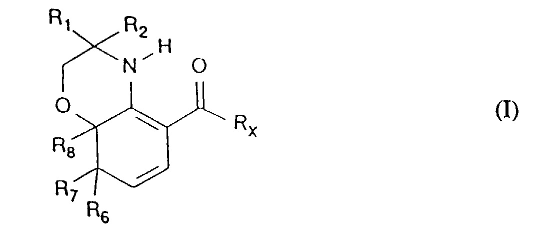 dans laquelle:
- R₁ et R₂, indépendamment l'un de l'autre, représentent un groupe alkyle de 1 à 3 atomes de carbone, ou un groupe de formule -CH₂-ORₐ, Rₐ représentant un atome d'hydrogène ou un groupe méthyle,
- Rₓ représente:
. un groupe alkyle de 1 à 3 atomes de carbone, ou
. un groupe -ORₓ₁, Rₓ₁ représentant un groupe alkyle de 1 à 3 atomes de carbone, ou
. un groupe de formule -CH₂-X dans laquelle X représente un atome d'halogène, ou
. un groupe de formule 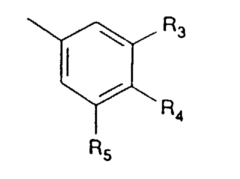 dans laquelle:
. R₃ et R₅, indépendamment l'un de l'autre, représentent un atome d'hydrogène, ou un groupe -OH, ou un groupe -OCH₃,
. R₄ représente un atome d'hydrogène, ou un groupe -OR_{b}, R_{b} représentant un atome d'hydrogène ou un groupe méthyle, ou R₄ représente un groupe de formule -OR_{c}, R_{c} représentant un groupe de formule -CH₂-CO-C₆H₄R' dans laquelle R' représente un atome d'hydrogène, -CN ou -OCH₃,
- soit R₆ représente un groupe -OR_{d}, R_{d} représentant un atome d'hydrogène ou un groupe méthyle, lorsque R₇ et R₈ s'associent pour former une double liaison,
- soit R₈ représente un groupe -ORₑ dans lequel Rₑ représentant un atome d'hydrogène ou un groupe méthyle, lorsque R₆ et R₇ s'associent pour former avec l'atome de carbone en position 8 du cycle, un groupe C = O.

2. Composés selon la revendication 1, caractérisés en ce qu'ils correspondent à la formule (I) dans laquelle Rₓ représente :
. un groupe méthyle, ou
. un groupe méthoxy, ou
. un groupe de formule -CH₂-X dans laquelle X représente un atome de chlore, ou
. un groupe de formule 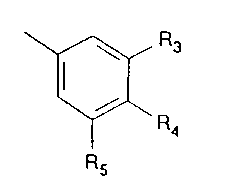 dans laquelle R₃, R₄ et R₅, sont tels que définis dans la revendication 1.

3. Composés selon la revendication 1 ou 2, caractérisés en ce qu'ils correspondent à la formule (II) suivante: 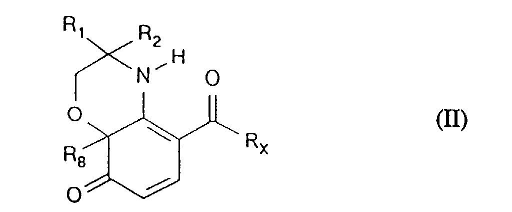 dans laquelle R₁, R₂, R₈ et Rₓ sont tels que définis dans la revendication 1.

4. Composés selon la revendication 1 ou 2, caractérisés en ce qu'ils correspondent à la formule (III) suivante: 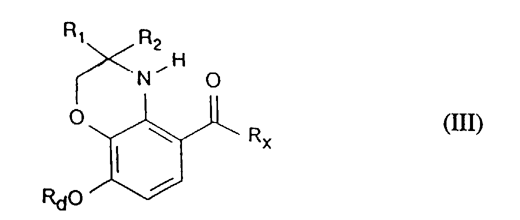 dans laquelle R₁, R₂, R_{d} et Rₓ sont tels que définis dans la revendication 1.

5. Composés selon la revendication 1 ou 2, caractérisés en ce qu'ils correspondent à la formule (IV) suivante: 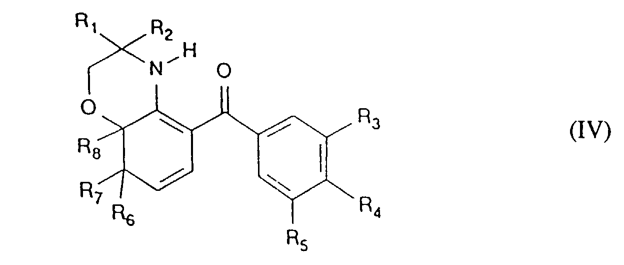 dans laquelle R₁ à R₈ sont tels que définis dans la revendication 1.

6. Composés selon la revendication 3 ou la revendication 5, caractérisés en ce qu'ils correspondent à la formule (V) suivante: 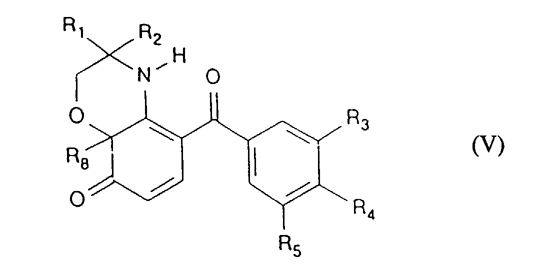 dans laquelle R₁ à R₅ et R₈ sont tels que définis dans la revendication 1.

7. Composés selon la revendication 6, caractérisés en ce qu'ils correspondent aux formules suivantes:

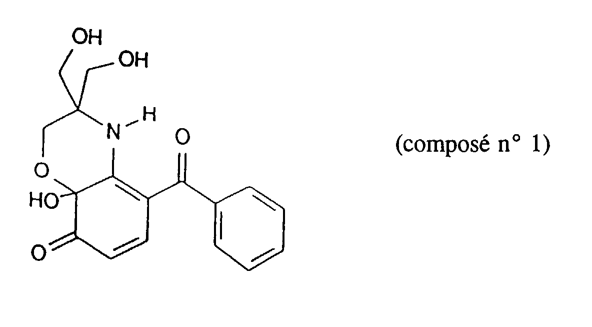

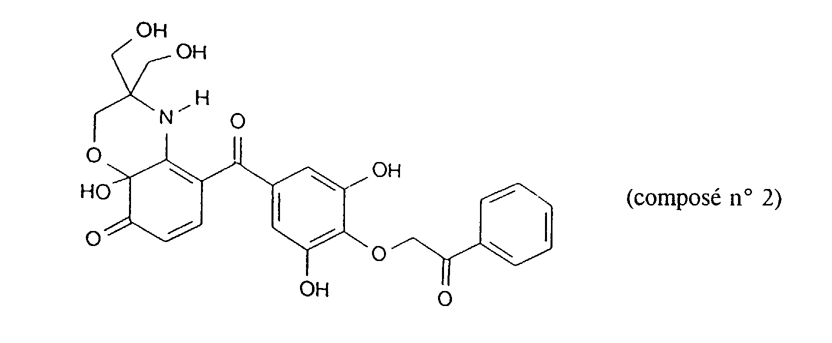

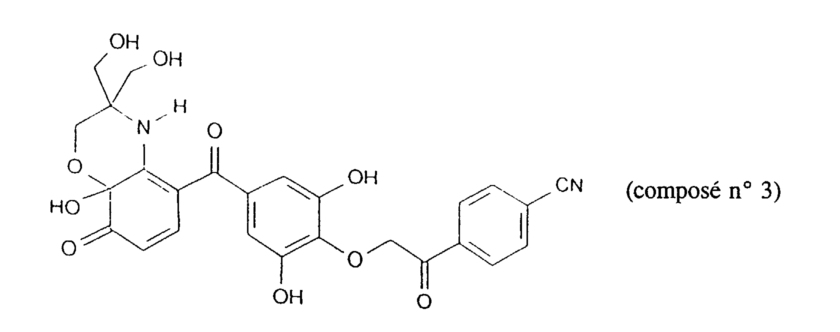

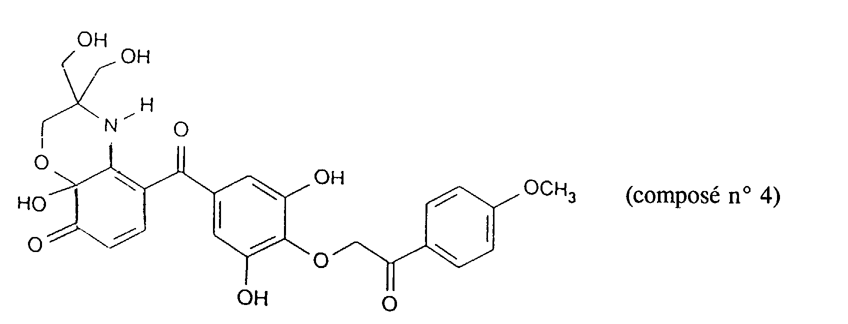

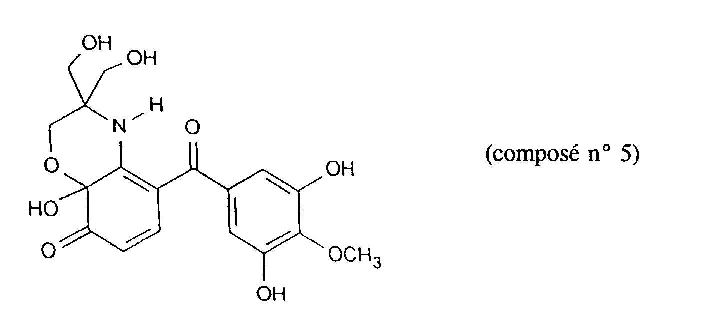

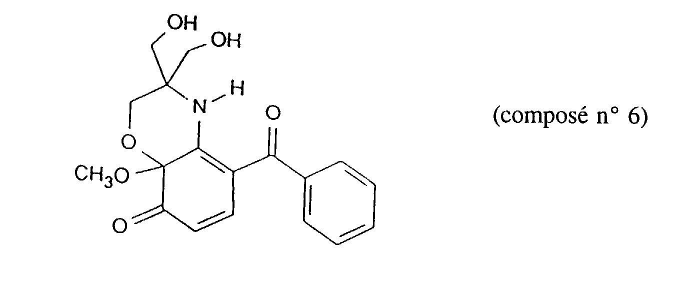

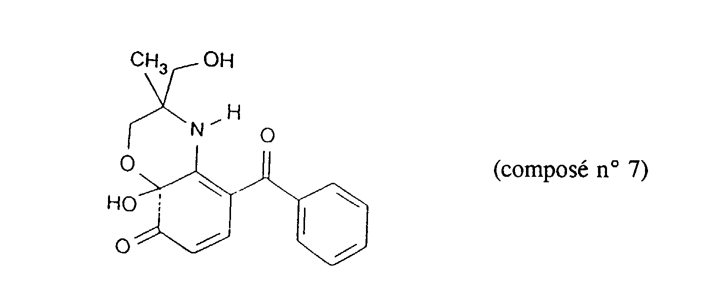

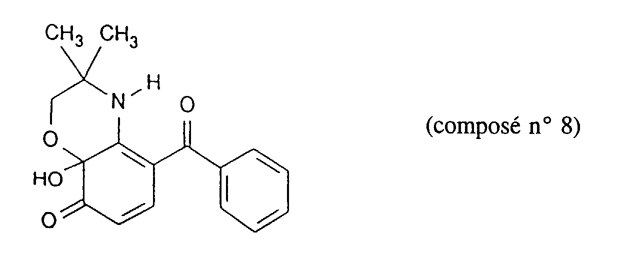

8. Composés selon la revendication 1 ou 2, caractérisés en ce qu'ils correspondent à la formule (VI) suivante: 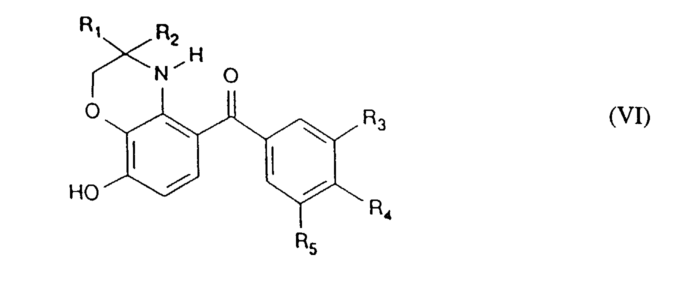 dans laquelle R₁, R₂, R₃, R₄ et R₅ sont tels que définis dans la revendication 1.

9. Composés selon la revendication 8, caractérisés en ce qu'ils correspondent aux formules suivantes:

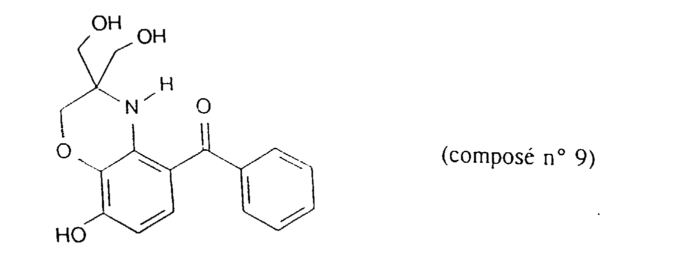

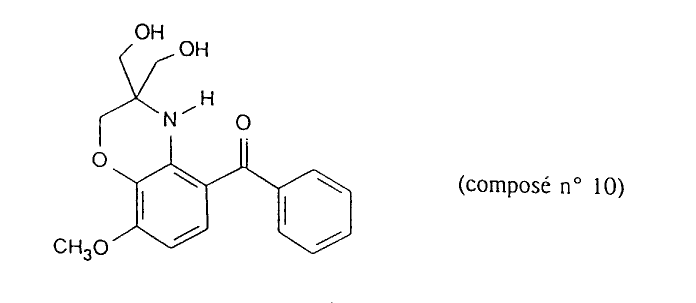

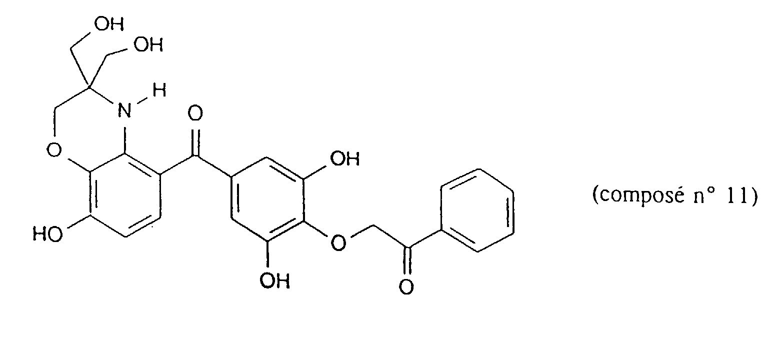

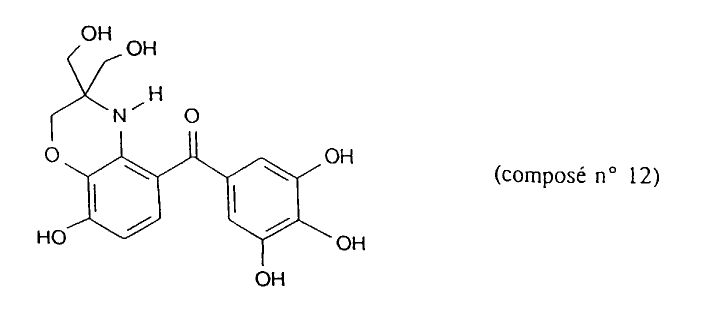

10. Composé selon la revendication 1 ou 2, caractérisé en ce qu'il correspond à la formule (VII) suivante: 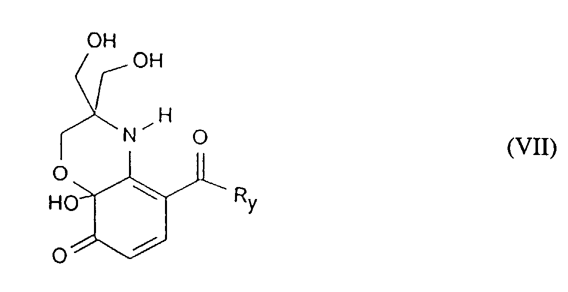 dans laquelle R_{y} représente:
- un groupe méthyle (composé n° 13), ou
- un groupe méthoxy (composé n° 14), ou
- un groupe -CH₂Cl (composé n° 15)

11. Composition pharmaceutique, caractérisée en ce qu'elle comprend, à titre de principe actif, au moins un composé selon l'une des revendications 1 à 10, en association avec un véhicule pharmaceutiquement acceptable.

12. Composition pharmaceutique selon la revendication 11, caractérisée en ce qu'elle est sous une forme administrable par voie orale, notamment sous forme de comprimés, ou de gélules, ou sous une forme administrable par voie parentérale, notamment sous forme de préparation injectable par voie IV, IM, ou SC.

13. Composition pharmaceutique selon la revendication 12, caractérisée en ce que la posologie journalière pour les formes orales est d'environ 3 mg/kg à environ 20 mg/kg, de préférence environ 15 mg/kg, et en ce que la posologie journalière pour les formes parentérales est d'environ 1 mg/kg à environ 5 mg/kg.

14. Composition pharmaceutique selon l'une des revendications 11 à 13, caractérisée en ce que les doses unitaires de principe actif en fonction des différentes formes de présentation de ladite composition, sont les suivantes:
- forme orale: environ 1 mg/kg à environ 10 mg/kg, de préférence environ 5 mg/kg,
- forme parentérale: environ 0,3 mg/kg à environ 1 mg/kg.

15. Utilisation d'au moins un composé selon l'une des revendications 1 à 10, pour l'obtention d'un médicament destiné au traitement des troubles cognitifs, tels que les troubles de la mémoire, notamment chez les sujets vieillissants.

16. Utilisation selon la revendication 15, pour l'obtention d'un médicament destiné au traitement des affections cérébrales susceptibles d'être dues, et/ou entretenues, voire aggravées, par la présence de radicaux libres dans le cerveau, et plus particulièrement au traitement de la maladie d'Alzheimer, de la maladie de Parkinson, de la Trisomie 21 (syndrome de Down), de la schizophrénie et de l'épilepsie.

17. Procédé de préparation de composés selon l'une des revendications 1 à 10, caractérisé en ce qu'il comprend les étapes suivantes:
a) traitement du dérivé de formule (VIII) suivante: 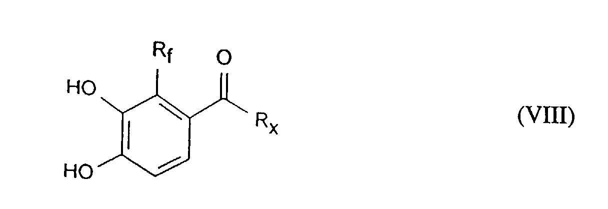 dans laquelle R_{f} représente H ou OH, et Rₓ est tel que défini dans la revendication 1,
. soit par voie chimique par mise en présence dudit dérivé de formule (VIII) avec un dérivé de formule NH₂-C(R₁,R₂)-CH₂OH dans laquelle R₁ et R₂ sont tels que définis dans la revendication 1, en présence d'un oxyde de métal, choisi notamment parmi Ag₂O, AgO et PtO₂, en milieu alcoolique, de préférence méthanolique,
. soit par voie électrochimique par électrolyse à potentiel contrôlé dudit dérivé de formule (VIII) en présence d'un dérivé de formule NH₂-C(R₁,R₂)-CH₂OH tel que décrit ci-dessus, en solution alcoolique, de préférence méthanolique, et en présence d'un électrolyte support, choisi notamment parmi les sels de tétraéthylammonium,
ce qui conduit à l'obtention d'un composé de formule (II) suivante: 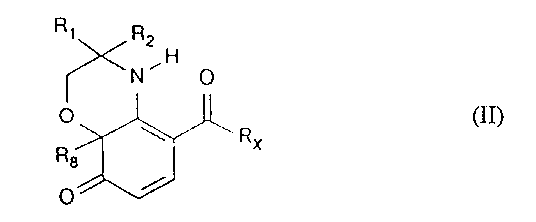 dans laquelle R₈ représente un groupe -OH, et R₁, R₂ et Rₓ sont tels que définis dans la revendication 1,
sous réserve que lorsque Rₓ du dérivé de la formule (VIII) susmentionnée, représente un groupe de formule 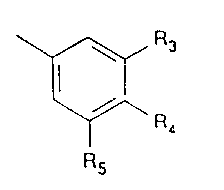 dans laquelle R₄ représente un groupe -OH, et l'un au moins des groupements R₃ ou R₅ représente un groupe -OH, ce qui correspond au dérivé de formule (VIII bis), l'étape de traitement chimique ou électrochimique décrite ci-dessus est précédée par une étape de protection du groupe -OH correspondant à R₄ dudit dérivé de formule (VIII bis), cette étape de protection étant réalisée par traitement du dérivé de formule (VIII bis) avec un dérivé de formule X¹-CH₃ ou X¹-R_{c} dans laquelle X¹ représente un atome d'halogène, notamment un atome d'iode ou de brome, et R_{c} est tel que défini dans la revendication 1,
ce qui conduit à l'obtention d'un dérivé de formule (VIII ter) correspondant à un dérivé de formule (VIII bis) dans laquelle l'un au moins de R₃ ou de R₅ représente -OH, tandis que R₄ représente un groupe -OR_{c}, R_{c} ayant la définition indiquée ci-dessus, ce dérivé de formule (VIII ter) étant ensuite traité par voie chimique ou électrochimique de la manière indiquée ci-dessus par un dérivé de formule NH₂-C(R₁,R₂)-CH₂OH en milieu alcoolique, ce qui conduit à l'obtention d'un composé de formule (II) susmentionnée dans laquelle R₈ représente un groupe -OH, et R₁ et R₂ sont tels que définis dans la revendication 1, et Rₓ représente un groupe de formule 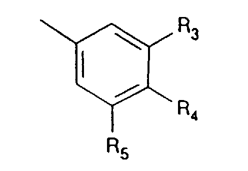 dans laquelle l'un au moins de R₃ ou de R₅ représente -OH, R₄ représente -OCH₃ ou -OR_{c}, R_{c} étant défini ci-dessus,
b) le cas échéant,
- soit alkylation du composé de formule (II) obtenu à l'étape a) précédente, par traitement de ce composé en milieu basique par un dérivé de formule X²-CH₃, dans laquelle X² représente un atome d'halogène, notamment l'iode, ce qui conduit à l'obtention d'un composé de formule (II) susmentionnée dans laquelle R₈ représente un groupe -OCH₃, et R₁, R₂ et Rₓ sont tels que définis dans la revendication 1,
- soit réduction du composé de formule (II) obtenu à l'étape a) précédente:
. soit par voie chimique par traitement du composé de formule (II) par une solution d'acide acétique en présence de Zn à température ambiante, ce qui conduit à l'obtention d'un composé de formule (III) suivante: 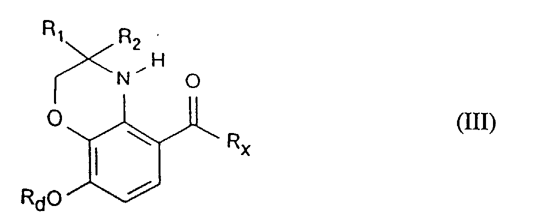 dans laquelle R_{d} représente H, et R₁, R₂, et Rₓ sont tels que définis dans la revendication 1,
sous réserve que lorsque le composé de formule (II) susmentionnée est tel que Rₓ représente un groupe de formule suivante: 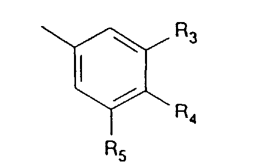 dans laquelle R₄ représente un groupe -OR_{c}, R_{c} étant tel que défini dans la revendication 1, et R₃ et R₅ étant tels que définis dans la revendication 1, la réduction par voie chimique susmentionnée est réalisée soit avec environ cinq équivalents de zinc, le temps de contact entre ledit composé de formule (II) et le zinc étant d'environ 2 minutes, ce qui conduit à l'obtention d'un composé de formule (III) susmentionnée, dans laquelle R₄, dans la définition de Rₓ, représente un groupe -OR_{c}, R_{c} étant tel que défini ci-dessus, et R₃ et R₅ étant tels que définis dans la revendication 1, soit avec environ cent équivalents de zinc, le temps de contact entre ledit composé de formule (II) et le zinc étant d'environ 10 minutes, ou avantageusement avec environ cinq équivalents du zinc, le temps de contact susmentionné étant alors d'environ 30 minutes, ce qui conduit, dans ces deux derniers cas, à l'obtention d'un composé de formule (III) susmentionnée dans laquelle R₄, dans la définition de Rₓ, représente un groupe -OH, et R₃ et R₅ étant tels que définis dans la revendication 1,
. soit par voie électrochimique, par électrolyse à potentiel contrôlé du composé de formule (II) susmentionnée en solution alcoolique, de préférence méthanolique, en présence d'un électrolyte support, choisi notamment parmi les sels de tétraéthylammonium, ce qui conduit à l'obtention du composé de formule (III) susmentionnée,
sous réserve que lorsque le composé de formule (II) susmentionnée est tel que Rₓ représente un groupe de formule suivante: 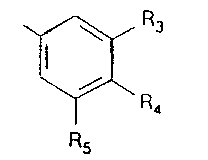 dans laquelle R₄ représente un groupe -OR_{c}, R_{c} étant tel que défini dans la revendication 1, et R₃ et R₅ étant tels que définis dans la revendication 1, le potentiel d'électrolyse est choisi soit de manière à obtenir un composé de formule (III) susmentionnée, dans laquelle R₄, dans la définition de Rₓ, représente un groupe -OR_{c}, R_{c} étant tel que défini ci-dessus, et R₃ et R₅ étant tels que définis dans la revendication 1, soit de manière à obtenir un composé de formule (III) susmentionnée dans laquelle R₄, dans la définition de Rₓ, représente un groupe -OH, et R₃ et R₅ étant tels que définis dans la revendication 1,
c) le cas échéant, alkylation du composé de formule (III) obtenu à l'étape b) précédente, par traitement de ce composé en milieu basique par un dérivé de formule X³-CH₃, dans laquelle X³ représente un atome d'halogène, notamment l'iode, ce qui conduit à l'obtention d'un composé de formule (III) susmentionnée dans laquelle R_{d} représente un groupe -OCH₃, et R₁, R₂ et Rₓ sont tels que définis dans la revendication 1.

18. Composés de formule (VIII) telle que décrite dans la revendication 17, et répondant aux formules suivantes:

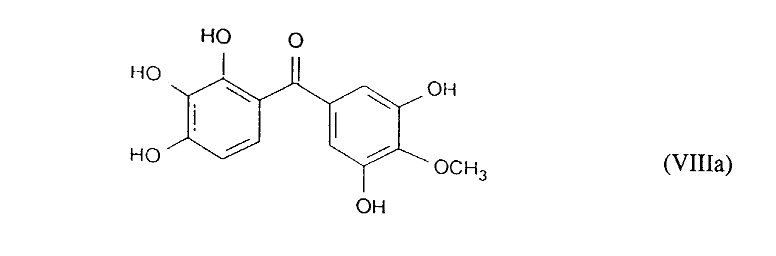

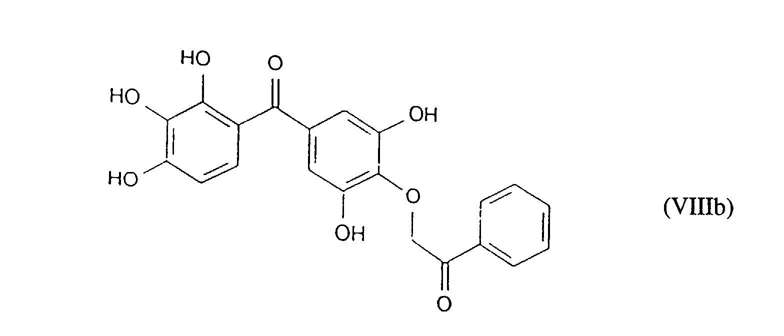

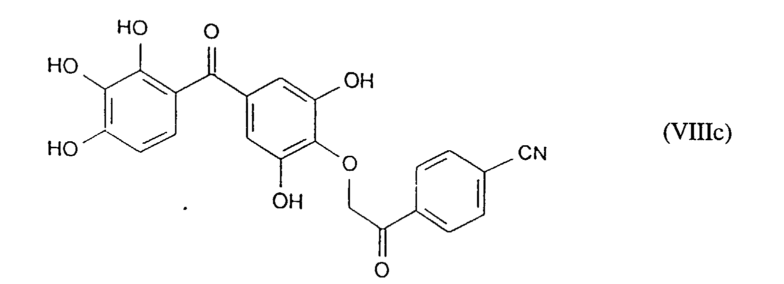

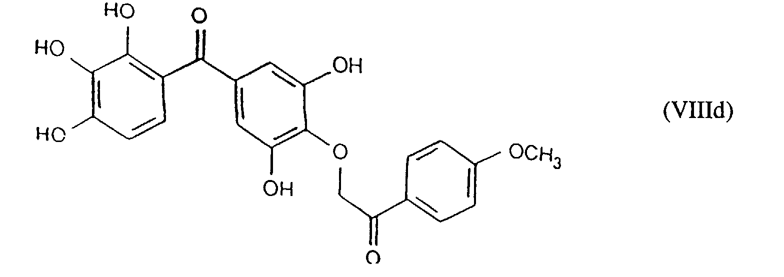

## Patentansprüche

1. Verbindungen, die der folgenden allgemeinen Formel (I) entsprechen: 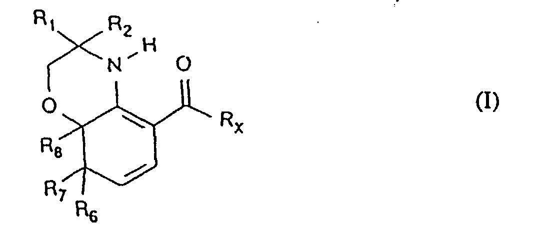
- in der:
- R₁ und R₂ unabhängig voneinander eine Alkylgruppe von ein bis drei Kohlenstoffatomen darstellen oder eine Gruppe der Formel -CH₂-ORₐ, wobei Rₐ ein Wasserstoffatom oder eine Methylgruppe darstellt.
- Rₓ darstellt:
- eine Alkylgruppe von ein bis drei Kohlenstoffatomen oder
- eine -ORₓ₁-Gruppe, in der Rₓ₁ eine Alkylgruppe von ein bis drei Kohlenstoffatomen darstellt oder
- eine Gruppe der Formel -CH₂-X, in der X ein Halogenatom darstellt oder
- eine Gruppe der Formel 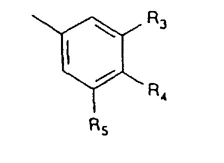 in der:
• R₃ und R₅ unabhängig voneinander ein Wasserstoffatom oder eine -OH-Gruppe oder eine -OCH₃-Gruppe darstellen,
• R₄ ein Wasserstoffatom darstellt oder eine -OR_{b}-Gruppe, in der R_{b} ein Wasserstoffatom oder eine Methylgruppe darstellt, oder R₄ eine Gruppe der Formel -OR_{c} darstellt, in der R_{c} eine Gruppe der Formel -CH₂-CO-C₆H₄R' darstellt, in der R' ein Wasserstoffatom, -CN, oder -OCH₃ darstellt,
- entweder R₆ eine -OR_{d}-Gruppe darstellt, in der R_{d} ein Wasserstoffatom oder eine Methylgruppe darstellt, wenn R₇ und R₈ durch eine Doppelbindung verbunden sind,
- oder R₈ eine -ORₑ-Gruppe darstellt, in der Rₑ ein Wasserstoffatom oder eine Methylgruppe darstellt, wenn R₆ und R₇ verbunden sind und mit dem Kohlenstoffatom in Position 8 des Rings eine C=O-Gruppe bilden.

2. Verbindungen nach Anspruch 1, dadurch gekennzeichnet, daß sie der Formel (I) entsprechen, in der Rₓ darstellt:
- eine Methylgruppe oder
- eine Methoxygruppe oder
- eine Gruppe der Formel -CH₂-X, in der X ein Chloratom darstellt oder
- eine Gruppe der Formel 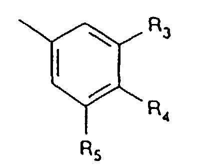 in der R₃, R₄ und R₅ entsprechend Anspruch 1 definiert sind.

3. Verbindungen nach Anspruch 1 oder 2, dadurch gekennzeichnet, daß sie der folgenden Formel (II) entsprechen: 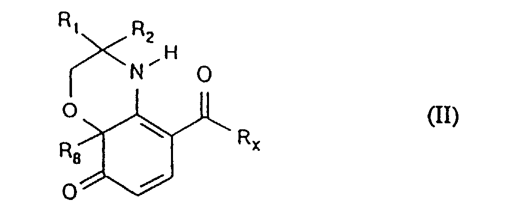 in der R₁, R₂, R₈ und Rₓ entsprechend Anspruch 1 definiert sind.

4. Verbindungen nach Anspruch 1 oder 2, dadurch gekennzeichnet, daß sie der folgenden Formel (III) entsprechen: 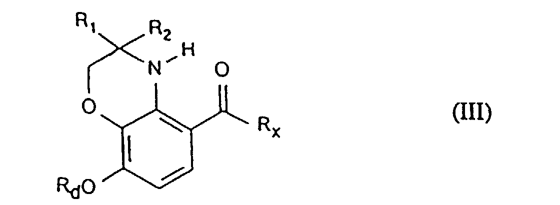 in der R₁, R₂, R_{d} und Rₓ entsprechend Anspruch 1 definiert sind.

5. Verbindungen nach Anspruch 1 oder 2, dadurch gekennzeichnet, daß sie der folgenden Formel (IV) entsprechen: 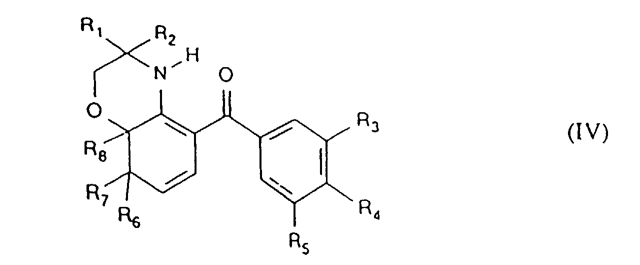 in der R₁ bis R₈ entsprechend Anspruch 1 definiert sind.

6. Verbindungen nach Anspruch 3 oder Anspruch 5, dadurch gekennzeichnet, daß sie der folgenden Formel (V) entsprechen: 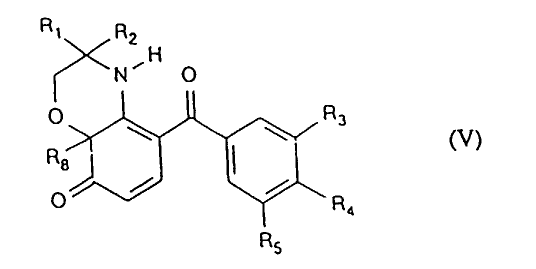 in der R₁ bis R₅ und R₈ entsprechend Anspruch 1 definiert sind.

7. Verbindungen nach Anspruch 6, dadurch gekennzeichnet, daß sie den folgenden Formeln entsprechen:

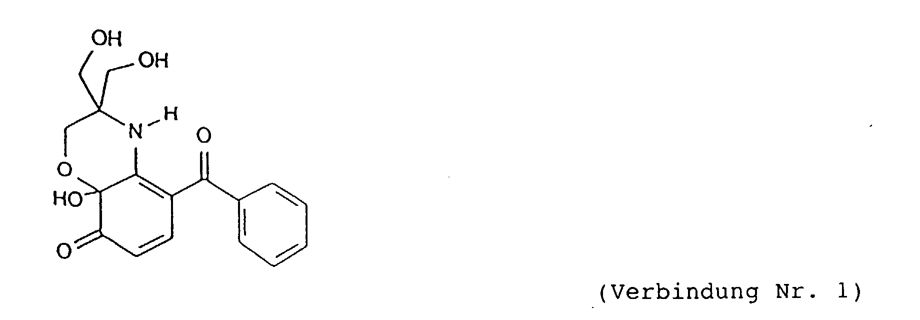

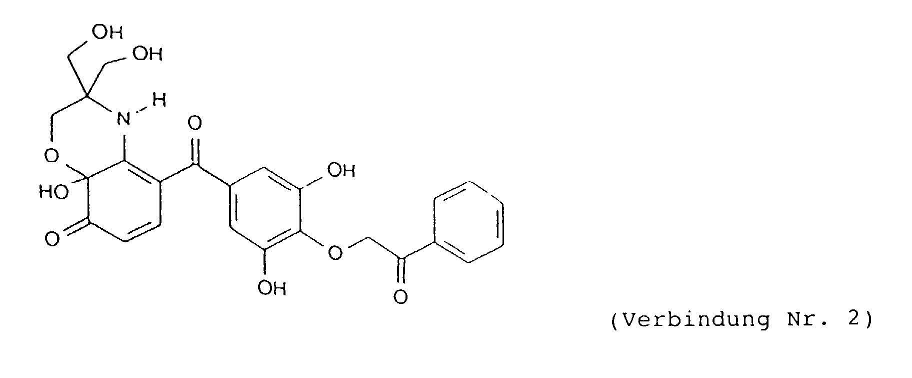

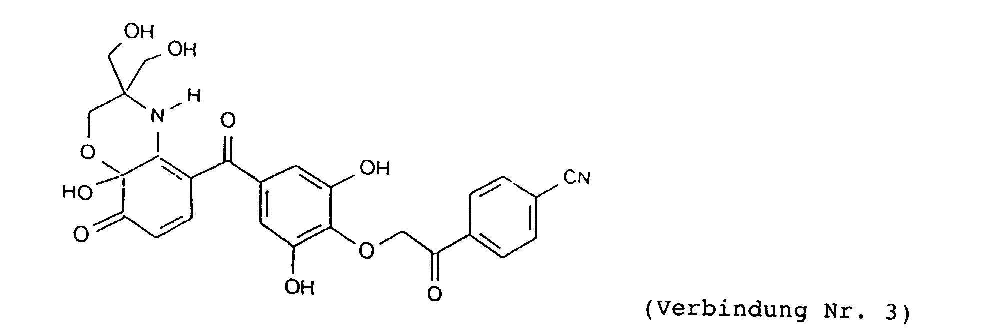

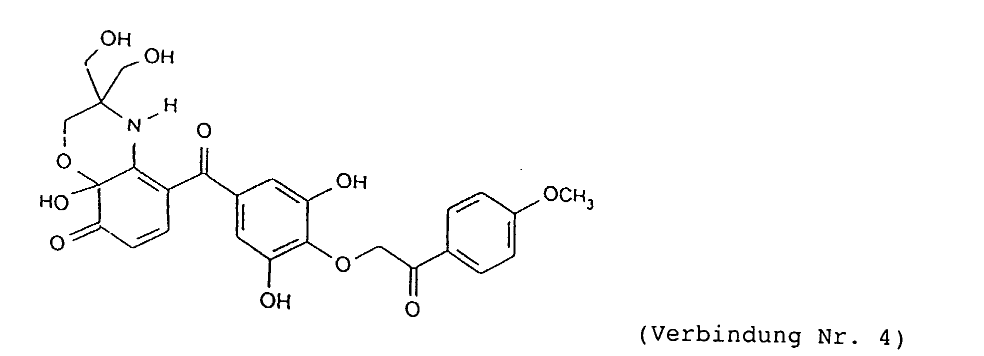

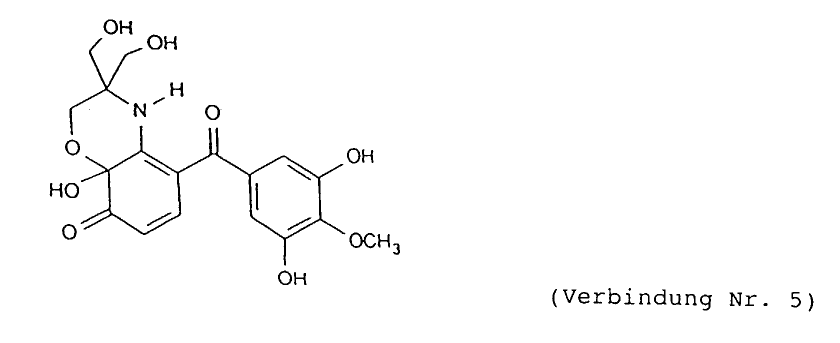

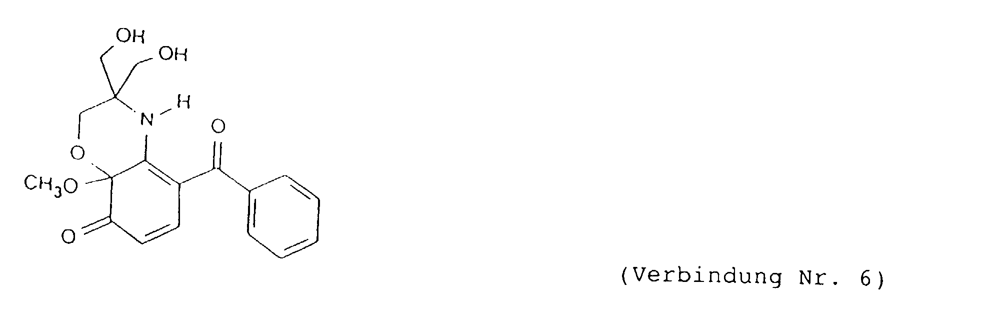

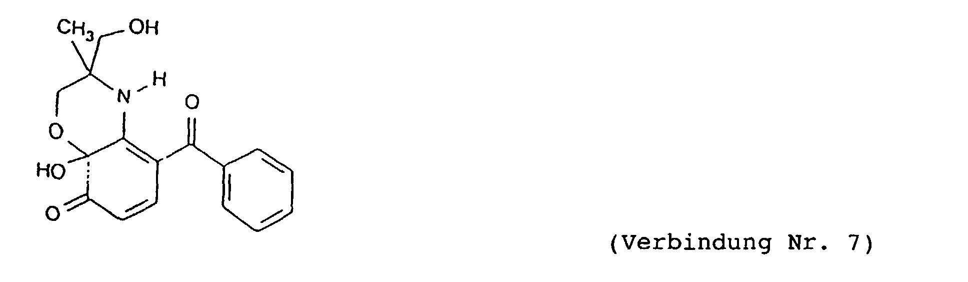

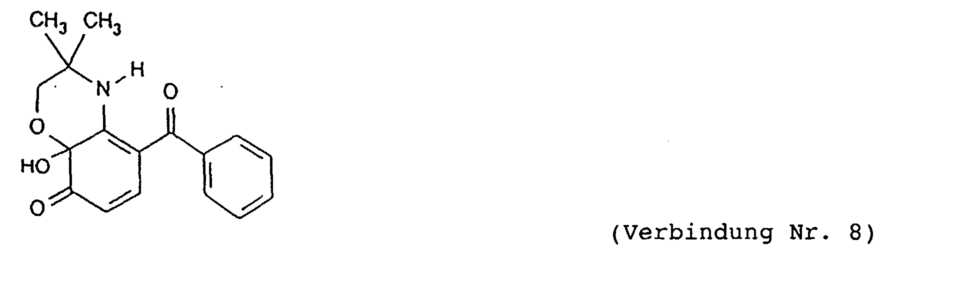

8. Verbindungen nach Anspruch 1 oder 2, dadurch gekennzeichnet, daß sie der folgenden Formel (VI) entsprechen: 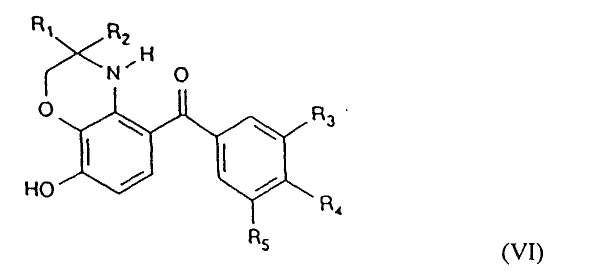 in der R₁, R₂, R₃, R₄ und R₅ entsprechend Anspruch 1 definiert sind.

9. Verbindungen nach Anspruch 8, dadurch gekennzeichnet, daß sie den folgenden Formeln entsprechen:

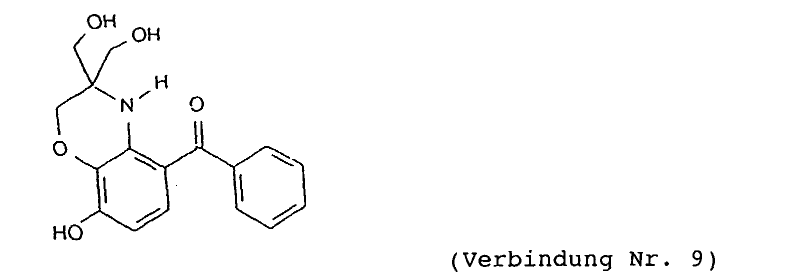

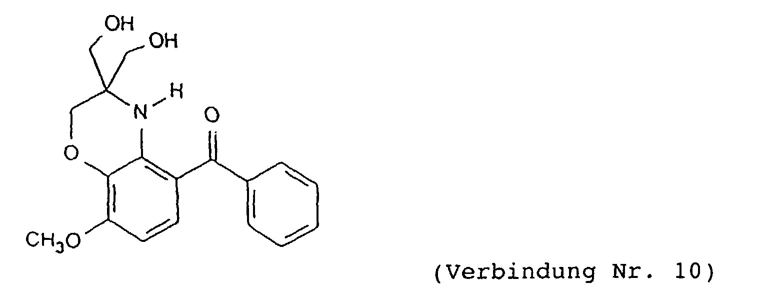

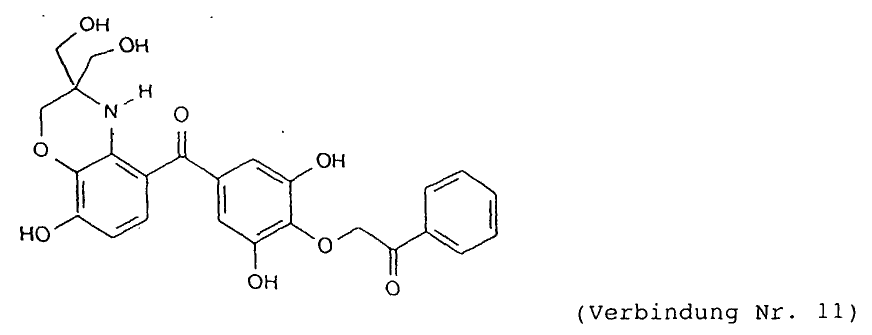

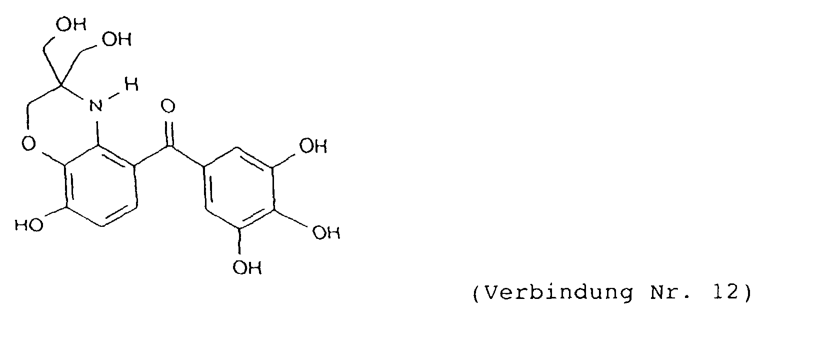

10. Verbindung nach Anspruch 1 oder 2, dadurch gekennzeichnet, daß sie der folgenden Formel (VII) entspricht: 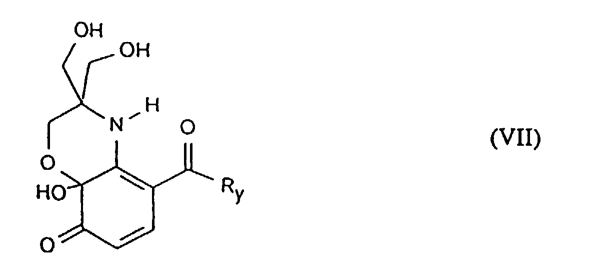 in der R_{y} darstellt:
- eine Methylgruppe (Verbindung Nr. 13)
- eine Methoxygruppe (Verbindung Nr. 14) oder
- eine -CH₂Cl-Gruppe (Verbindung Nr. 15)

11. Pharmazeutische Zusammensetzung, dadurch gekennzeichnet, daß sie als aktive Basis wenigstens eine Verbindung nach einem der Ansprüche 1 bis 10 enthält, in Kombination mit einem pharmazeutisch verträglichen Träger.

12. Pharmazeutische Zusammensetzung nach Anspruch 11, dadurch gekennzeichnet, daß sie auf oralem Weg verabreicht werden kann, insbesondere in Gestalt von Tabletten oder von Gelkapseln, oder daß sie auf parenteralem Weg verabreicht werden kann, insbesondere in Gestalt einer injizierbaren Zubereitung auf intravenösem, intramuskulärem oder subkutanem Weg.

13. Pharmazeutische Zusammensetzung nach Anspruch 12, dadurch gekennzeichnet, daß ihre tägliche Verabreichungsmenge für die oralen Formen zwischen ungefähr 3 mg/kg und ungefähr 20 mg/kg liegt, vorzugsweise ungefähr 15 mg/kg, und dadurch, daß ihre tägliche Verabreichungsmenge für die parenteralen Formen zwischen ungefähr 1 mg/kg und ungefähr 5 mg/kg liegt.

14. Pharmazeutische Zusammensetzung nach einer der Ansprüche 11 bis 13, dadurch gekennzeichnet, daß ihre Einheitsdosen der aktiven Basis in Abhängigkeit der verschiedenen Formen der Verabreichung der Zusammensetzung wie folgt sind:
- orale Form: ungefähr 1 mg/kg bis ungefähr 10 mg/kg, vorzugsweise ungefähr 5 mg/kg,
- parenterale Form: ungefähr 0,3 mg/kg bis ungefähr 1 mg/kg.

15. Verwendung wenigstens einer Verbindung nach einer der Ansprüche 1 bis 10, um ein Medikament zu erhalten, das zur Behandlung von Wahrnehmungsstörungen, wie Gedächtnisstörungen, insbesondere auf dem Gebiet der Alterung, bestimmt ist.

16. Verwendung nach Anspruch 15, um ein Medikament zu erhalten, das zur Behandlung von Gehirnkrankheiten bestimmt ist, die hervorgerufen und/oder begünstigt und sogar erschwert werden können durch die Anwesenheit von freien Radikalen im Gehirn, und im besonderen zur Behandlung der Alzheimerkrankheit, der Parkinsonkrankheit, der Trisomie 21 (Down-Syndrom), der Schizophrenie und der Epilepsie.

17. Verfahren zur Herstellung der Verbindungen nach einem der Ansprüche 1 bis 10, dadurch gekennzeichnet, daß es die folgenden Schritte umfaßt:
a) Behandlung des Derivats der folgenden Formel (VIII): 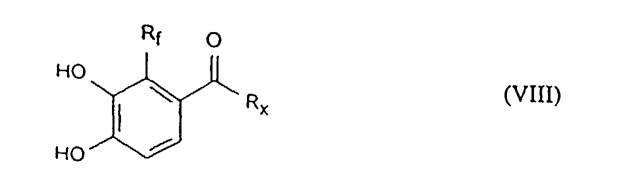 in der R_{f} H oder OH darstellt und Rₓ entsprechend Anspruch 1 definiert ist,
- entweder auf chemischen Weg durch Umsetzung des Derivats der Formel (VIII) mit einem Derivat der Formel NH₂-C(R₁,R₂)-CH₂OH, in der R₁ und R₂ entsprechend Anspruch 1 definiert sind, in Gegenwart eines Metalloxids, das insbesondere aus Ag₂O, AgO und PtO₂ ausgewählt wird, in alkoholischem, vorzugsweise methanolischem Milieu.
- oder auf elektrochemischen Weg durch Elektrolyse bei kontrolliertem Potential des Derivats der Formel (VIII) in Gegenwart eines Derivats der oben beschriebenen Formel NH₂-C(R₁,R₂)-CH₂OH in alkoholischer, vorzugsweise methanolischer Lösung und in Gegenwart eines Trägerelektrolyts, der insbesondere aus Tetraethylammonium-Salzen ausgewählt wird,
wodurch eine Verbindung der folgenden Formel (II) erhalten wird: 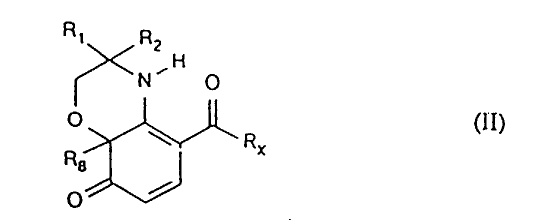 in der R₈ eine -OH-Gruppe darstellt und R₁, R₂ und Rₓ entsprechend Anspruch 1 definiert sind,
mit der Maßgabe, daß wenn Rₓ des Derivats mit der oben genannten Formel (VIII) darstellt eine Gruppe der Formel 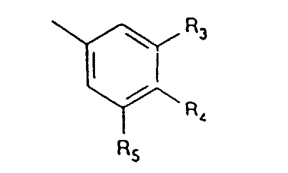 in der R₄ eine -OH-Gruppe darstellt, und wenigstens einer der Reste R₃ und R₅ eine -OH-Gruppe darstellt, was einem Derivat der Formel (VIII a) entspricht, dem oben beschriebenen Schritt der chemischen oder elektrochemischen Behandlung ein Schritt zum Schutz der -OH-Gruppe vorangeht, die R₄ des entsprechenden Derivats der Formel (VIII a) entspricht, wobei der Schritt des Schützens durch Behandlung des Derivats der Formel (VIII a) mit einem Derivat der Formel X¹-CH₃ oder X¹-R_{c} durchgeführt wird, in der X¹ ein Halogenatom darstellt, insbesondere ein Iod- oder Bromatom und R_{c} entsprechend Anspruch 1 definiert ist,
wodurch ein Derivat der Formel (VIII b) erhalten wird, das einem Derivat der Formel (VIII a) entspricht, in der wenigstens ein Rest von R₃ und R₅ -OH darstellt, während R₄ eine -OR_{c}-Gruppe darstellt, in der R_{c} die oben angegebene Definition hat, und das Derivat der Formel (VIII b) dann auf chemischem oder elektrochemischem Weg nach der oben gezeigten Weise mit einem Derivat der Formel NH₂-C(R₁,R₂)-CH₂OH in alkoholischem Milieu behandelt wird, wodurch eine Verbindung der oben genannten Formel (II) erhalten wird, in der R₈ eine -OH-Gruppe darstellt und R₁ und R₂ entsprechend Anspruch 1 definiert sind und Rₓ darstellt eine Gruppe der Formel 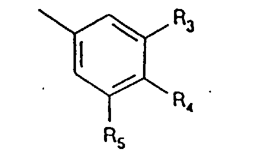 in der wenigstens ein Rest von R₃ und R₅ -OH darstellt, R₄ -OCH₃ oder -OR_{c} darstellt, wobei R_{c} wie oben definiert ist,
b) gegebenenfalls,
- entweder Alkylierung der Verbindung der Formel (II), die durch den vorstehenden Schritt a) erhalten wird, durch die Behandlung dieser Verbindung im basischen Milieu mit einem Derivat der Formel X²-CH₃, in der X² ein Halogenatom darstellt, insbesondere Iod, wodurch eine Verbindung der oben genannten Formel (II) erhalten wird, in der R₈ eine -OCH₃-Gruppe darstellt und R₁, R₂ und Rₓ entsprechend Anspruch 1 definiert sind,
- oder Reduktion der Verbindung der Formel (II), die durch den vorstehenden Schritt a) erhalten wird:
- entweder auf chemischem Weg durch Behandlung der Verbindung der Formel (II) durch eine Essigsäurelösung in Gegenwart von Zn bei Raumtemperatur, wodurch eine Verbindung der folgenden Formel (III) erhalten wird: 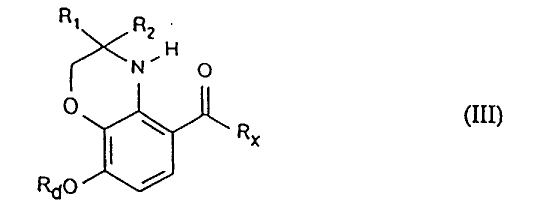 in der R_{d} H darstellt und R₁, R₂ und Rₓ entsprechend Anspruch 1 definiert sind,
mit der Maßgabe, daß wenn in der Verbindung der oben genannten Formel (II) Rₓ eine Gruppe der folgenden Formel darstellt: 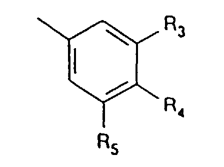 in der R₄ eine -OR_{c}-Gruppe darstellt, R_{c} entsprechend Anspruch 1 definiert ist und R₃ und R₅ entsprechend Anspruch 1 definiert sind, die Reduktion auf dem oben genannten chemischen weg entweder mit ungefähr fünf Äquivalenten Zink durchgeführt wird, wobei die Kontaktzeit zwischen der Verbindung der Formel (II) und dem Zink ungefähr 2 Minuten ist, wodurch eine Verbindung der oben genannten Formel (III) erhalten wird, in der R₄, in der Definition von Rₓ, eine -OR_{c}-Gruppe darstellt, R_{c} so wie oben definiert ist und R₃ und R₅ entsprechend Anspruch 1 definiert sind, oder die Reduktion auf dem oben erwähnten chemischen Weg mit ungefähr hundert Äquivalenten Zink durchgeführt wird, wobei die Kontaktzeit zwischen der Verbindung der Formel (II) und dem Zink ungefähr 10 Minuten ist, oder vorteilhafterweise mit ungefähr fünf Äquivalenten Zink, wobei dann die oben genannte Kontaktzeit ungefähr 30 Minuten ist, wodurch in diesen beiden letzten Fällen eine Verbindung der oben genannten Formel (III) erhalten wird, in der R₄, in der Definition von Rₓ, eine -OH-Gruppe darstellt und R₃ und R₅ entsprechend Anspruch 1 definiert sind,
- oder auf elektrochemischem Weg, durch Elektrolyse bei kontrolliertem Potential der Verbindung der oben genannten Formel (II) in alkoholischer, vorzugsweise methanolischer Lösung in Gegenwart eines Trägerelektrolyts, der insbesondere aus Tetraethylammonium-Salzen ausgewählt wird, wodurch eine Verbindung der oben genannten Formel (III) erhalten wird,
mit der Maßgabe, daß wenn in der Verbindung der oben genannten Formel (II) Rₓ eine Gruppe der folgenden Formel darstellt: 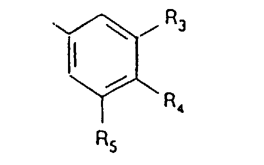 in der R₄ eine -OR_{c}-Gruppe darstellt, R_{c} entsprechend Anspruch 1 definiert ist und R₃ und R₅ entsprechend Anspruch 1 definiert sind, das Potential der Elektrolyse entweder auf eine Weise gewählt wird, mit der eine Verbindung der oben genannten Formel (III) erhalten wird, in der R₄, in der Definition von Rₓ, eine -OR_{c}-Gruppe darstellt, R_{c} so wie oben definiert ist und R₃ und R₅ entsprechend Anspruch 1 definiert sind, oder auf eine Weise, mit der eine Verbindung der oben genannten Formel (III) erhalten wird, in der R₄, in der Definition von Rₓ, eine -OH-Gruppe darstellt und R₃ und R₅ entsprechend Anspruch 1 definiert sind,
c) gegebenenfalls, Alkylierung der Verbindung der Formel (III), die durch den vorstehenden Schritt b) erhalten wird, durch Behandlung dieser Verbindung in basischem Milieu mit einem Derivat der Formel X³-CH₃, in der X³ ein Halogenatom darstellt, insbesondere Iod, wodurch eine Verbindung der oben genannten Formel (III) erhalten wird, in der R_{d} eine -OCH₃-Gruppe darstellt und R₁, R₂ und Rₓ entsprechend Anspruch 1 definiert sind.

18. Verbindungen der Formel (VIII), die so wie in Anspruch 17 beschrieben sind und folgenden Formeln entsprechen:

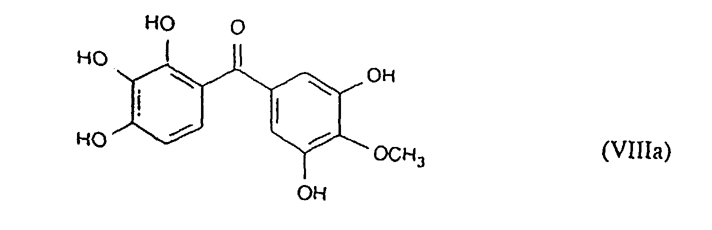

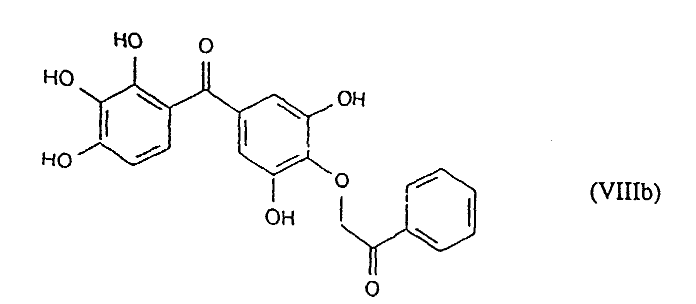

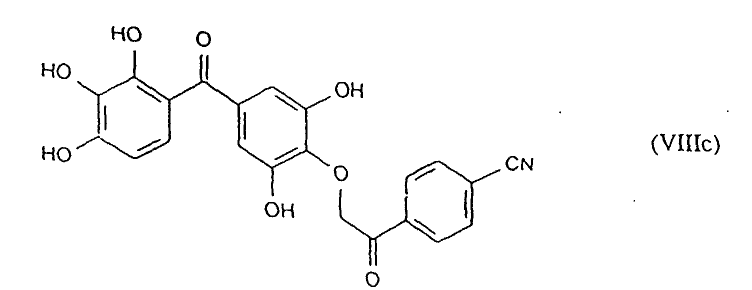

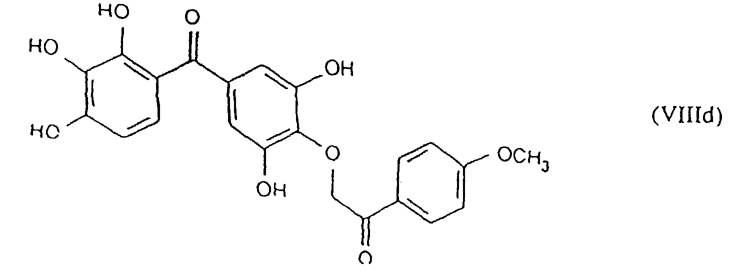

## Claims

1. Compounds corresponding to the following general formula (I): 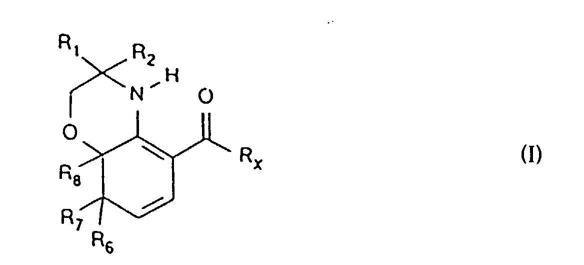 in which:
- R₁ and R₂, independent of each other, represent an alkyl group with 1 to 3 carbon atoms, or a group of formula -CH₂-ORa, Ra representing a hydrogen atom or a methyl group,
- Rₓ represents:
. an alkyl group with 1 to 3 carbon atoms, or
. an -ORₓ₁ group, Rₓ₁ representing an alkyl group with 1 to 3 carbon atoms, or
. a group of formula -CH₂-X in which X represents a halogen atom, or
. a group of formula 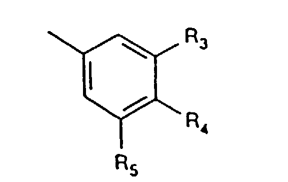 in which:
. R₃ and R₅, independent of each other, represent a hydrogen atom or an -OH group, or an -OCH₃ group,
. R₄ represents a hydrogen atom or an -OR_{b} group, R_{b} representing a hydrogen atom or a methyl group, or R₄ represents a group of formula -OR_{c}, R_{c} representing a group of formula -CH₂-CO-C₆H₄R' in which R' represents a hydrogen atom, -CN or -OCH₃,
- either R₆ represents an -OR_{d} group, R_{d} representing a hydrogen atom or a methyl group, when R₇ and R₈ are associated to form a double bond,
- or R₈ represents an -ORₑ group in which Rₑ represents a hydrogen atom or a methyl group, when R₆ and R₇ are associated to form a C = O group with the carbon atom in position 8 of the cycle.

2. Compounds according to claim 1, characterised in that they correspond to formula (I) in which Rₓ represents:
. a methyl group, or
. a methoxy group, or
. a group of formula -CH₂-X in which X represents a chlorine atom, or
. a group of formula 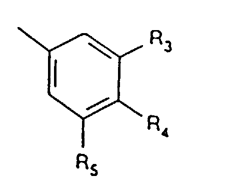 in which R₃, R₄ and R₅ are as defined in claim 1.

3. Compounds according to claim 1 or 2, characterised in that they correspond to the following formula (II): 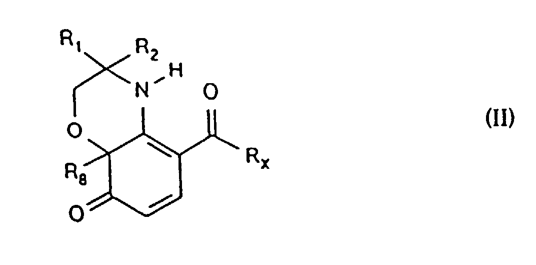 in which R₁, R₂, R₈ and Rₓ are as defined in claim 1.

4. Compounds according to claim 1 or 2, characterised in that they correspond to the following formula (III): 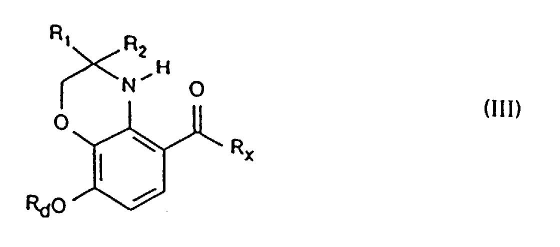 in which R₁, R₂, R_{d} and Rₓ are as defined in claim 1.

5. Compounds according to claim 1 or 2, characterised in that they correspond to the following formula (IV): 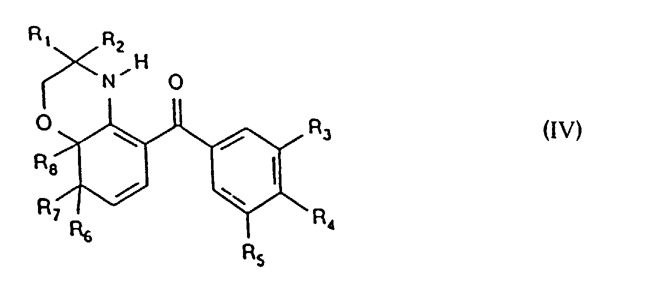 in which R₁ to R₈ are as defined in claim 1.

6. Compounds according to claim 3 or claim 5, characterised in that they correspond to the following formula (V): 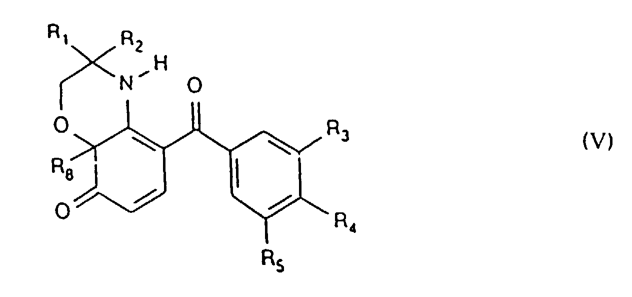 in which R₁ to R₅ and R₈ are such as defined in claim 1.

7. Compounds according to claim 6, characterised in that they correspond to the following formulae:

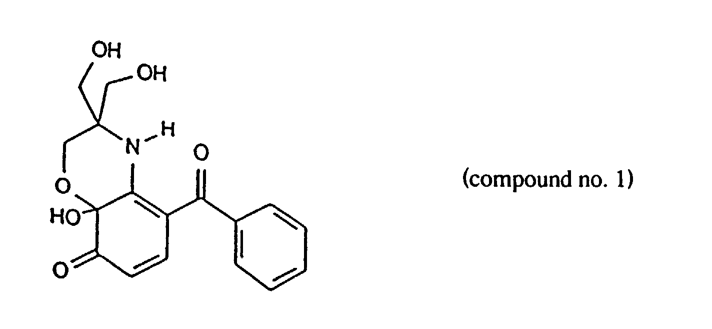

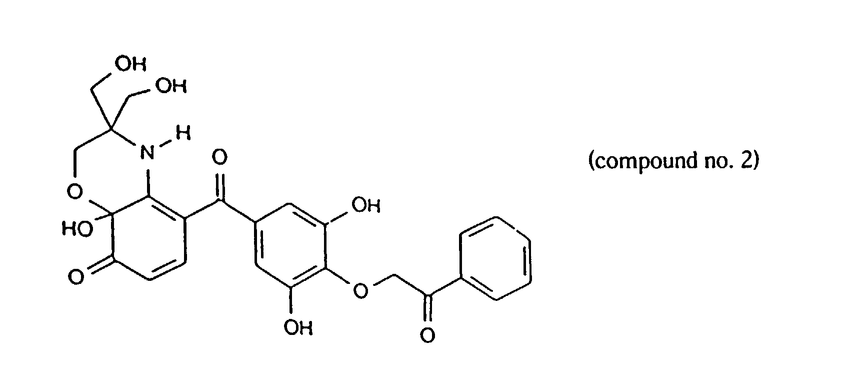

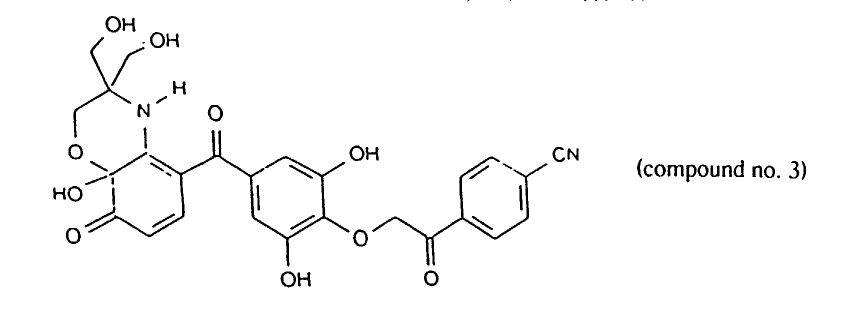

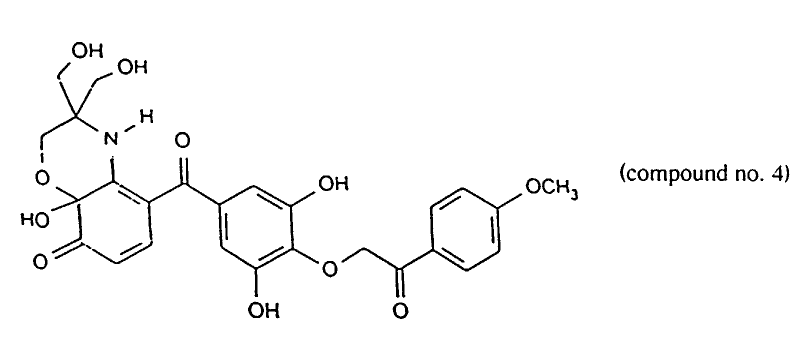

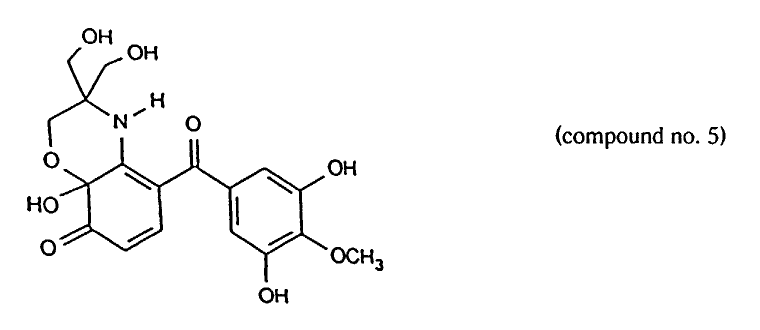

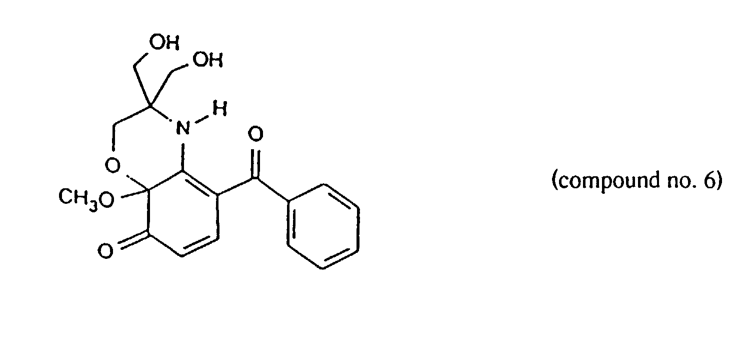

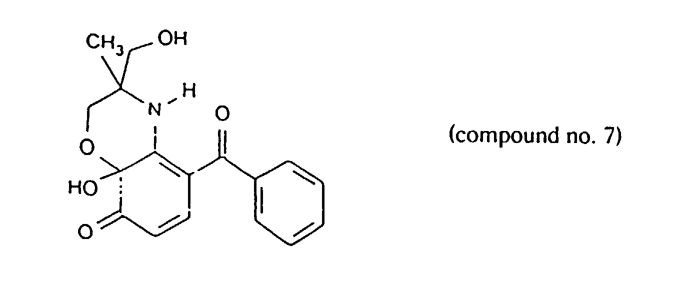

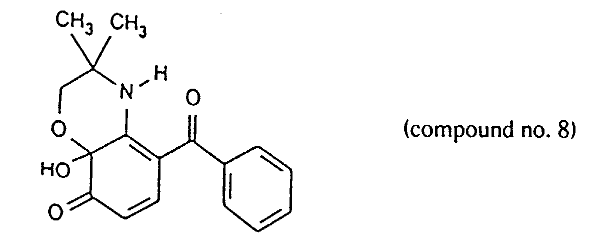

8. Compounds according to claim 1 or 2, characterised in that they correspond to the following formula (VI): 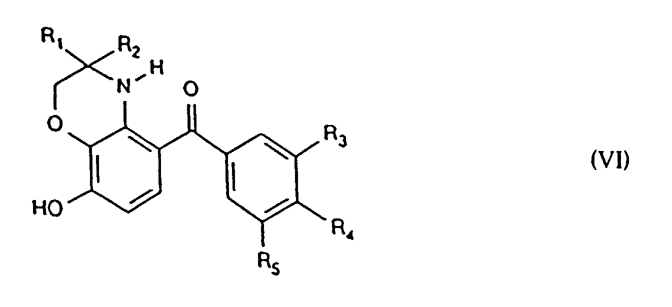 in which R₁, R₂, R₃, R₄ and R₅ are such as defined in claim 1.

9. Compounds according to claim 8, characterised in that they correspond to the following formulae:

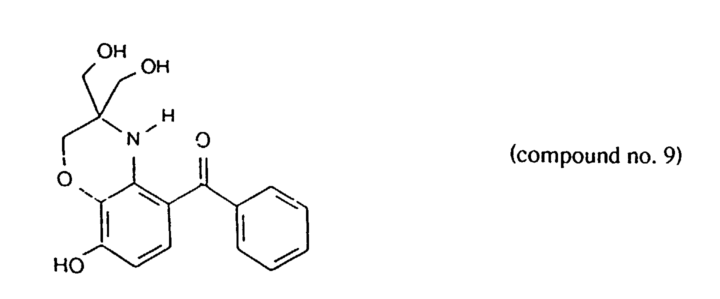

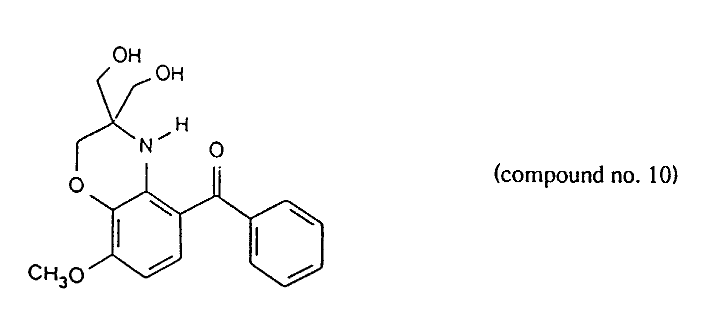

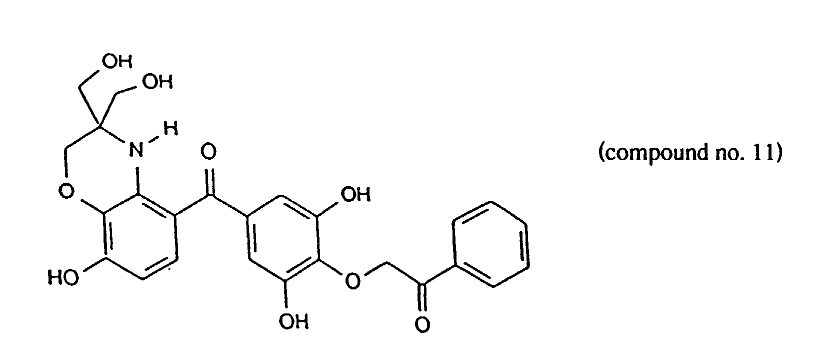

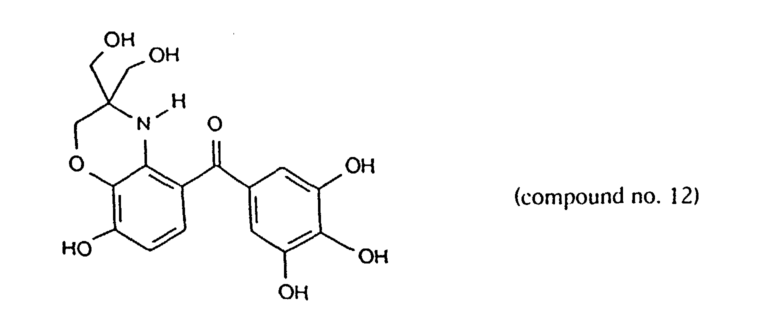

10. Compound according to claim 1 or 2, characterised in that it corresponds to the following formula (VII): 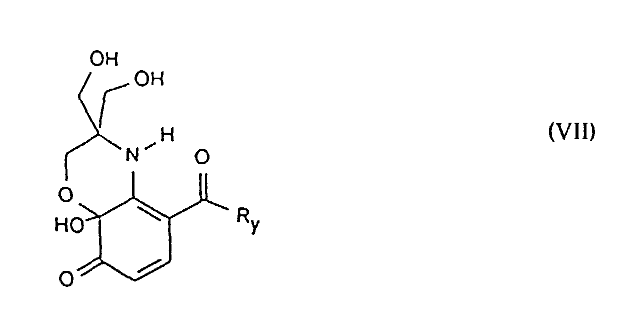 in which R_{y} represents:
- a methyl group (compound no. 13), or
- a methoxy group (compound no. 14), or
- a -CH₂Cl group (compound no. 15)

11. Pharmaceutical composition, characterised in that it includes as an active ingredient, at least on compound according to one of claims 1 to 10, combined with an pharmaceutically acceptable vehicle.

12. Pharmaceutical composition according to claim 11, characterised in that it is in a form which can be administered by oral means, in particular in the form of tablets or capsules, or in a form which can be administered by parenteral means, in particular in the form of an injectable preparation by IV, IM, or SC route.

13. Pharmaceutical composition according to claim 12, characterised in that the daily dose for the oral forms is approximately from 3 mg/kg to approximately 20 mg/kg, preferably approximately 15 mg/kg, and in that the daily dose for the parenteral forms is approximately from 1 mg/kg to 5 mg/kg.

14. Pharmaceutical composition according to one of the claims 11 to 13, characterised in that the unit doses of active ingredient as a function of the different forms of presentation of said composition are the following:
- oral form: approximately 1 mg/kg to approximately 10 mg/kg, preferably approximately 5 mg/kg,
- parenteral form: approximately 0.3 mg/kg to approximately 1 mg/kg.

15. Use of at least one composition according to one of the claims 1 to 10 for obtaining a medicament intended for the treatment of cognitive disorders such as memory disorders, in particular in the elderly.

16. Use according to claim 15 for obtaining a medicament intended for the treatment of cerebral affections likely to be due to, and/or sustained by, even aggravated by the presence of free radicals in the brain, and more particularly for the treatment of Alzheimer's disease, Parkinson's disease, trisomy 21 (Down's syndrome), schizophrenia and epilepsy.

17. Process for the preparation of compounds according to one of the claims 1 to 10, characterised in that it includes the following steps:
a) treatment of the derivative of the following formula (VIII): 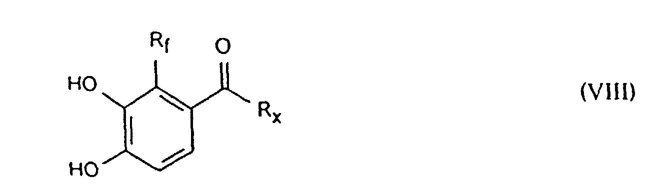 in which R_{f} represents H or OH, and Rₓ is as defined in claim 1,
- either by chemical route by introducing said derivative of formula (VIII) with a derivative of formula NH₂-C(R₁,R₂)-CH₂OH, in which R₁ and R₂ are as defined in claim 1, in the presence of a metal oxide, in particular selected from Ag2O, AgO and PtO₂, in an alcoholic medium, preferably methanol,
- or by electrochemical route by controlled voltage electrolysis of the said derivative of formula (VIII) in the presence of a derivative of formula NH₂-C(R₁,R₂)-CH₂OH such as described above, in an alcoholic solution, preferably methanol, and in the presence of an electrolyte support, in particular selected from tetraethyl ammonium salts,
which leads to the obtaining of a compound of the following formula (II): 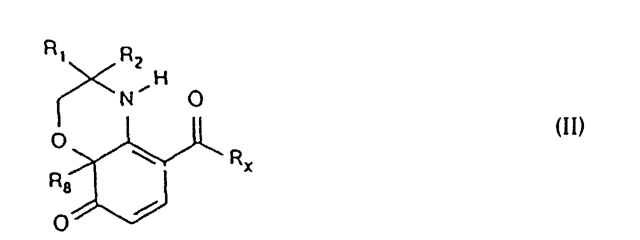 in which R₈ represents an -OH group, and R₁, R₂ and Rₓ are as defined in claim 1, providing that when Rₓ of the derivative of the above-mentioned formula (VIII) represents a group of formula 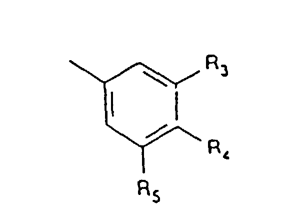 in which R₄ represents an -OH group and at least one of the R₃ or R₅ groups represents an -OH group which corresponds to the derivative of formula (VIIIa), the chemical or electrochemical treatment step described above is preceded by a step which protects the -OH group corresponding to R₄ of the said derivative of formula (VIIIa), this protective step being carried out by treating the derivative of formula (VIIIa) with a derivative of formula X¹-CH₃ or X¹-R_{c} in which X¹ represents a halogen atom, in particular an iodine or bromine atom, and R_{c} is as defined in claim 1,
which leads to the obtaining of a derivative of the formula (VIIIb) corresponding to a derivative of formula (VIIIa) in which at least one of R₃ or R₅ represent -OH, whilst R₄ represents an -OR_{c} group, R_{c} having the definition indicated above, this derivative of the formula (VIIIb) then being treated by chemical or electrochemical route in the manner indicated above by a derivative of formula NH₂-C(R₁,R₂)-CH₂OH in an alcoholic medium, which leads to the obtaining of a compound of the above-mentioned formula (II) in which R₈ represents an -OH group, and R₁ and R₂ are as defined in claim 1, and Rₓ represents a group of formula 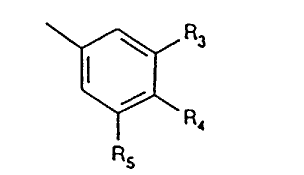 in which at least one of R₃ or R₅ represents -OH, R₄ represents -OCH₃ or -OR_{c}, R_{c} being defined above,
b) if appropriate,
- either alkylation of the compound of formula (II) obtained in the preceding step a) by treatment of this compound in a basic medium by a derivative of formula X²-CH₃ in which X² represents a halogen atom, in particular iodine, which leads to the obtaining of a compound of the above-mentioned formula (II) in which R₈ represents an -OCH₃ group, and R₁, R₂ and Rₓ are as defined in claim 1,
- or reduction of the compound of formula (II) obtained in the preceding step a) :
- either by chemical route by treating the compound of formula (II) with an acetic acid solution in the presence of Zn at ambient temperature, which leads to the obtaining of a compound of the following formula (III): 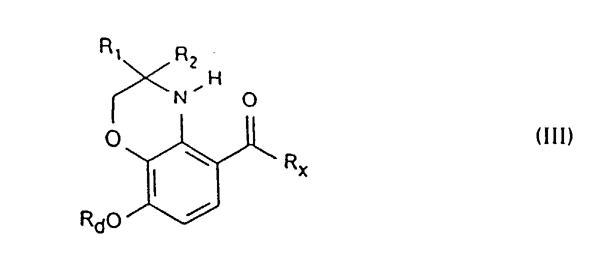 in which R_{d} represents H, and R₁, R₂, and Rₓ are as defined in claim 1,
providing that when the compound of the above-mentioned formula (II) is such that Rₓ represents a group of the following formula: 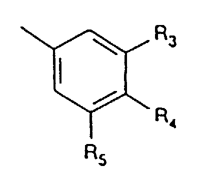 in which R₄ represents an -OR_{c} group, R_{c} being as defined in claim 1, and R₃ and R₅ being as defined in claim 1, the reduction by the above-mentioned chemical route is carried out either with approximately five equivalents of zinc, the contact time between the said compound of formula (II) and the zinc being approximately 2 minutes, which leads to the obtaining of a compound of the above-mentioned formula (III) in which R₄, in the definition of Rₓ, represents an -OR_{c} group, R_{c} being as defined above, and R₃ and R₅ being as defined in claim 1, or with approximately five equivalents of zinc, the contact time between the said compound of formula (II) and the zinc being approximately 10 minutes, or advantageously with approximately five equivalents of zinc, the above-mentioned contact time then being approximately 30 minutes which leads, in the two latter cases, to the obtaining of a compound of the above-mentioned formula (III) in which R₄, in the definition of Rₓ, represents an -OH group, and R₃ and R₅ being as defined in claim 1,
- or by electrochemical route, by controlled voltage electrolysis of the compound of the above-mentioned formula (II) in an alcoholic solution, preferably methanol, in the presence of an electrolyte support, selected in particular from tetraethyl ammonium salts, which leads to the obtaining of the compound of the above-mentioned formula (III),
provided that when the compound of the above-mentioned formula (II) is such that Rₓ represents a group of the following formula: 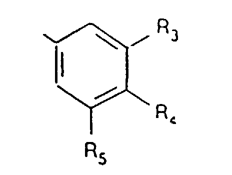 in which R₄ represents an -OR_{c} group, R_{c} being as defined in claim 1, and R₃ and R₅ being as defined in claim 1, the electrolysis voltage is selected either in a manner so as to obtain a compound of the above-mentioned formula (III) in which R₄, in the definition of Rₓ, represents an -OR_{c} group, R_{c} being as defined above, and R₃ and R₅ being as defined in claim 1, or in a manner so as to obtain a compound of the above-mentioned formula (III) in which R₄, in the definition of Rₓ, represents an -OH group, and R₃ and R₅ being as defined in claim 1,
c) if appropriate, alkylation of the compound of formula (III) obtained in preceding step b) by treatment of this compound in a basic medium with a derivative of formula X³-CH₃, in which X³ represents a halogen atom, in particular iodine, which leads to the obtaining of a compound of the above-mentioned formula (III) in which R_{d} represents an -OCH₃ group, and R₁, R₂ and Rₓ are as defined in claim 1.

18. Compounds of formula (VIII) as described in claim 17 and corresponding to the following formulae:
